# DEMANDE DE BREVET EUROPEEN

(11) **EP 2 354 234 A2**
(43) Date de publication de la demande: **10.08.2011**
(21) Numéro de dépôt: 11154403.7
(22) Date de dépôt: 05.01.2007
(51) Int. Cl.: C12N 15/82, C07K 14/415, A01H 5/00

(54) **Promoteurs de gènes pouvant etre utilises chez les plantes**

(30) Priorité: 06.01.2006 FR 0600137
(62) Demande divisionnaire de: 07712640.7
(71) Demandeur: Vilmorin & Cie, 75001 Paris (FR); Centre National de la Recherche Scientifique, 75794 Paris Cedex 16 (FR); Université De Poitiers, 86034 Poitiers Cedex (FR)
(72) Inventeur: Landouar-Arsivaud, Lucie, 33141 Saillans (FR); Lemoine, Rémi, 86000 Poitiers (FR)
(74) Mandataire: Sellin, Carole

(57) **Abrégé**

La présente invention concerne des séquences d'acides nucléiques présentant une activité de promoteur transcriptionnel préférentiellement dans le phloème de plantes en conditions de stress, ou dans les racines, des séquences dérivées, des construits contenant de telles séquences ainsi que des cellules transformées par lesdits construits et des plantes transgéniques. La présente invention permet de mettre un quelconque transgène sous le contrôle transcriptionnel d'un promoteur dont l'activité est tissu-spécifique, organe-spécifique et/ou inductibles par des facteurs environnementaux, tels que des stress biotiques ou abiotiques.

## Description

Dans le domaine agricole il peut s'avérer intéressant de pouvoir augmenter ou diminuer l'expression de gènes d'intérêt au niveau de certains tissus d'une plante et/ou en réponse à des facteurs environnementaux tels que des stress biotiques ou abiotiques qui influent sur la productivité des cultures agricoles.

La présente invention concerne de nouvelles séquences régulatrices de transcription identifiées chez le céleri, dont l'activité est tissu-spécifique, organe-spécifique et/ou inductible par des facteurs environnementaux, tels que des stress biotiques ou abiotiques, et leur utilisation pour transformer des plantes et mettre un gène d'intérêt sous leur contrôle.

La salinité est par exemple, l'un des plus sévères facteurs environnementaux limitant la productivité des cultures agricoles. Bien que la salinité des sols soit largement antérieure à l'agriculture, le problème a été aggravé par les pratiques de l'agriculture, telle que l'irrigation. A l'heure actuelle, environ 20% des surfaces cultivées dans le monde et presque la moitié des surfaces irriguées sont touchées par la salinité.

En plus d'un énorme coût financier, la salinité a d'autres impacts sérieux sur l'infrastructure, les réserves en eau, et sur la structure sociale et la stabilité des communautés humaines.

Les réponses à la salinisation ont été de deux ordres : (i) introduire une gestion de l'environnement afin de contrôler l'augmentation de sel dans le sol, grâce à l'aménagement de l'irrigation et du drainage, (ii) utiliser l'ingénierie génétique sur les plantes afin d'augmenter leur tolérance au sel.

La présente invention concerne notamment le second axe consistant à rechercher et à comprendre les mécanismes qui permettent à certaines plantes de mieux tolérer le sel et le stress en général, pour développer des stratégies rendant les plantes cultivées plus tolérantes au sel ou à d'autres stress.

Les plantes fixées à leur substrat sont soumises, tout au long de leur vie, à de nombreuses variations des conditions environnementales auxquelles elles doivent faire face en adaptant leur croissance et leur développement. Les stress abiotiques correspondent à des modifications importantes de facteurs chimiques ou physiques de l'environnement, alors que les stress biotiques sont induits par une interaction entre la plante et un organisme vivant. Les facteurs abiotiques, qui affectent particulièrement les rendements des cultures, sont le stress hydrique (sécheresse ou surplus d'eau), les modifications extrêmes de température, les déficits en éléments minéraux du sol et la forte concentration en sels ou en métaux lourds dans le sol.

Le stress salin provoqué par une concentration trop importante en NaCl dans l'environnement de la plante entre donc dans la catégorie des stress abiotiques. Bien que le Na⁺ soit nécessaire pour quelques plantes, particulièrement pour les halophytes, une forte concentration en NaCl est un facteur limitant, voire toxique, pour la croissance des plantes. Ce phénomène est répandu sur de grandes surfaces arables du monde entier.

En réponse aux stress environnementaux, les plantes ont développé un panel de stratégies physiologiques et biochimiques, pour s'adapter ou du moins tolérer les conditions de stress. Ces stratégies sont liées à des modifications de l'expression génique, comme il a été mis en évidence par des changements dans les quantités d'ARNm et de protéines nouvellement synthétisées. Les gènes identifiés codent pour des protéines associées à de nombreuses fonctions telles que la compartimentation des ions, l'équilibre du potentiel redox, la dégradation ou la protection des protéines, la synthèse d'osmolytes. De nombreuses études ont été menées en rapport avec les molécules nommées « osmolytes », parce que leur synthèse est augmentée dans grand nombre de stress abiotiques (Hasegawa et al. 2000a et b). Ces molécules peuvent s'accumuler à de forts taux dans la réponse au sel (ou dans le stress osmotique) permettant ainsi de restaurer l'équilibre en eau dans la cellule.

Il a été proposé que le génie métabolique puisse jouer un rôle majeur dans l'augmentation de la tolérance des plantes au stress. Il a été montré que des plantes transformées pour exprimer des enzymes conduisant à la synthèse de certains osmolytes étaient plus résistantes au stress salin que des plantes non transformées (Tarczinsky et al., 1993, Shen et al., 1997a et b), confirmant ainsi l'importance des osmolytes. Cependant, certains de ces résultats sont sujet à controverse dans la mesure où les taux d'osmolytes dans les plantes transgéniques étaient trop faibles pour expliquer un effet osmotique (Karakas et al., 1997). Cet effet a donc été attribué à un rôle anti-oxydant plutôt qu'à un rôle purement d'osmolyte.

La synthèse de tels osmolytes a lieu dans des tissus sources ou s'appuie sur des molécules dérivant de photoassimilats. Par conséquent, le transport sur des longues distances des photoassimilats dans le phloème est certainement affecté durant le stress salin, bien que peu de données soient disponibles sur ce point (Noiraud et al. 2000).

La sève élaborée du phloème transporte également une large gamme d'ions, de métabolites et de macromolécules telles que des protéines et des acides nucléiques, dont un grand nombre est impliqué dans la signalisation. Le phloème peut donc être considéré comme l'acteur majeur de la communication entre tissus dans les plantes vasculaires (Ruiz-Medrano et al., 2001).

Cependant, à ce jour, les connaissances sur les gènes spécifiquement exprimés dans le phloème ou dans les racines sont peu nombreuses, surtout chez des espèces potagères ou maraîchères telles que le céleri, et ce malgré les récents travaux sur différents modèles de plantes (Vilaine et al., 2003). Les connaissances sur les promoteurs de ces gènes, notamment sur leurs propriétés phloème-spécifique, racine-spécifique et inductible par le stress, sont encore moins nombreuses voire inexistantes.

En l'absence de telles connaissances, il n'est donc pas possible de mettre une quelconque séquence codante sous le contrôle d'un promoteur qui permette de n'exprimer cette séquence codante que dans des conditions de stress, préférentiellement dans le phloème ou les racines.

Les présents inventeurs ont maintenant déterminé la séquence de trois promoteurs, dont l'activité est préférentiellement dans les tissus vasculaires et plus particulièrement le phloème, ou les racines, cette activité étant le cas échéant fonction des conditions de stress de la cellule. Les inventeurs ont également réalisé des constructions à l'aide de ces promoteurs, permettant de n'exprimer des séquences d'intérêt que dans des conditions de stress, préférentiellement dans le phloème, ou préférentiellement dans les racines.

En conséquence, la présente demande concerne, selon un premier aspect de l'invention, une séquence d'acides nucléiques ayant une activité de promoteur transcriptionnel, telle que ladite séquence comprend SEQ ID N°1, 2 ou 3, ou bien un fragment ou des fragments (ou parties) d'au moins l'une de ces séquences. Un fragment ou une partie de SEQ ID N°1, 2 ou 3 est défini comme une séquence comprenant au moins 30 nucléotides consécutifs de SEQ ID N°1, 2 ou 3.

Une partie de SEQ ID N°1, 2 ou 3 peut ne contenir que 30 nucléotides, mais elle peut contenir avantageusement au moins 50 nucléotides consécutifs de SEQ ID N°1, 2 ou 3, par exemple exactement 50, ou plus de 50, ou bien plus de 75, 80 ou 90. Une partie peut également contenir plus de 100 nucléotides consécutifs de SEQ ID N°1, 2 ou 3, notamment 120, 150 voire 180 ou 200. Selon la présente invention, des parties de SEQ ID N°1, 2 ou 3 qui sont préférées sont des fragments correspondant à la quasi-totalité de SEQ ID N°1, 2 ou 3, avec uniquement la délétion de 1 à 10, 20, 30 ou 50 nucléotides, aux extrémités 3' et/ou 5' ou bien au sein des séquences SEQ ID N°1, 2 ou 3.

Il est également envisagé qu'une séquence selon l'invention comprenne au moins deux fragments de SEQ ID N°1, 2 ou 3, l'un au moins desdits fragments ayant une longueur minimale de 30 nucléotides. Les différents fragments peuvent être des fragments issus de séquences distinctes, par exemple un fragment de SEQ ID N°1 et un fragment de SEQ ID N°2, ou bien des fragments de la même séquence. Dans ce derniers cas, les fragments sont de préférence non consécutifs, par exemple séparés de 2, 10, 20 ou 50 nucléotides.

Selon une mise en oeuvre préférée de l'invention, une séquence selon l'invention comprend ou consiste en l'intégralité de l'une des séquences SEQ ID N°1, 2 ou 3.

Par séquence ayant une activité de promoteur transcriptionnel, on entend une séquence présentant une activité promotrice, c'est-à-dire de nature à promouvoir la transcription d'une séquence placée en aval de ladite séquence promotrice, éventuellement en présence de co-facteurs adéquats.

Une telle activité de promoteur transcriptionnel peut être testée en clonant une séquence susceptible de présenter une telle activité en amont d'une séquence à transcrire quelconque, en présence d'ARN polymérase et de ribonucléotides. Si on obtient des molécules d'ARN, alors qu'aucune molécule d'ARN n'est obtenue en l'absence de clonage de la séquence à tester, on peut alors en déduire que ladite séquence à tester présente une activité de promoteur transcriptionnel. Un test adéquat est décrit dans la partie expérimentale.

Le site d'initiation de la transcription peut se trouver ou bien au sein d'une séquence selon l'invention, ou bien à l'extrémité 3' d'une séquence selon l'invention ou en aval côté 3' d'une séquence selon l'invention. Dans le premier cas, une partie de la séquence selon l'invention est transcrite.

En sus de tout ou partie de l'une des séquences SEQ ID N°1, 2 ou 3, une séquence selon l'invention peut également contenir des séquences additionnelles, dans la mesure où elles ne suppriment pas la propriété de promoteur transcriptionnel. De telles séquences additionnelles peuvent notamment être des « enhancers », ou d'autres séquences telles que des sites de fixation pour différentes protéines. Ladite activité de promoteur transcriptionnel peut se manifester dans tout type de cellule, *in vivo,* et/ou également *in vitro* en solution, en présence d'ARN polymérase et de tous les éléments nécessaires à la transcription, notamment en présence de ribonucléotides.

Avantageusement, une séquence selon la présente invention présente une activité promotrice ubiquitaire, c'est-à-dire tant dans des cellules procaryotes qu'eucaryotes, dans des cellules végétales ou animales, ou en présence d'une ARN polymérase d'une de ces cellules. Une séquence selon la présente invention est de préférence active comme promoteur dans des cellules végétales, ou bien en présence d'une ARN polymérase issue d'une cellule végétale.

Une propriété préférée d'une séquence selon l'invention est sa capacité à promouvoir la transcription dans une cellule végétale en réponse à un stimulus. Ledit stimulus est de préférence un stimulus lié à un stress. De préférence, l'activité de promoteur transcriptionnel possédée par une séquence selon l'invention est sensible à un stress biotique ou abiotique ; cette activité est induite ou bien augmentée sous conditions de stress biotique ou abiotique.

On appelle stress biotique le stress d'une plante qui est induit par une interaction entre la plante et un organisme vivant, par exemple lors d'une attaque de pucerons.

Les stress abiotiques quant à eux correspondent à des modifications importantes de facteurs chimiques ou physiques de l'environnement. Les facteurs abiotiques, qui affectent particulièrement les rendements des cultures, sont le stress hydrique (sécheresse ou surplus d'eau), les modifications extrêmes de température, les déficits en éléments minéraux du sol et la forte concentration en sels ou en métaux lourds dans le sol.

Le stress peut être ressenti au niveau cellulaire, ou au niveau d'un organe de la plante, ou bien au niveau de l'organisme dans son entier.

L'activité transcriptionnelle d'une séquence selon l'invention peut également être induite lorsque certaines composantes ou conditions liées au stress sont reproduites. Par exemple, l'activité transcriptionnelle d'une séquence selon l'invention peut être induite suite à la présence de certains facteurs qui sont liés au stress, tels que des protéines de stress.

Selon une mise en oeuvre préférée, une séquence selon l'invention présente une activité de promoteur transcriptionnel préférentiellement dans les tissus vasculaires. Des tissus vasculaires tout particulièrement concernés sont le phloème et/ou le xylème, mais de préférence le phloème.

Selon une autre mise en oeuvre préférée, une séquence selon l'invention présente une activité de promoteur transcriptionnel spécifique d'un ou plusieurs organes, c'est-à-dire un promoteur dont l'activité est plus importante dans certains organes de la plante, par exemple, les tiges, les feuilles ou les racines, voire limitée à ces organes. Dans une mise en oeuvre préférée, une séquence selon l'invention présente une activité de promoteur transcriptionnel spécifique des racines.

L'activité de promoteur transcriptionnel présentée par une séquence selon l'invention se manifeste de préférence dans des cellules végétales et tout particulièrement dans des cellules de plantes maraîchères ou potagères, c'est-à-dire des plantes utilisées pour la production individuelle ou intensive des légumes des fines herbes et de certains fruits (tels que le melon ou la pastèque), dont font notamment partie les Apiacées, Astéracées, Brassicacées (ou Crucifères), Chénopodiacées, Cucurbitacées, Poacées, Rosacées, Solanacées, Valérianaceés ou Légumineuses. Plus particulièrement l'activité de promoteur transcriptionnel se manifeste dans des cellules de céleri (*Apium graveolens* L.), *d'Arabidopsis thaliana ou* de tomate (*Solanum lycopersicum L.*).

Les séquences selon l'invention comprennent SEQ ID N°1, 2 ou 3 ou bien un ou des fragments (ou parties) d'au moins l'une de ces séquences. De préférence, les séquences selon l'invention consistent en l'une des séquences suivantes : SEQ ID N°1, 2, 3, 4, 5 ou 6, ou bien elles comprennent l'une de ces séquences. SEQ ID N°4 est une séquence comprenant SEQ ID N°1, SEQ ID N°5 est une séquence comprenant SEQ ID N°2 et SEQ ID N°6 est une séquence comprenant SEQ ID N°3.

Selon un second aspect de la présente invention, une séquence selon l'invention peut également être une séquence qui présente au plus 5 mutations ponctuelles par rapport à une séquence telle que définie précédemment selon le premier aspect, c'est-à-dire par rapport à une séquence comprenant SEQ ID N°1, 2 ou 3 ou bien un ou des fragments (ou parties) d'au moins l'une des séquences SEQ ID N°1, 2 ou 3, où ledit fragment comprend au moins 30 nucléotides consécutifs de SEQ ID N°1, 2 ou 3. Une séquence selon ce second aspect de l'invention peut présenter par exemple une unique mutation par rapport à une séquence selon le premier aspect, mais de préférence au moins deux mutations ponctuelles, voire 3, 4 ou 5 mutations ponctuelles.

Par mutation ponctuelle, on entend une seule modification d'un unique acide nucléique, ladite modification pouvant être la suppression d'un acide nucléique par rapport à la séquence sans mutation, ou bien l'addition d'un acide nucléique par rapport à la séquence sans mutation ou bien la modification d'un acide nucléique par rapport à la séquence sans mutation. Par modification d'un acide nucléique, on entend à la fois la substitution d'un acide nucléique par un autre acide nucléique naturel (par exemple substitution de A en G) et la modification chimique d'un acide nucléique naturel (par exemple le remplacement d'une adénine en 2-méthyl adénosine ou en 4-acétylcytidine).

Les différents acides nucléiques modifiés susceptibles d'être incorporés dans une séquence selon l'invention sont bien connus de l'homme du métier.

Selon une mise en oeuvre particulière, une mutation peut être introduite afin de supprimer l'activité de promoteur transcriptionnel d'une séquence selon le premier aspect de l'invention, ou bien pour en modifier ses caractéristiques.

De préférence, une séquence selon ce second aspect de l'invention présente au plus 5 mutations ponctuelles par rapport à une séquence comprenant un fragment de SEQ ID N°1, 2 ou 3 d'au moins 40 nucléotides consécutifs, de préférence d'au moins 50 nucléotides consécutifs.

Une séquence selon ce second aspect de l'invention peut également posséder une activité de promoteur transcriptionnel, mais pas nécessairement. Les mutations introduites par rapport à la séquence selon le premier aspect de l'invention peuvent permettre de modifier l'activité de promoteur d'une telle séquence, par exemple en en modifiant son taux d'initiation par seconde, ou bien en en modifiant sa spécificité cellulaire ou les conditions d'initiation de la transcription.

Selon un troisième aspect, la présente invention concerne également une séquence d'acides nucléiques qui présente une activité de promoteur transcriptionnel et qui possède au moins 70% d'identité avec une séquence choisie parmi SEQ ID N°1, SEQ ID N°2, SEQ ID N°3, SEQ ID N°4, SEQ ID N°5 et SEQ ID N°6. De préférence, le pourcentage d'identité est supérieur à 70%, c'est-à-dire au moins 75 ou 80%, mais de préférence au moins 85 ou 90% d'identité, voire au moins 95% d'identité. Des séquences également envisagées présentent 97%, 98%, 99%, voire 99,5% d'identité avec l'une des séquences SEQ ID N°1 à 6.

Le pourcentage d'identité entre deux séquences S1 et S2 d'acides nucléiques se calcule en alignant les deux séquences de telle manière à maximiser les séquences communes, éventuellement par l'insertion de trous (« gap »), puis en divisant le nombre total d'acides nucléiques communs par le nombre d'acides nucléiques de la séquence la plus longue.

Un fort pourcentage d'identité se traduit également par l'hybridation des séquences S1 et S2 monocaténaires dans des conditions de forte stringence. Les conditions assurant une forte stringence sont bien connues de l'homme du métier. En effet, la stringence est définie par les conditions expérimentales de température, de pH et de force ionique permettant l'hybridation moléculaire. Deux paramètres conditionnent notamment la stringence : la température et la concentration en sel. Des conditions de haute stringence correspondent par exemple à une haute température (au-dessus du Tm) et/ou faible force ionique, afin de favoriser les appariements spécifiques.

Des conditions de forte stringence peuvent être illustrées, à titre d'exemple, par un milieu ayant les propriétés décrites dans la partie expérimentale.

Par « séquence selon l'invention », on désigne aussi bien une séquence selon le premier, le deuxième ou bien le troisième aspect de l'invention.

Une séquence selon l'invention peut être notamment de l'ADN double brin, mais également de l'ADN simple brin ou bien de l'ADN partiellement double-brin. Par ailleurs, une séquence selon l'invention peut avoir des topologies très variées, il peut également s'agir d'ADN circulaire, éventuellement surenroulé.

La présente invention concerne également une séquence complémentaire de l'une des séquences précédemment décrites.

Une séquence selon l'invention peut être utilisée dans divers domaines techniques et tout particulièrement en agriculture, notamment dans le secteur des plantes transgéniques.

L'invention concerne également des sondes d'acides nucléiques telles que lesdites sondes sont capables de s'hybrider dans des conditions de forte stringence avec une séquence choisie parmi les séquences SEQ ID N°1, 2 et 3. Une sonde telle que mentionnée comprend au moins 25 nucléotides, de préférence au moins 30. En règle générale toutefois, les sondes d'acides nucléiques comprennent moins de 150 et même moins de 100, voire moins de 50 acides nucléiques.

De telles sondes peuvent permettre d'identifier des séquences homologues à l'une des séquences SEQ ID N°1, 2 ou 3 dans différents organismes, ou bien permettre de détecter la présence de l'une de ces séquences, in vitro ou in vivo. De telles sondes peuvent être des sondes ADN ou ARN, simple ou double brin. Elles peuvent être utilisées par exemple comme amorces dans des expériences de PCR ou bien dans un Southern Blot.

La présente invention porte également sur un construit d'ADN, ou construction d'ADN, qui est constitué de ou bien comprend une séquence ayant une activité promotrice telle que décrite précédemment et qui comprend aussi une séquence d'acides nucléiques à transcrire. Le construit selon l'invention est réalisé de telle manière que la séquence d'intérêt à transcrire est située en aval de la séquence promotrice, c'est-à-dire à l'extrémité 3' de la séquence promotrice, et la transcription de la séquence d'intérêt est dirigée par la séquence promotrice. La séquence selon l'invention et la séquence à transcrire peuvent être contiguës ou bien peuvent être séparées par des acides nucléiques intercalaires. Dans ce dernier cas, la séquence intercalaire comprend de préférence moins de 200, voire moins de 50 ou moins de 20 nucléotides.

Un construit selon l'invention peut également comprendre des séquences permettant la terminaison de la transcription, voire également des séquences permettant la terminaison de la traduction (codon stop).

On dit que la transcription d'une séquence d'acides nucléiques est dirigée par un promoteur quand ledit promoteur permet la transcription de la séquence et qu'il contrôle cette transcription. La séquence d'intérêt est donc transcrite dans des conditions et à un taux qui est fonction du promoteur.

Il est donc possible, à l'aide de l'enseignement de l'invention, de placer une séquence d'intérêt sous le contrôle d'une séquence promotrice de l'invention afin que ladite séquence d'intérêt soit transcrite uniquement dans des conditions de stress, par exemple uniquement dans des conditions de stress salin dans des cellules de plantes, ou encore spécifiquement au niveau d'un organe de la plante, tel que les racines, ou encore d'un tissu, tel que le phloème ou et xylème. Du fait des propriétés spécifiques des séquences promotrices de l'invention, il est donc possible d'obtenir la transcription d'une séquence d'intérêt préférentiellement dans des cellules vasculaires, et préférentiellement dans des conditions de stress ou encore préférentiellement au niveau racinaire.

Selon une mise en oeuvre préférée, une séquence d'intérêt est placée sous le contrôle de la SEQ ID n°1 ou de la SEQ ID n°3 ou encore d'une séquence selon l'invention dérivée de l'une de ces deux séquences et présentant une activité de promoteur transcriptionnel, afin que la transcription de ladite séquence d'intérêt soit induite en réponse à un stress, notamment un stress salin, et plus particulièrement au niveau du phloème de la plante.

Selon une autre mise en oeuvre préférée, une séquence d'intérêt est placée sous le contrôle de la SEQ ID n°2 ou d'une séquence selon l'invention dérivée de la SEQ ID n°2 et présentant une activité de promoteur transcriptionnel, afin que ladite séquence d'intérêt soit transcrite de façon plus importante au niveau phloème de la plante.

Selon une autre mise en oeuvre préférée, une séquence d'intérêt est placée sous le contrôle de la SEQ ID n°1 ou d'une séquence selon l'invention dérivée de la SEQ ID n°1 et présentant une activité de promoteur transcriptionnel, afin que ladite séquence d'intérêt soit transcrite de façon plus importante au niveau des racines qu'au niveau des tiges et des feuilles.

Selon une mise en oeuvre particulière de l'invention, la séquence promotrice et la séquence d'intérêt à transcrire qui se trouvent au sein dudit construit, sont hétérologues l'une par rapport à l'autre. Par hétérologues, on entend qu'elles proviennent de sources différentes, par exemple d'organismes différents. On dira également que deux séquences sont hétérologues quand l'une au moins des deux séquences est artificielle (c'est-à-dire non présente dans la nature). Le construit comprenant une séquence à transcrire hétérologue par rapport à une séquence promotrice selon l'invention comprend donc un gène chimère ou gène chimérique. De préférence, la séquence à transcrire est autre que la séquence à transcrire naturellement associée à la séquence promotrice selon l'invention.

Selon une mise en oeuvre toute préférée, la séquence d'intérêt à transcrire est une séquence codante, qui donc non seulement est à transcrire, mais également à traduire. Il peut s'agir d'une séquence intégralement codante, par exemple une séquence d'ADNc, ou bien d'une séquence partiellement codante, par exemple une séquence comprenant des introns et des exons. La protéine ou le peptide codé par une telle séquence d'intérêt peut être par exemple une protéine de résistance à un herbicide ou à un antibiotique, ou bien un facteur de croissance, un facteur de résistance au stress, une protéine toxique ou létale. En fonction de ses besoins, l'homme du métier saura quelle séquence d'intérêt placer sous le contrôle d'un promoteur selon l'invention.

Il s'agit de préférence de séquences codant pour des peptides d'intérêt dans le domaine végétal, notamment de peptides ou protéines ayant une activité dans des cellules végétales, ou bien ayant un intérêt nutritionnel ou esthétique. Il peut s'agir également de protéines létales, permettant de supprimer toute plante ayant subi un stress. Il peut également s'agir d'une protéine ou d'un peptide permettant la détection de cellules l'exprimant.

Par construit d'ADN, dans le cadre de la présente invention, on entend tout support d'ADN non naturel. Un tel construit peut être notamment un vecteur permettant le transfert du construit dans une cellule. Le construit est de préférence un vecteur, par exemple un vecteur viral mais de préférence un vecteur plasmidique ou plasmide. Un plasmide avantageux dans le cadre de la présente invention est le plasmide Ti des agrobactéries, ou un plasmide dérivé du plasmide Ti, ayant conservé les propriétés de transfert d'ADN, mais dépourvu de gènes oncogènes.

Un plasmide selon la présente invention peut comprendre, en plus de la séquence promotrice selon l'invention et d'une séquence d'intérêt à transcrire, des gènes de résistance permettant notamment de faire des sélections positives ou négatives. Lesdits gènes de résistances peuvent être des gènes de résistances à des herbicides ou bien à des antibiotiques.

Il peut également être avantageux que le construit ou le plasmide selon l'invention comprenne des gènes de résistance bactériens, par exemple pour faciliter des étapes antérieures de multiplication en bactéries.

L'invention concerne également une cellule de plante qui a été transformée par une séquence selon l'invention ou par un construit tel que décrit précédemment, notamment par un construit comprenant une séquence à transcrire hétérologue par rapport à une séquence promotrice selon l'invention, quel que soit le moyen employé pour effectuer la transformation. En effet, à l'heure actuelle, des moyens très divers sont connus pour permettre la transformation de cellules par une séquence d'acides nucléiques. On peut citer, à titre d'exemple, la transformation par électroporation, par bombardement et par l'utilisation d'agrobactéries. Il est évident que, en fonction du type de cellules à transformer et de l'espèce en question, notamment selon l'espèce végétale, certaines techniques sont préférées à d'autres. L'homme du métier connaît, pour chaque type cellulaire, quelles sont les techniques les plus appropriées pour effectuer une transformation.

De même, il est bien connu de l'homme du métier quelles sont les techniques qui permettent d'obtenir une transformation transitoire de la cellule, le matériel génétique transféré étant perdu par la suite, et quelles sont les techniques qui permettent l'intégration de la séquence transférée de manière stable, dans le génome de la cellule. Par génome de la cellule, on entend aussi bien le génome nucléaire que le génome chloroplastique ou mitochondrial. De préférence, il s'agit du génome nucléaire.

La cellule peut être tout type de cellule, procaryote ou eucaryote, bien que les cellules eucaryotes soient préférées dans le cadre de la présente invention. Une cellule selon l'invention est également de préférence une cellule végétale, mais il peut également s'agir d'une cellule bactérienne, d'une cellule animale, par exemple de mammifère ou de tout autre type de cellule, par exemple une cellule de levure. De préférence, il s'agit d'une cellule de plante d'intérêt agronomique.

La présente invention concerne également une plante transgénique comprenant dans son génome une séquence selon l'invention, ladite séquence étant de nature exogène. On entend par « plante transgénique » et « séquence exogène » que la séquence selon l'invention a été transférée de manière délibérée à la plante et que ladite séquence n'était pas présente naturellement auparavant dans la plante.

Une plante transgénique selon l'invention peut également comprendre un construit tel que décrit, notamment un construit comprenant une séquence à transcrire hétérologue par rapport à une séquence promotrice selon l'invention.

Une plante transgénique selon l'invention comprend donc dans son génome une séquence (ou un construit) de l'invention ; de préférence, ladite séquence (ou construit) est insérée dans le génome nucléaire de toute cellule de la plante, mais l'invention comprend également les situations dans lesquelles la séquence est insérée dans le génome mitochondrial ou dans le génome chloroplastique. Il est également possible de maintenir la séquence ou le construit selon l'invention de manière extrachromosomale.

La séquence (ou le construit comprenant ladite séquence) est insérée de préférence de manière stable, bien qu'il soit également envisageable de l'insérer de manière transitoire.

La présente invention concerne également des plantes transgéniques comprenant des cellules transformées comme décrites précédemment.

De préférence, la totalité des cellules d'une plante transgénique selon l'invention comprend une séquence ou un construit selon l'invention. Il est également envisageable que seules certaines parties de ces plantes comprennent de telles cellules transformées, par exemple lorsqu'il s'agit de plantes chimériques ou du fait de l'excision du transgène dans certaines cellules.

L'invention porte aussi sur des parties desdites plantes transgéniques. Des parties particulièrement intéressantes sont notamment les fruits, les fleurs, les racines, les tiges, les feuilles, mais également les semences, les bourgeons, les graines et le matériel de reproduction, dont le matériel de reproduction mâle et le matériel de reproduction femelle, ainsi que le cal, lesdites parties selon l'invention ayant des cellules transformées comprenant une séquence ou un construit selon l'invention. De préférence, elles comprennent un construit comprenant une séquence à transcrire hétérologue par rapport à une séquence promotrice selon l'invention. Lesdites parties sont donc également transgéniques.

Une plante transgénique selon l'invention peut être tout type de plantes. Il peut s'agir d'une plante monocotylédone ou bien dicotylédone. De préférence, une plante transgénique présente un intérêt agronomique, il peut s'agir notamment d'une plante céréalière, d'une plante maraîchère ou potagère, d'un arbre fruitier. De préférence, il s'agit d'une plante autre que le céleri.

Parmi les plantes, les plantes des familles suivantes sont particulièrement préférées : les plantes de la famille des Cucurbitacées, Chénopodiacées, Cruciféracées, Poacées, Légumineuses, Apiacées, Rosacées, Valérianacées, Solanacées et Astéracées.

Des exemples particulièrement préférés de plantes transgéniques selon l'invention sont la plante de tomate, la plante de melon et la laitue. D'autres plantes préférées sont le céleri, l'oignon, la betterave, le brocoli, le blé, l'asperge, le maïs doux et le colza.

Une plante transgénique selon la présente invention peut également contenir dans son génome d'autres transgènes, indépendamment de la séquence selon l'invention, il peut s'agir notamment d'un gène de résistance à une infection virale sous le contrôle d'un promoteur constitutif. Dans ce cas, les différentes transformations peuvent avoir été réalisées simultanément, au cours d'une unique étape de transformation, ou bien séquentiellement.

Une plante transgénique selon l'invention peut avoir été régénérée à partir de cellules transformées. Il est également possible d'obtenir une plante selon l'invention par la descendance d'une autre plante transgénique selon l'invention.

La présente invention concerne également une plante transgénique comprenant dans son génome une séquence d'acides nucléiques comprenant tout ou partie de la SEQ ID N°1, telle que ladite séquence présente une activité de promoteur transcriptionnel, ladite partie comprend au moins 30 nucléotides consécutifs de la SEQ ID N°1, et ladite séquence est en association fonctionnelle avec une séquence codante hétérologue et exprimant ladite séquence codante de façon spécifique dans les racines. De préférence, ladite plante appartient à la famille des Apiacées, Astéracées, Brassicacées (ou Crucifères), Chénopodiacées, Cucurbitacées, Poacées, Rosacées, Solanacées, Valérianaceés ou Légumineuses.

La présente invention concerne également une plante transgénique comprenant dans son génome une séquence d'acides nucléiques comprenant tout ou partie de la SEQ ID N°1, 2 ou 3 telle que ladite séquence présente une activité de promoteur transcriptionnel, ladite partie comprend au moins 30 nucléotides consécutifs de la SEQ ID N°1, 2 ou 3, et ladite séquence est en association fonctionnelle avec une séquence codante hétérologue et exprimant ladite séquence codante de façon spécifique dans le phloème. Dans une mise en oeuvre particulière, ladite séquence est la SEQ ID 2 ou une partie de la SEQ ID 2 comprenant au moins 30 nucléotides consécutifs, et ladite plante appartient à la famille des Apiacées, des Astéracées, des Brassicacées, des Chénopodiacées, des Convolvulacées, des Cucurbitacées, des Fabacées, des Grossulariacées, des Lamiacées, des Liliacées, des Poacées, des Polygonacées, des Rosacées, des Solanacées ou des Valérianacées. Dans une autre mise en oeuvre particulière, ladite séquence est la SEQ ID 1 ou 3 ou une partie de la SEQ ID 1 ou 3 comprenant au moins 30 nucléotides consécutifs, ladite plante appartient à la famille des Brassicacées, et l'expression de ladite séquence codante est induite par un stress biotique ou abiotique. Dans une mise en oeuvre préférée, ledit stress biotique ou abiotique est un stress salin.

La présente invention porte également sur un procédé permettant d'obtenir une plante transgénique selon l'invention. Un tel procédé comprend les étapes suivantes :
- l'obtention d'un construit selon l'invention tel que décrit précédemment,
- L'introduction du construit dans une cellule provenant d'une plante d'intérêt et
- la régénération d'une plante transgénique, à partir des cellules transformées.

Un procédé selon l'invention peut bien entendu comprendre de nombreuses autres étapes, précédant ou suivant les étapes mentionnées. Un procédé selon l'invention peut avantageusement également comprendre une étape supplémentaire de croisement de la plante transgénique obtenue avec d'autres plantes, également transgéniques ou non. Des croisements additionnels peuvent bien entendu être réalisés. On peut également réaliser des étapes de multiplication, sexuelle ou asexuelle, en fonction de l'espèce, et obtenir ainsi de la descendance.

De préférence, la descendance obtenue à l'issue du procédé est examinée afin de déterminer les plantes de la descendance qui comprennent une séquence ou un construit selon l'invention. Les plantes comprenant une séquence ou un construit selon l'invention peuvent aussi être isolées. De telles plantes, qui comprennent dans leur génome une séquence ou un construit selon l'invention et qui sont issues du procédé tel que décrit, font également partie de l'invention. De préférence, les plantes issues de la descendance et qui comprennent une séquence ou un construit selon l'invention sont déterminées par une étape de sélection. Ladite sélection peut être une sélection en champ ou en serre, ou bien une sélection génétique à l'aide de marqueurs génétiques.

La présente invention concerne également l'utilisation d'une séquence ou d'un construit selon l'invention, pour la réalisation de plantes transgéniques, c'est-à-dire dans le domaine du génie génétique.

En effet, comme explicité plus haut, une application de la présente invention est l'utilisation de séquences promotrices de l'invention positionnées en amont d'une séquence codante d'intérêt (transgène), afin que la protéine codée par la séquence d'intérêt ne soit exprimée que dans des conditions spécifiques de stress, et préférentiellement dans certains organes vasculaires de la plante transgénique. Par ce biais, le transgène est peu ou pas exprimé dans des conditions normales et son expression est induite uniquement en conditions de stress. Cette caractéristique de l'invention est d'un grand intérêt puisqu'il devient possible de n'exprimer certains transgènes, notamment des gènes de résistance, que lorsque cela s'avère nécessaire, c'est-à-dire en condition de stress. En effet, l'expression constitutive de certains transgènes est parfois inutile, voire néfaste en conditions normales. Grâce à la présente invention, il est donc possible de n'exprimer des transgènes que dans des conditions de stress.

Dans le cadre de l'invention, les inventeurs ont également découvert une nouvelle protéine, à savoir un nouveau transporteur de mannitol chez le céleri *Apium graveolens,* dénommé AgMaT3. Par conséquent, la présente demande concerne également un peptide comprenant une séquence qui présente au moins 70% d'identité avec la séquence SEQ ID N°8, de préférence au moins 80% d'identité, voire 90% ou même 95% d'identité avec ladite séquence SEQ ID N°8. Des peptides préférés selon cet aspect de l'invention sont des peptides comprenant ou consistant en la séquence SEQ ID N°11. D'autres peptides préférés sont ceux qui sont codés par tout ou partie de la séquence SEQ ID N°7, 13 ou 14, ou bien par une séquence issue de la séquence SEQ ID N°7, 13 ou 14 du fait de la dégénérescence du code génétique.

De préférence, une telle séquence d'acides aminés selon cet aspect de l'invention possède la capacité de transporter le mannitol à travers une bicouche lipidique, notamment dans des cellules végétales.

La demande concerne également les séquences d'acides nucléiques codant pour les peptides tels que décrits précédemment. Selon une mise en oeuvre possible, ladite séquence est en aval d'un promoteur selon l'invention, par exemple SEQ ID N°1.

### Légende des figures

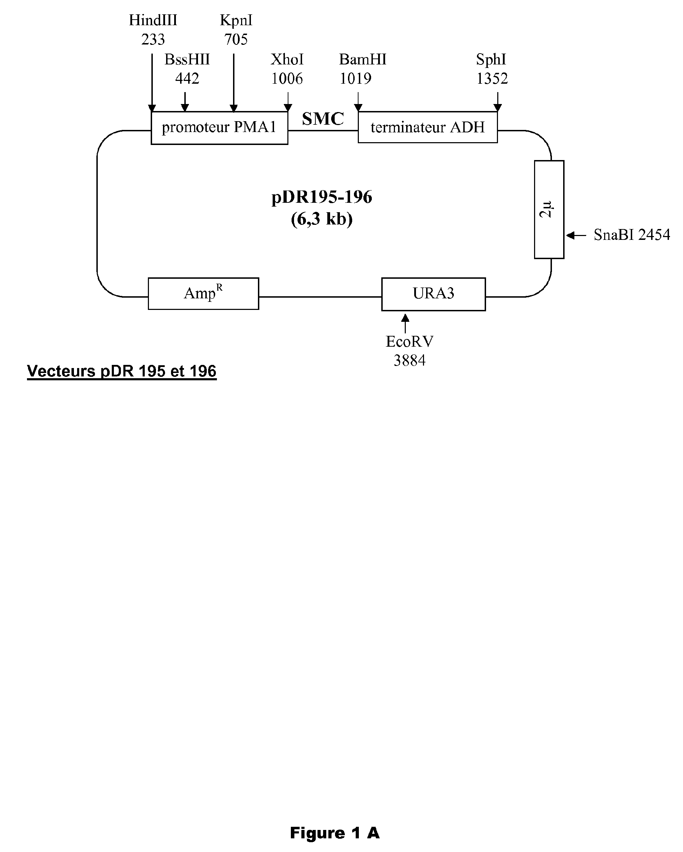

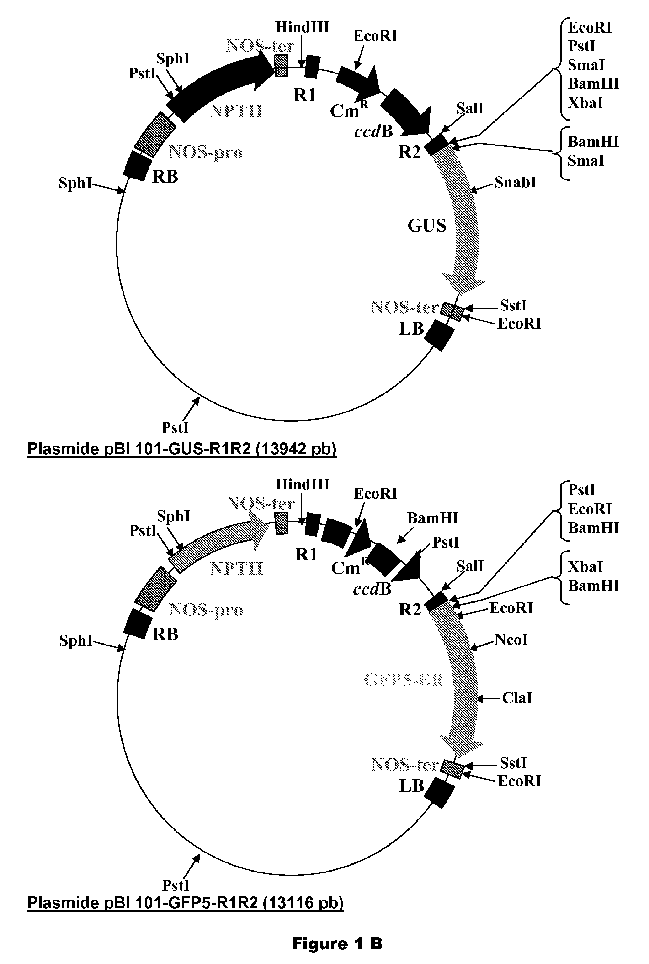

**Figure 1**: Cartes des vecteurs de clonage utilisés
   **1A**: Vecteurs pDR 195 et 196
      Site Multiple de Clonage de pDR 195 : Xhol-Notl-Sacll-BamHl
      Site Multiple de Clonage de pDR 196 : **Spel-BamHl*-Smal-Pstl-EcoRI**-EcoRV-Hindlll-**Sall-Xhol-**Acc65l-Kpnl-**BamHl***
      En gras sont représentées les enzymes qui ne coupent pas ailleurs dans le plasmide et avec *, celles qui coupent deux fois dans le SMC.
   **1B** : Plasmide pBI 101-GUS-R1R2 (13942 pb) et Plasmide pBI 101-GFP5-R1R2 (13116pb)

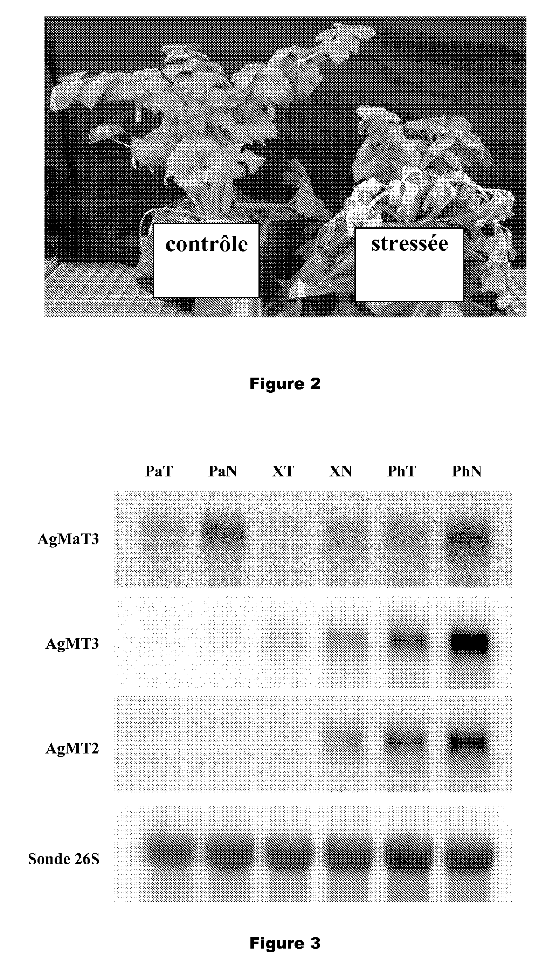

**Figure 2** : Céleris entretenus pendant trois semaines avec de l'eau ou une solution d'engrais (plante témoin) et avec 300 mM NaCl (plante stressée).
**Figure 3** : *Hybridations en Northern d'ARN de parenchyme de stockage, xylème et phloème de pétioles de plantes "témoins" ou "stressées" au NaCl (300 mM,* 3 *semaines) par des sondes produites à partir des séquences obtenues par la banque soustractive.* La quantification a été réalisée grâce à la sonde ribosomique 26S. Abréviations : PaT, Parenchyme Témoin; PaN, Parenchyme NaCl; XT, Xylème Témoin; XN, Xylème NaCl; PhT, Phloème Témoin; PhN, Phloème NaCl.

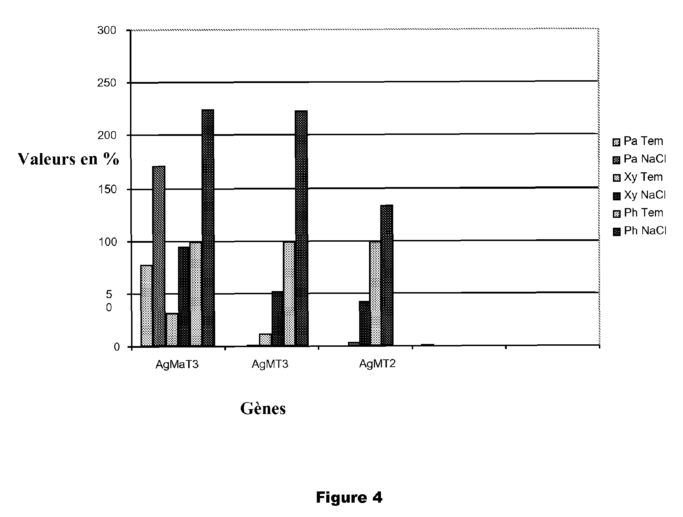

**Figure 4** : *Représentation graphique du niveau d'expression des sondes testées lors de l'hybridation en Northern, en fonction du tissu.* Abréviations: AgMaT3, *Apium graveolens* Mannitol Transporter 3; AgMT2 et 3, *Apium graveolens* métallothionéines 2 et 3.

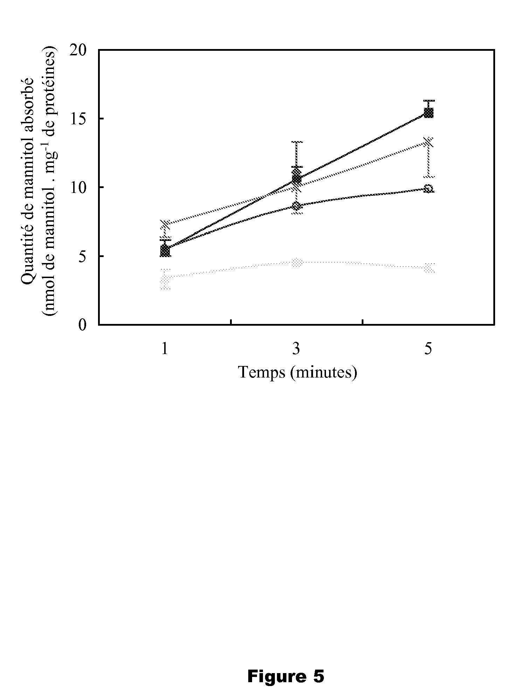

**Figure 5** : *Absorption de mannitol (0,55 mM) par les levures RS453 complémentées par pDR (*▲*), ou par AgMaT1*/*pDR (*◆*), ou bien par trois clones indépendants* AgMaT3/*pDR (*■, O et **x***)*, *en fonction du temps.* La cinétique réalisée avec la souche transformée par le plasmide vide sert de contrôle. Les valeurs correspondent à la moyenne ± erreur-standard d'une même expérience avec trois répétitions par point.

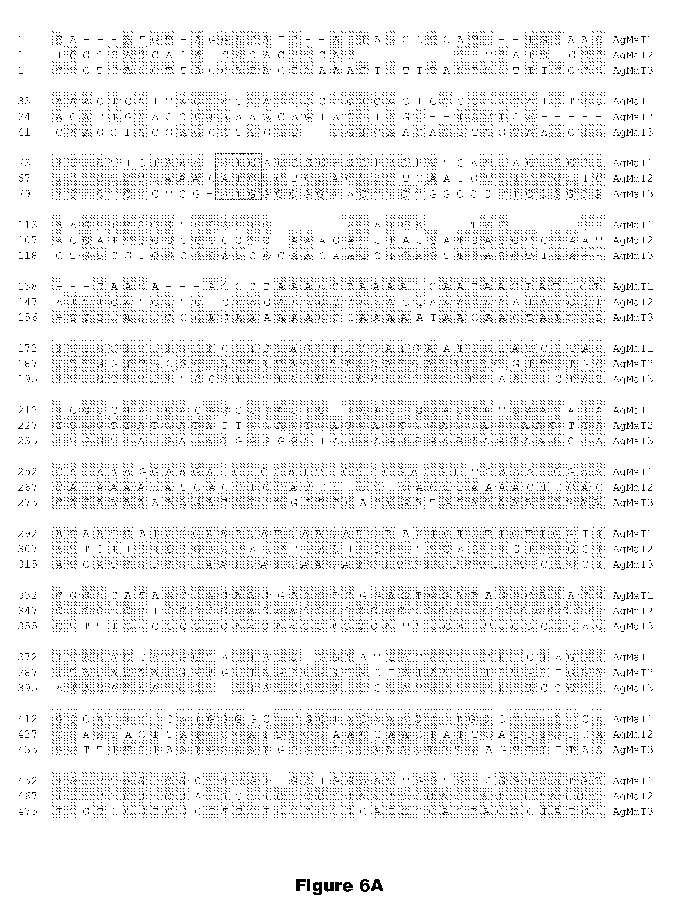

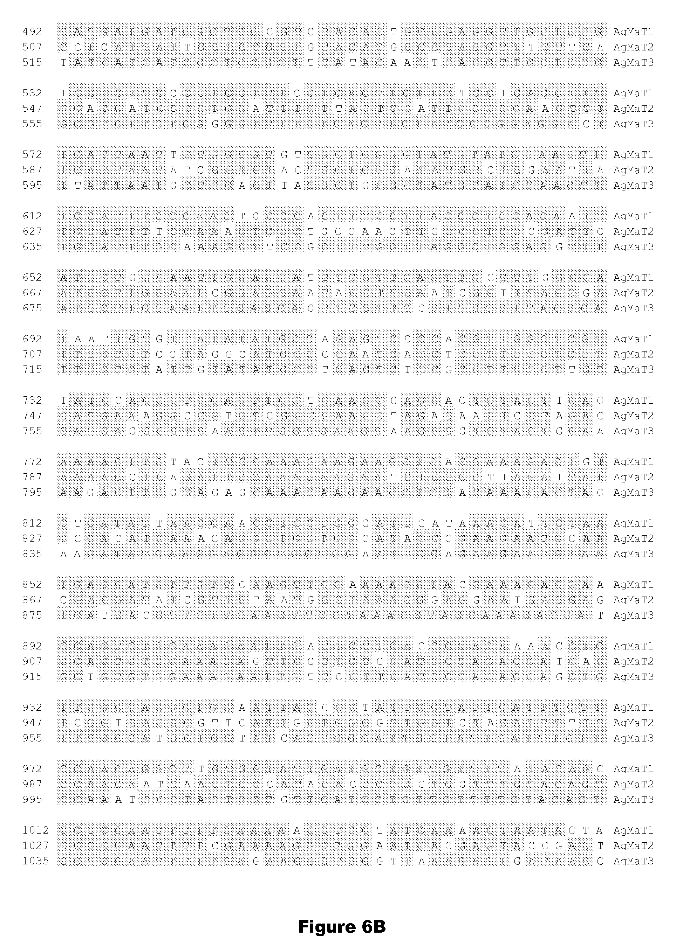

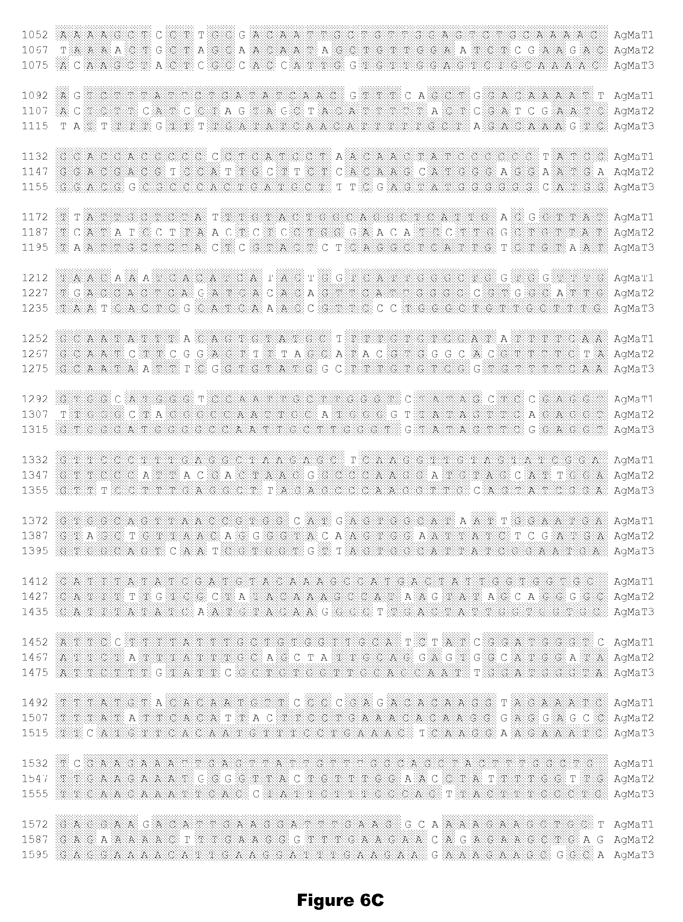

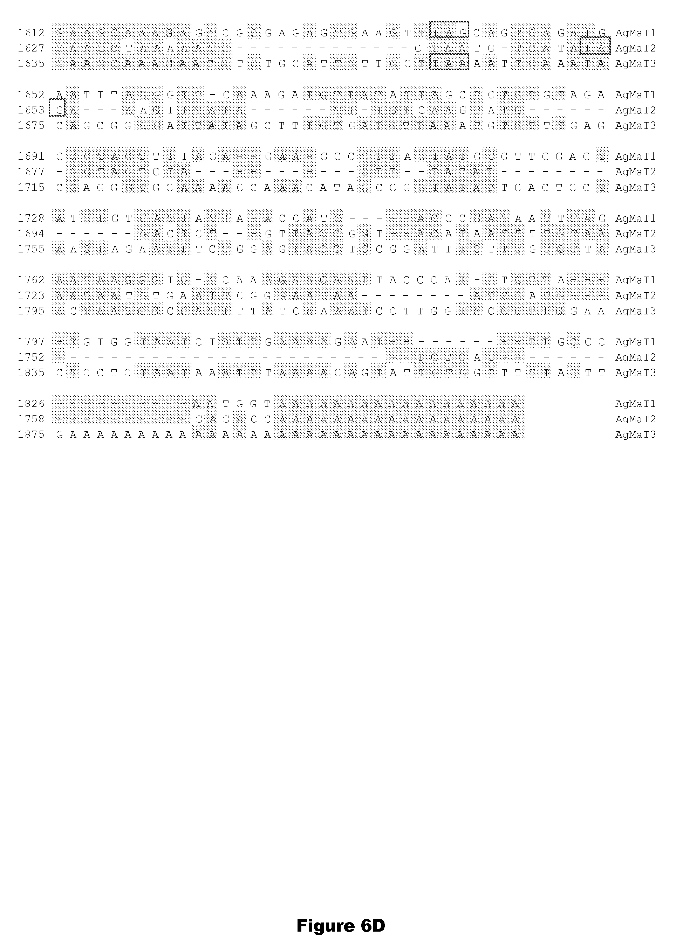

**Figure 6** : *Alignement des* séquences *nucléotidiques des ADNc d'AgMaT 1, 2 et 3.* Les séquences ont été alignées par la méthode Clustal du programme MEGALIGN (DNAstar, Madison, WI). Les résidus identiques à la séquence consensus ont été surlignés. Les codons ATG et stop sont encadrés.

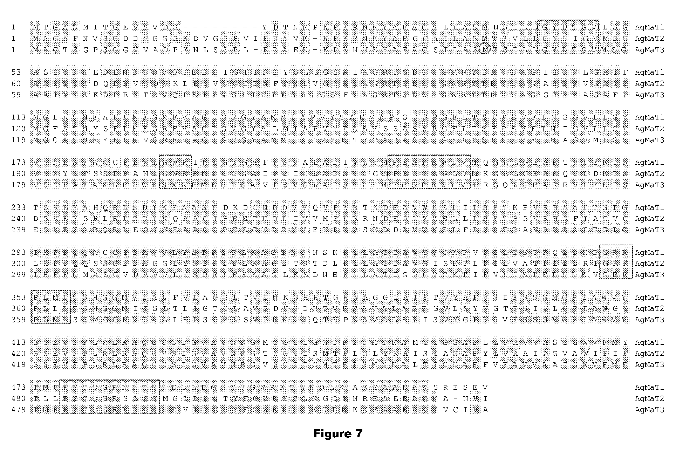

**Figure 7** : *Alignement des* séquences *protéiques d'AgMaT 1, 2 et 3.* Les séquences déduites en acides aminés ont été alignées par la méthode Clustal du programme MEGALIGN (DNAstar, Madison, WI). Les résidus identiques à la séquence consensus ont été surlignés. Les résidus encadrés correspondent aux séquences conservées entre les transporteurs de "sucre" de la famille des MFS (selon NCBI Conserved Domain Search, www.ncbi.nlm.nih.gov/Structure/cdd/wrpsb.cgi). La méthionine entourée correspond au début de la séquence protéique initialement clonée.

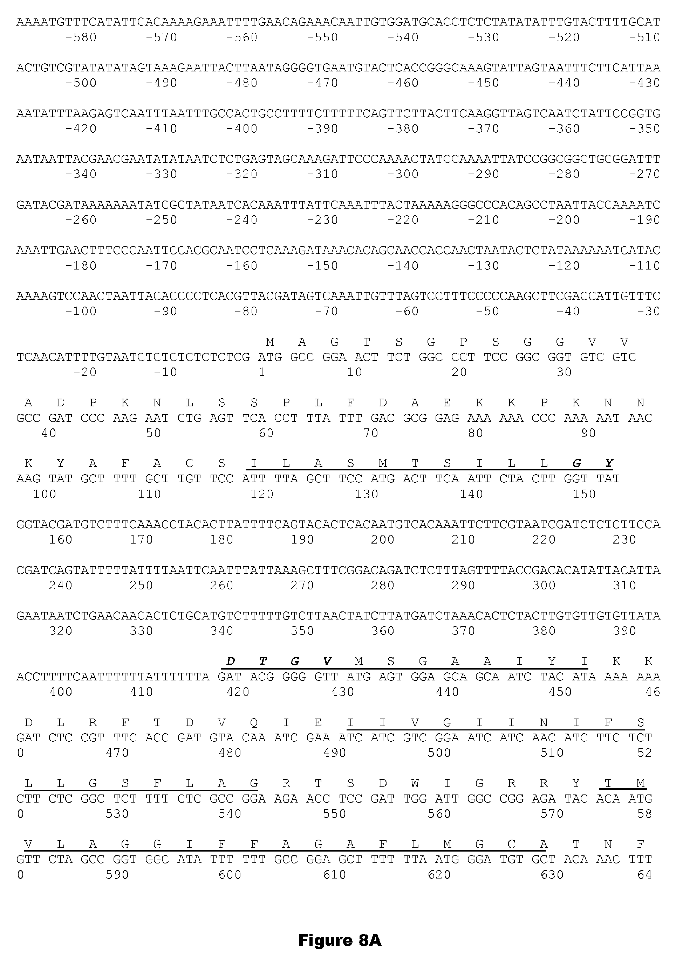

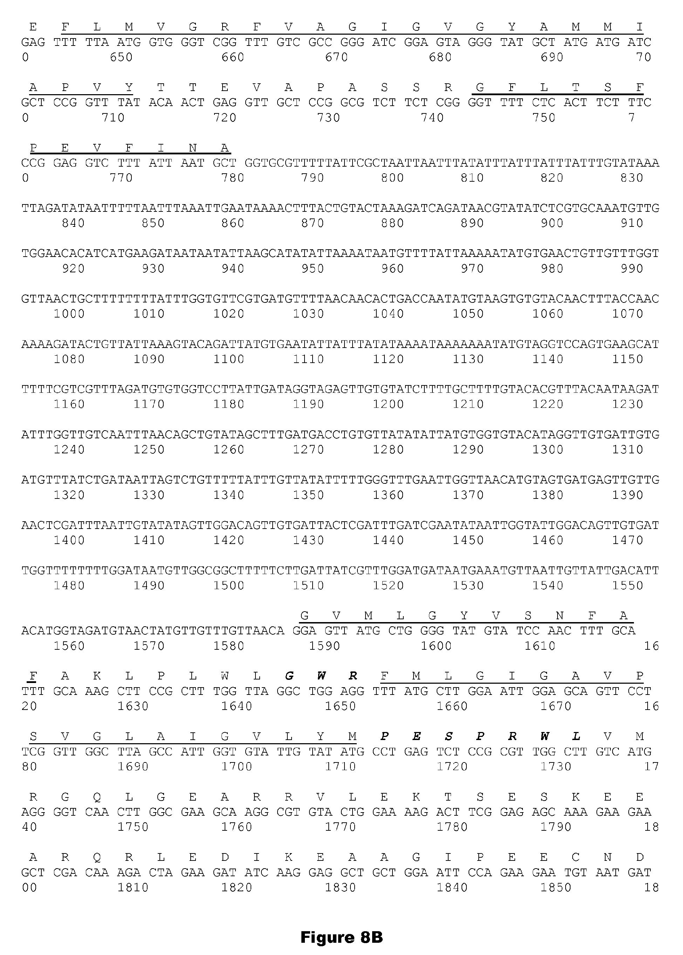

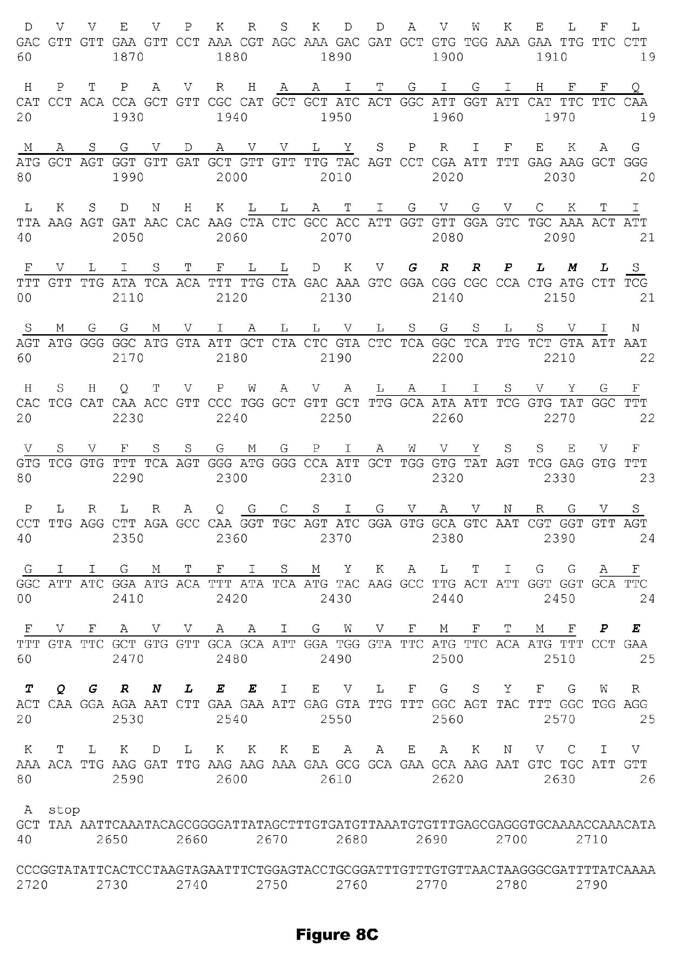

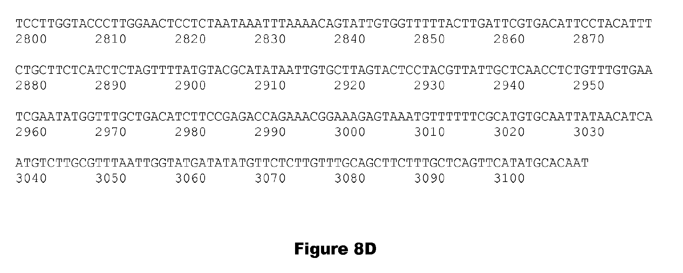

**Figure 8** : *Séquence promotrice et génomique d'AgMaT3 et traduction de la région codante.* La séquence peptidique prédite est donnée en utilisant l'abréviation à une lettre. Les résidus en italique correspondent aux séquences conservées de la sous-famille des transporteurs de "sucre" des MFS. Les hélices transmembranaires putatives sont soulignées. La numérotation est basée sur le site d'initiation de la traduction.

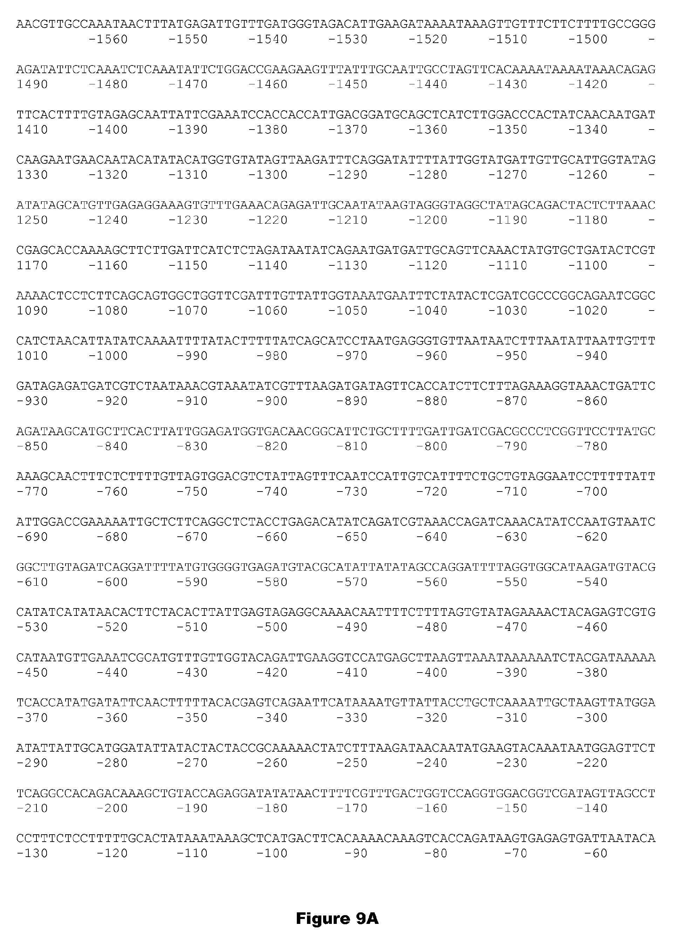

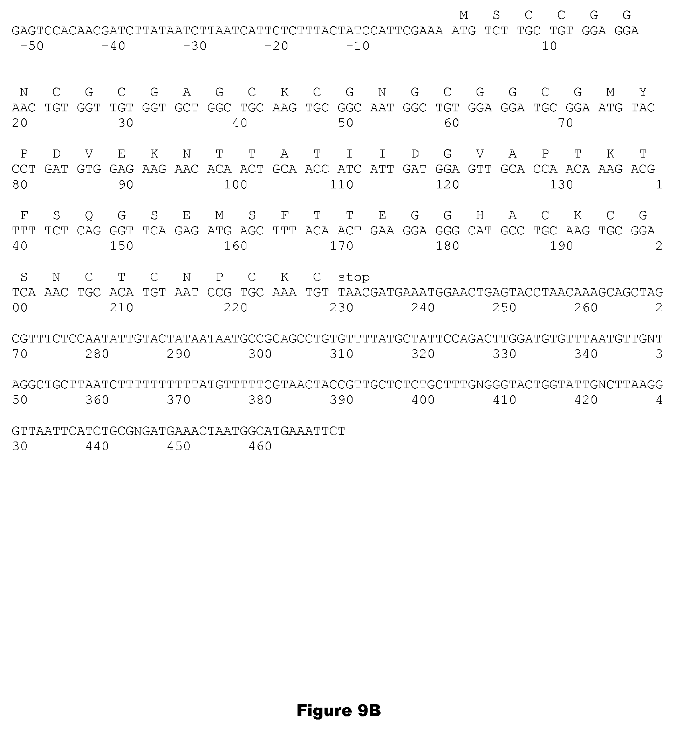

**Figure 9** : *Séquence promotrice d'AgMT2 et traduction de la région codante.* La séquence peptidique prédite est donnée en utilisant l'abréviation à une lettre. La numérotation est basée sur le site d'initiation de la traduction.

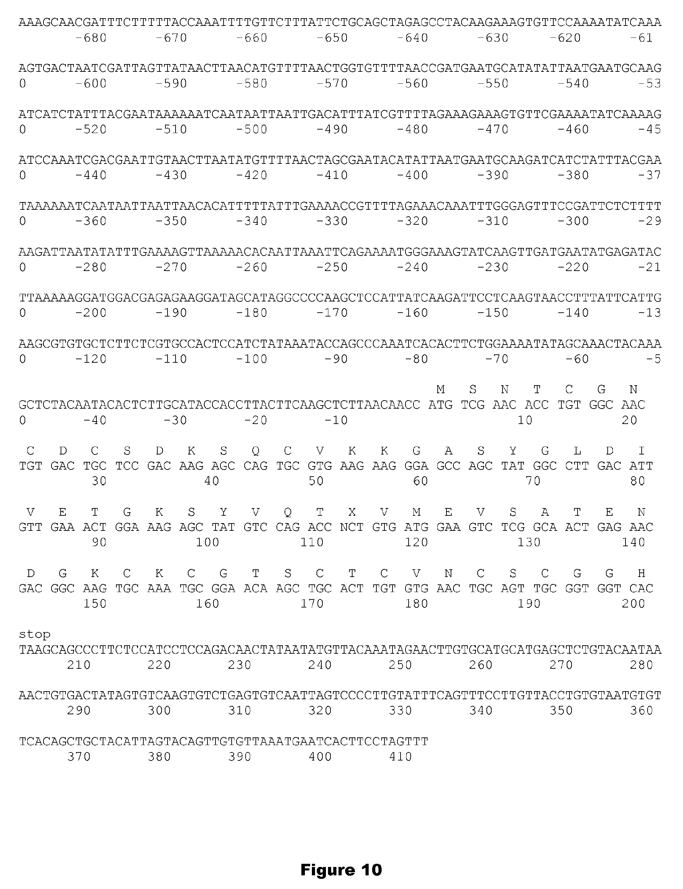

**Figure 10** : *Séquence promotrice d'AgMT3 et traduction de la région codante.* La séquence peptidique prédite est donnée en utilisant l'abréviation à une lettre. La numérotation est basée sur le site d'initiation de la traduction.

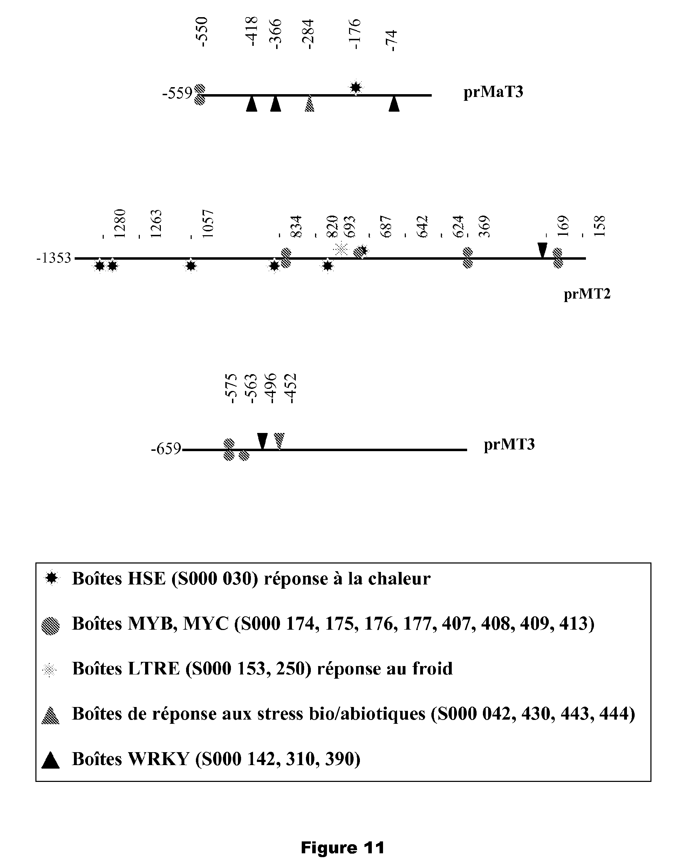

**Figure 11** *: Emplacement de certains éléments* cis *(identifiés par analyse informatique) sur les régions promotrices d'AgMaT3 et AgMT 2 et 3, utilisées pour les constructions.* Les positions des motifs sont indiquées à partir du site d'initiation de la traduction. Les éléments placés sur le brin complémentaire (-) sont représentés sous la ligne.

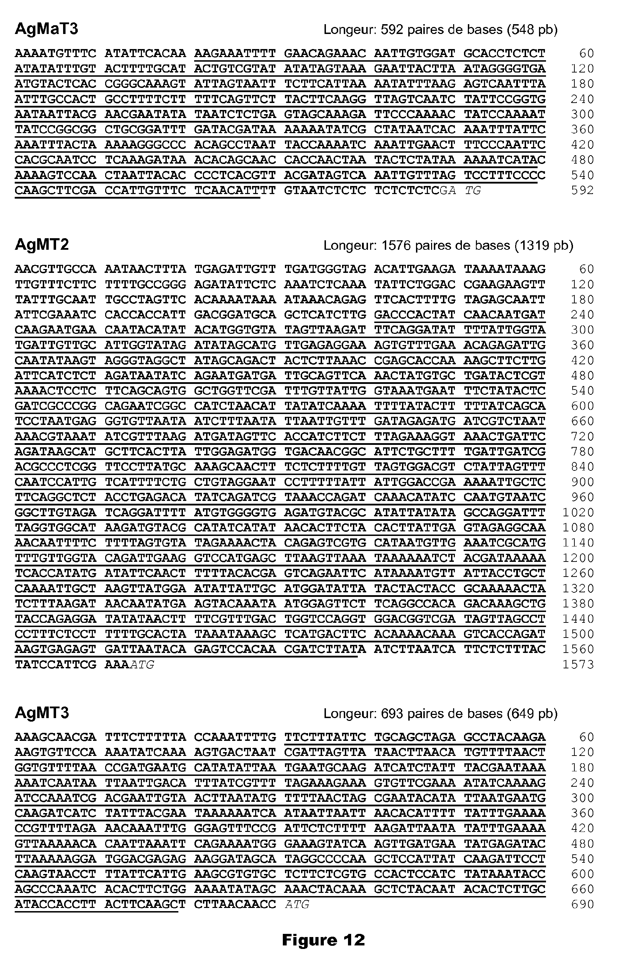

**Figure 12** : Séquence des régions amont 5' utilisées comme promoteurs. Les séquences sont représentées jusqu'au premier ATG correspondant au site d'initiation de la traduction. Les régions clonées en amont des gènes rapporteurs sont soulignées et leur longueur est indiquée entre parenthèse. Les séquences SEQ ID N°1 (AgMaT3), SEQ ID N°2 (AgMT2) et SEQ ID N°3 (AgMT3) correspondent aux séquences soulignées, les séquences SEQ ID N° 4 (AgMaT3), SEQ ID N°5 (AgMT2) et SEQ ID N°6 (AgMT3) correspondent aux séquences en gras ; elles se terminent juste avant le 'A' de l'ATG.

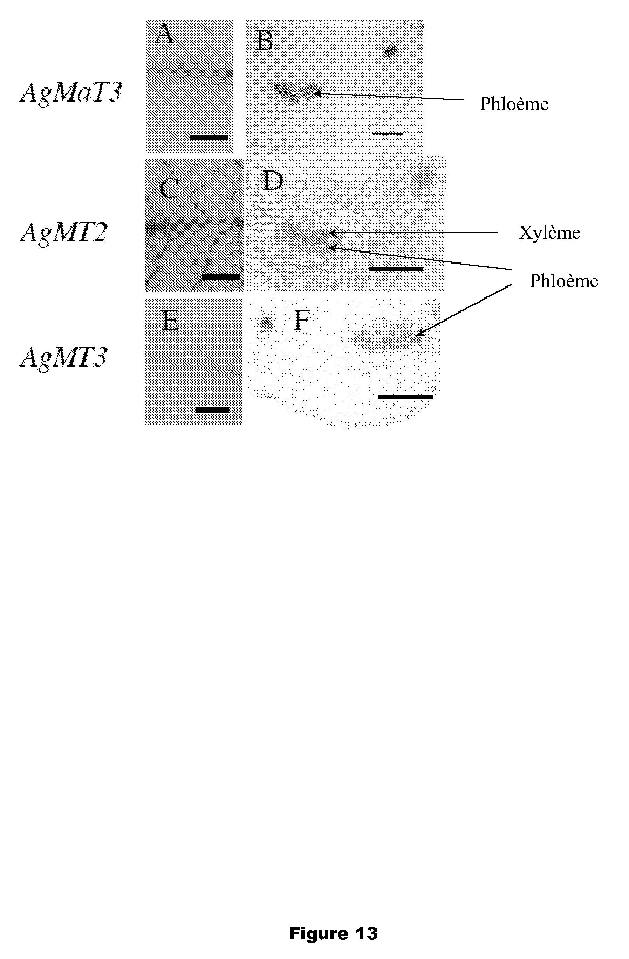

**Figure 13** : coloration GUS des feuilles matures de plantes *Arabidopsis* exprimant les constructions suivantes: *AgMaT3 ::uiad* (A, B), *AgMT2 ::uiad3* (C, D), *AgMT3 ::uiad3* (E, F). Les photographies représentent soit les feuilles entières (A, C, E, barre d'échelle 5mm) soit des sections transversales (B, D, F, barre d'échelle 50µm). Toutes les photographies ont été prises à partir de plantes après un stress salin.

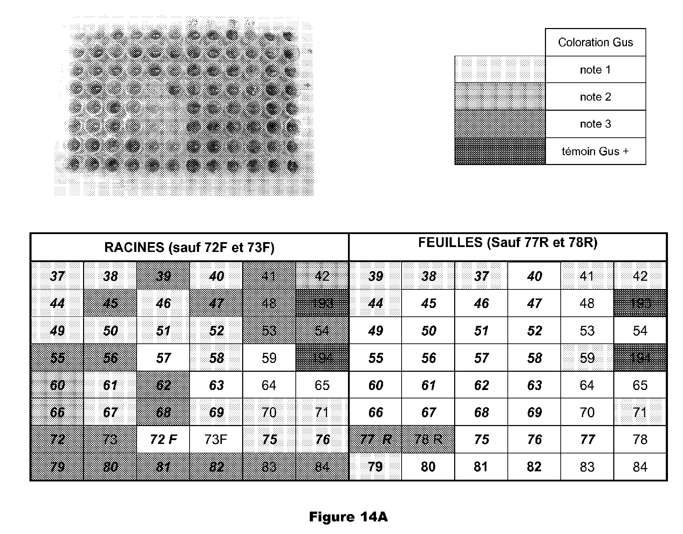

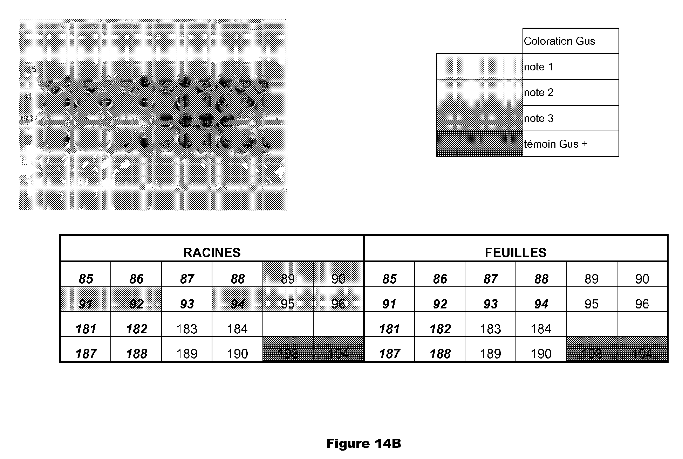

**Figure 14** : Résultats du test Gus sur fragments racinaires et foliaires des plantes (tomates Kemer) transformées avec le construit pAgMat3-GUS-tNOS. a) 1^{ère} Plaque : plantes 37 à 84 et témoins ; b) 2^{ème} Plaque : plantes 85 à 96 et témoins. Les plantes soumises à un stress salin (50mM pendant 4 jours) sont mentionnées en italique gras. Le niveau de coloration Gus est noté de 0 à 3. Un contrôle positif GUS sous contrôle d'un promoteur constitutif et un contrôle de plantes KEMER non transformées sont utilisés en tant que témoins.

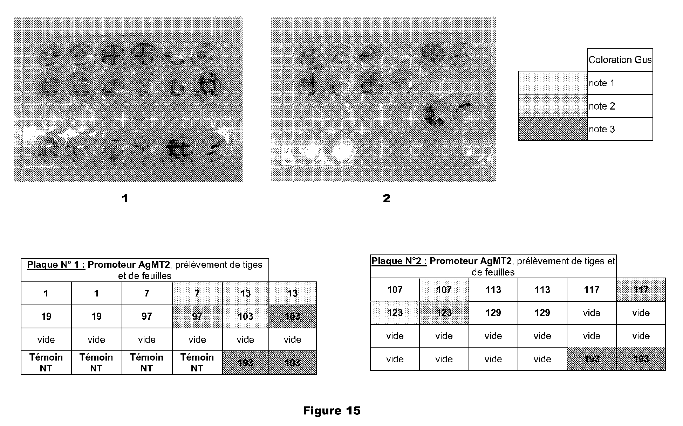

**Figure 15** : Résultats du test Gus sur des fragments de tiges et feuilles de six plantes (tomates Kemer) transformées avec le construit pAgMT2-GUS-tNOS. Le niveau de coloration Gus est noté de 0 à 3. Un contrôle positif GUS sous contrôle d'un promoteur constitutif et un contrôle de plantes KEMER non transformées sont utilisés en tant que témoins.

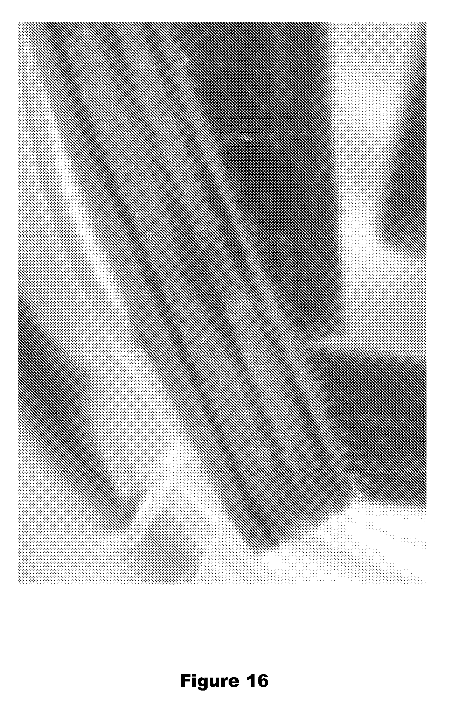

**Figure 16** : analyse histo-cytologique de fragments Gus positifs de plantes (tomates Kemer) transformées avec le construit pAgMT2-GUS-tNOS et observation de la coloration au niveau des tissus vasculaires. La lecture a eu lieu 4 jours après le test. Les premières observations montrent une coloration bleue plutôt concentrée sur les tissus vasculaires de la tige, dans certains cas, uniquement sur les nervures des feuilles.

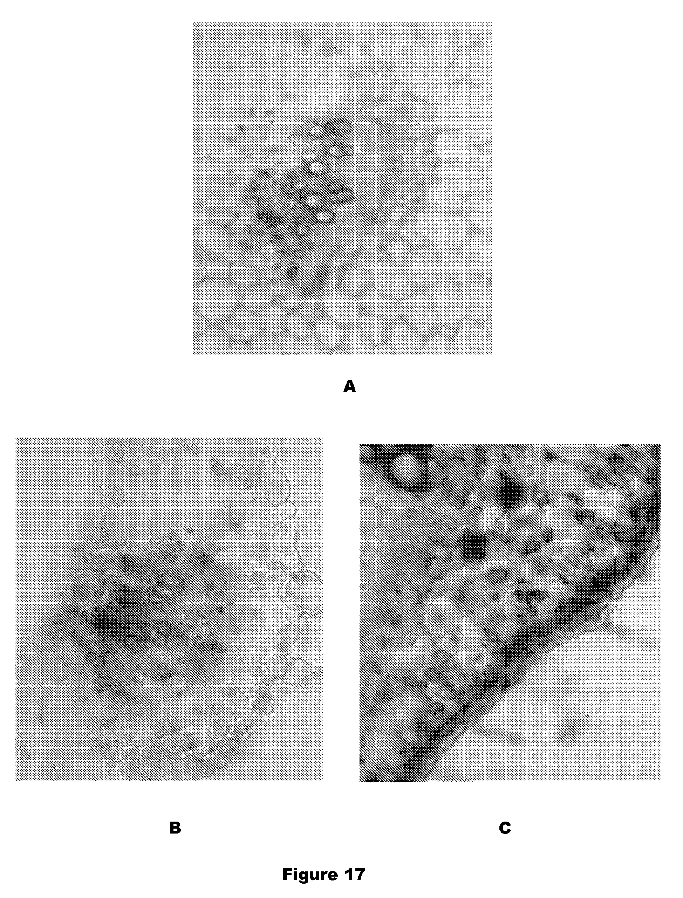

**Figure 17** : coupes tissulaires de plantes (tomates Kemer) transformées avec le construit pAgMT2-GUS-tNOS. Coupes tissulaires de la plante 13 au niveau de la tige (A) et de la plante 103 au niveau de la feuille (B) et de la tige (C). Ces données cytologiques indiquent une expression au niveau des tissus phloémiens.

### Partie expérimentale

L'équipe de Bohnert *et al.* (2001) a mené une étude génomique de grande échelle sur différents organismes (Riz, *Mesembryanthemum crystallinum* L., *Arabidopsis,* Maïs, Tabac et Orge) en réponse au stress salin, sur différents tissus (principalement racines et feuilles) à différentes étapes du développement et dans des conditions très variables (concentrations et durée). Cette étude a été poursuivie par des stratégies de mutagénèse insertionnelle et de recherches de QTL (quantitative trait loci), réalisées sur le Riz pour identifier les caractères permettant à cette espèce d'être tolérante au stress salin (Koyama *et al.,* 2001). Des recherches globales ont également été réalisées sur le transcriptome *d'Arabidopsis thaliana* traité au NaCl (Kreps *et al.,* 2002) et sur le protéome de Riz (Salekdeh *et al.,* 2002) en réponse à une salinisation modérée (100-150 mM NaCl). Ces techniques qui étudient l'ensemble des gènes et protéines exprimés chez la plante lors du stress salin ne sont pleinement exploitables que lorsque l'ensemble du génome de l'espèce étudiée est disponible, ce qui n'est pas le cas du céléri. L'équipe de Masmoudi *et al.* (2001) a recherché par expression différentielle, des ADNc de racines du Blé dont l'expression est modifiée par un traitement avec 200 mM de NaCl. La technique utilisée est beaucoup plus ciblée (pour étudier les gènes induits dans un organe particulier) mais ne permet pas toujours d'identifier de nombreux gènes.

Les présents inventeurs ont donc choisi d'utiliser la technique de banque soustractive permettant à la fois de cloner les gènes spécifiquement induits par le stress salin, et d'obtenir un grand nombre d'ADNc, beaucoup plus représentatifs de l'état transcriptionnel de la plante, lors de cette contrainte environnementale.

De plus, il n'y a pas ou peu d'informations sur les localisations tissulaires des différents gènes impliqués dans la tolérance au sel. Le phloème, quant à lui, est un tissu clé dans la redistribution du Na⁺ entre les différents organes (Lohaus *et al.,* 2000). Il intervient également dans le transport d'osmolytes vers les tissus accumulateurs cibles (Popova *et al.,* 2003). Le myo-inositol et l'ABA sont transportés rapidement dans les tubes criblés, agissant comme des signaux descendants (des feuilles jusqu'aux racines) et activant de nombreux mécanismes nécessaires à la mise en place de la tolérance au stress salin (Nelson *et al.,* 1999). Il est reconnu que le phloème joue un rôle important dans la délivrance de molécules informatives jusqu'à des organes localisés à distance (Ruiz-Medrano *et al.,* 2001 Il apparaissait donc intéressant d'étudier l'expression de gènes lors d'un stress salin dans ce tissu complexe. Les inventeurs ont travaillé sur une banque soustractive construite à partir de phloème de plantes ayant subit un stress salin et de phloème d'une plante témoin (en hydratation normale).

La plante la mieux adaptée pour cette étude s'est avérée être le Céleri, modérément sensible à un excès de macroéléments ou un stress salin (De Pascale *et al.,* 2003). Cette tolérance a été mise en relation avec une accumulation de mannitol (osmolyte) aux dépens du saccharose par modification des activités enzymatiques, la mannose-6-phosphate réductase et la mannitol déshydrogénase (Noiraud *et al.,* 2000). Le céleri a un autre avantage car le tissu phloèmien (cellules du parenchyme phloèmien + complexe conducteur) peut être aisément isolé, microchirurgicalement, du reste du faisceau conducteur (Noiraud, 1999). C'est donc la variété Céleri branche *Apium graveolens* L. *Dulce* (cultivar Vert d'Elne) qui a été utilisée par les inventeurs.

L'objectif est l'identification de promoteurs de gènes induits dans le phloème par un stress salin chez le Céleri par la technique de banque soustractive.

### Exemple 1 : matériel et méthodes

### I. Matériel végétal

### 1. A. Céleri branche

Les pieds de Céleri branche utilisés sont semés et cultivés sur du terreau en serre. Les plantes sont arrosées automatiquement pendant 90 sec toutes les 3 h durant la journée et reçoivent une solution d'engrais (solution Peters 20:20:20 contenant N, P et K à 100 ppm), trois fois par semaine. Une fois par semaine, une pulvérisation du coeur et des jeunes feuilles doit être effectuée avec du nitrate de calcium à 4 g.L⁻¹ en alternance avec du Cosynol SC à 10 g.L⁻¹, afin d'éviter le pourrissement du Céleri (maladie du coeur brun).

### I. B. Stress salin (Céleri)

Lors de l'application d'un stress salin, deux lots de Céleri branche sont préparés : plantes "témoins" (T) et plantes "stressées" (S). Les pots de 5.4 L sont recouverts d'un film plastique noir afin de limiter l'évaporation. Le stress salin débute à une concentration de 25 mM de NaCl, puis augmente par palier de 25 mM lors de chaque apport (deux fois 500 mL par pot et par jour, matin et soir) en eau ou en engrais jusqu'à atteindre la concentration désirée soit 300 mM NaCl. Les plantes "témoins" sont arrosées de la même façon, mais sans apport de sel. Le stress salin est appliqué pendant trois semaines. Engrais et calcium sont appliqués avec la même fréquence que sur les plantes témoins.

### I. C. Récolte et conservation des tissus et organes

Après 3 semaines de stress salin, les tissus sont récoltés selon un critère déterminé (âge ou taille) et conservés. Une foliole, les parenchyme, phloème, xylème des pétioles correspondant à la foliole et les racines sont prélevés sur plusieurs plantes et réunis par lots selon le critère établi avant d'être congelés dans de l'azote liquide et conservés à -80 °C.

### I. D. Culture d'Arabidopsis thaliana

L'arabette est utilisée pour la transformation végétale par les séquences promotrices des différents gènes étudiés. Les plantes *d'Arabidopsis thaliana* d'écotype *Columbia* (Col-0) sont semées et cultivées sur du terreau en serre, en condition de jours courts (jour ≈ 21°C, 8h et nuit ≈ 17°C, 16h) pour le développement des rosettes, puis en jours longs (jour ≈ 21°C, 16h et nuit ≈ 17°C, 8h) pour l'induction de la floraison, jusqu'à la montée de la hampe florale. Les plantes sont arrosées automatiquement, dans leurs soucoupes, pendant 90 sec toutes les 3h durant la journée et reçoivent une solution d'engrais (solution Peters 20:20:20 contenant N, P et K à 100 ppm), trois fois par semaine.

### II. Matériel biologique pour les approches moléculaires

### II. A. Microorganismes hôtes

### II. A. 1. Souche de bactéries

La souche bactérienne *Escherichia coli* DH5α (GibcoBRL) est utilisée pour le clonage et l'amplification d'ADN et des fragments de PCR. Ces bactéries sont cultivées à 37 °C, avec du milieu dYT solide ou liquide, en présence de l'antibiotique approprié ; ampicilline à 100 µg.mL⁻¹, kanamycine à 100 µg.mL⁻¹ ou gentamycine à 50 µg.mL⁻¹.

### II. A. 2. Souche de levures

La souche de levures RS453 (Sauer et Stadler, 1993) est incapable de se développer sur un milieu sans uracile. Le plasmide pDR portant le gène *URA3* est utilisé pour transformer cette souche. Cette souche est cultivée avec du milieu YPD, si elle n'a pas été complémentée, sinon, sur du milieu SC-glucose (sans uracile).

### II. A. 3. Souche d'agrobactéries

La souche d'agrobactéries utilisée pour la transformation des hampes florales *d'Arabidopsis thaliana* est *Agrobacterium tumefaciens* LBA 4404. Elle est cultivée sur du milieu YEB ou dans du milieu LB sous agitation, à 28 °C, en présence de rifampicine à 100 µg.mL⁻¹ et de streptomycine à 200 µg.mL⁻¹

### II. B. Vecteurs de clonage

Les cartes des vecteurs de clonage utilisés sont données dans la fig. 1. Le vecteur utilisé pour le clonage des fragments de PCR est le plasmide pGEM-T-Easy (Promega).

Les vecteurs d'expression pDR195 ou pDR196 ont été utilisés pour complémenter les souches de levure S. *cerevisiae* RS453. Ces vecteurs ont été utilisés pour l'expression hétérologue de transporteurs de mannitol de végétaux chez la levure, sous le contrôle du promoteur PMA1.

Le plasmide pDONR 207 (Invitrogen) a été utilisé comme vecteur donneur dans le kit "Gateway Technology" (Invitrogen).

Les plasmides pBl 101-GUS-R1R2 et pBl 101-GFP-R1R2 possèdent les bordures R1 et R2 (*att*R1 et *att*R2) qui permettent le clonage du promoteur d'intérêt en amont des gènes rapporteurs GUS (β-glucuronidase, *uid*A) ou GFP (Green Fluorescent Protein 5-ER), par recombinaison entre le clone d'entrée et les vecteurs destination pBl (kit "Gateway Technology", Invitrogen). Après l'étape de recombinaison, le vecteur pBI 101 porte les gènes rapporteurs codant GUS ou GFP, sous le contrôle du promoteur étudié, et du terminateur NOS.

### III. Méthodes

### III. A. Extraction des ARN totaux de tissus végétaux

La technique utilisée pour extraire les ARN totaux a été décrite par Kay et *al.* (1987).

### III. B. Clonage d'ADN par amplification (PCR)

### III. B. 1. Amplification d'ADN Génomique par PCR

10 ng d'ADN sont mis en présence de 50 µL de milieu réactionnel contenant 250 µM de dNTP ; 1 µM d'amorces sens et antisens ; 1 U d'enzyme *Taq* ADN polymérase "GoTaq" (Promega) et 1X de tampon PCR. Chaque cycle comprend (après une première étape de dénaturation de 1 min à 94°C et suivi d'une dernière étape d'élongation de 5 min à 72 °C) : une étape de dénaturation de 15 sec à 94 °C ; une étape d'hybridation de 2 min à la température adéquate et une étape d'élongation à 72 °C dont la durée est proportionnelle à la taille du fragment à amplifier (1000 bp.min⁻¹). Les couples d'amorces utilisés pour les clonages dans le vecteur pDONR207 sont les suivants : Pour le clonage de la partie promotrice d'AgMaT3 :
Amorce 5'
   5'-GGGGACAAGT TTGTACAAAA AAGCAGGCTG AACAGAAACAATTGTGGATG- 3'
Amorce 3'
   5'-GGGGACCACT TTGTACAAGA AAGCTGGGTA ATGTTGAGAA ACAATGGTCG-3'

Pour le clonage de la partie promotrice d'AgMT2 :
Amorce 5'
   5'- GGGGACAAGT TTGTACAAAA AAGCAGGCTG ACCCACTATC AACAATGATC-3'
Amorce 3'
   5'- GGGGACCACT TTGTACAAGA AAGCTGGGTA TAAGATCGTT GTGGACTCTG-3'

Pour le clonage de la partie promotrice d'AgMT3.
Amorce 5'
   5'- GGGGACAAGT TTGTACAAAA AAGCAGGCTT CTTTATTCTG CAGCTAGAGC-3'
Amorce 3'
   5'- GGGGACCACT TTGTACAAGA AAGCTGGGTG CTTGAAGTAA GGTGGTATGC-3'

Le choix de la température d'hybridation dépend du calcul préalable de la température de fusion (Tm). Les produits d'amplification obtenus sont analysés sur gel d'agarose 1 %.

### III. B. 2. Amplification d'ADN à partir d'ARN inversement transcrits (RT-PCR)

Après dénaturation de 6 µg d'ARN totaux à 70 °C pendant 10 min, la transcription inverse est menée durant 60 min à 42 °C en présence d'amorce oligo(dT)₁₈ 2,5 µM, de dNTP 500 µM et de "M-MLV Reverse Transcriptase" (200U, Promega). Les hétéroduplex ARN/ADNc obtenus sont dénaturés 5 min à 100 °C avant qu'une réaction d'amplification par PCR de la région d'ADNc cible ne soit faite en prenant 2 µL du milieu de synthèse du premier brin d'ADNc comme matrice

### III. B. 3. Clonage des fragments de PCR dans le plasmide pGEM-T-Easy

Le vecteur du système "pGEM-T-Easy Vector Systems" (Promega) est un vecteur linéarisé adapté au clonage direct des produits de PCR grâce aux résidus thymidine greffés à chacune de ses extrémités 3'. Les produits de PCR sont ligaturés dans 50 ng de plasmide pGEM-T-Easy selon un rapport molaire insert/vecteur de 3, pendant une nuit, à 16 °C en présence de 3U d'ADN ligase T4 (kit Promega). 3 des 10 µL du milieu de ligature ont été utilisés pour transformer 200 µL de bactéries compétentes.

### III. C. Transformation de bactéries Escherichia coli compétentes

Les bactéries sont rendues compétentes en présence de CaCl₂.

### III. C. 1. Préparation de bactéries compétentes

Les bactéries *E. coli* DH5α sont rendues compétentes selon la méthode décrite par Sambrook et al, (1989). Les bactéries sont alors congelées dans l'azote liquide en aliquots de 200 µL et stockées à - 80°C.

### III. C. 2. Transformation des bactéries thermocompétentes

La transformation des bactéries *E. coli* DH5α compétentes est réalisée selon la technique décrite par Sambrook et al, (1989). Le choc thermique se passe à 42°C, pendant 90 sec. L'addition de 800 µL de milieu SOCt est suivie d'une incubation à 37°C sous agitation pendant 1 h pour permettre l'expression phénotypique du gène de résistance à l'antibiotique porté par le plasmide. Le milieu de transformation est ensuite étalé sur boîte dYT contenant l'antibiotique approprié. Ces boîtes sont placées à 37°C pendant 16 h.

### III. D. Analyse de l'ADN plasmidique

### III. D. 1. Minipréparation d'ADN plasmidique

Cette extraction repose sur la technique par lyse alcaline des bactéries, adaptée de Sambrook *et al.* (1989) ; une colonie bactérienne est mise en culture dans 5 mL de milieu dYT contenant l'antibiotique approprié pendant une nuit à 37°C sous agitation. Les cellules provenant de 3 mL de culture sont concentrées par centrifugation de 1 min à 6000 g. Le culot est resuspendu dans 200 µL de solution 1 [Tris HCl 25 mM (pH 8) ; EDTA 10 mM ; Glucose 50 mM] supplémentée en lysozyme à 1 mg.mL⁻¹ final, puis après vortexage, 400 µL de solution 2 [NaOH 0,2 N ; SDS 1 %] et 300 µL de solution 3 [acétate de potassium 3 M ; acide acétique glacial 11,5 % (v/v); pH 4,8 ] sont successivement ajoutés. Après une incubation de 5 min dans la glace, les débris cellulaires sont sédimentés par une centrifugation de 10 min à 14000 g. Le surnageant est transféré dans un microtube propre où de la RNase A à 100 µg.mL⁻¹ final est ajoutée avant une incubation à 65 °C pendant 15 min. Le surnageant est purifié par une extraction au phénol/chloroforme/alcool isoamylique (25:24:1). L'ADN est précipité avec un volume d'isopropanol, rincé à l'éthanol 70 % à deux reprises, séché et repris dans 20 µL d'eau.

### III. D. 2. Etablissement de profil de restriction

Un microlitre de solution d'ADN plasmidique (0,5 g.L⁻¹) est digéré par différentes enzymes de (3U) durant 2h à 37°C. Les produits de restriction sont analysés sur gel d'agarose 1 %.

### III. E. Séquençage et analyse des clones

Les clones les plus intéressants sont séquencés. L'alignement des séquences nucléotidiques, la recherche des sites de restriction par les endonucléases, mais aussi la recherche de cadres ouverts de lecture et la traduction des fragments d'ADN en acides aminés ont été effectuées avec le logiciel Mac Molly Tetra (Soft Gene GmbH). Les comparaisons avec les séquences contenues dans les banques de données ont été réalisées grâce au programme BLAST sur le serveur NCBI (http://www.ncbi.nlm.nih.gov).

L'analyse des promoteurs et la recherche de boîtes spécifiques de reconnaissance et de fixation de protéines régulatrices de la transcription ont été réalisées sur le serveur PLACE (Higo *et al.,* 1999, http://www.dna.affrc.go.jp/htdocs/PLACE/signalscan.html).

### III. F. Banque soustractive

Cette banque représente tous les gènes dont l'expression est modifiée dans un tissu suite à un traitement donné. Elle a été construite selon les kits "SMART PCR cDNA Synthesis" et "PCR-Select cDNA Subtraction" (Clontech) à partir d'ARN totaux de phloème extraits soit de plantes "témoins", soit de plantes "stressées au NaCl 300 mM" pendant trois semaines. Les ADNc sont directement produits et amplifiés à partir d'ARN totaux, avant digestion enzymatique par Rsal, Les ADNc tester (phloème de plantes "stressées" S) sont divisés en deux et sur chaque sous-lot un adaptateur différent est ligaturé donnant S1 et S2. S1 et S2 sont hybridés avec l'ADNc driver (phloème de plantes "témoins" T) en excès, puis réunis et soumis à une deuxième hybridation. Les ADNc correspondant à des gènes exprimés à la fois en conditions stressées et témoins vont pouvoir s'hybrider. Seules les ADNc de plantes stressées ne s'étant pas hybridés avec un homologue "témoin" et possédant des adaptateurs différents pourront être amplifiées et clonés.

### III. F. 1. Clonage de la banque soustractive

Les produits de la PCR (fragments de 90 à 900 pb) sont directement clonés dans le vecteur pGEM-T-Easy (Promega), puis dans la souche d'*E. coli* DH5α. Les colonies obtenues sont réparties sur des plaques de 96 puits, cultivées ainsi dans du milieu dYT liquide et conservées à -80 °C (50 % glycérol).

### III. F. 2. Minipréparation sur plaque

Les minipréparations sont réalisées directement sur plaques de 96 puits. La banque est copiée et cultivée sur plaques à puits profonds dans 2 mL de milieu dYT, pendant 16 h à 37 °C et sous agitation. Après centrifugation (4000 g pendant 10 min à 4 °C), le culot est resuspendu avec 100 µL des solutions P1 (Resuspension buffer, Qiagen) froide à 50 µg.mL-1 de RNase A, P2 (Lysis buffer, Qiagen), et P3 (Neutralization buffer, Qiagen) froide, sous agitation jusqu'à formation des précipités. Après centrifugation, le surnageant est précipité avec un volume d'isopropanol. Suite à une nouvelle centrifugation, les culots sont lavés à l'éthanol 70 %, puis sont séchés et resuspendus dans du tampon TE (25µL ; Tris 50 mM, EDTA 10 mM), sous agitation. La concentration finale en ADN plasmidique est déterminée par spectrophotométrie.

### III. F. 3. Dépôts sur filtres

L'ADN plasmidique, contenu dans chaque puits des plaques à 96 puits, est dénaturé au NaOH (0.4 M final) et déposé sur des membranes de nylon Hybond-N (Amersham ; 8 x 12 cm) à l'aide d'un réplicateur. Les clones plasmidiques (100 ng) sont déposés sur les filtres et fixés aux membranes par incubation à 80 °C pendant 2 h.

### III. F. 4. Hybridation et révélation des filtres

Les filtres sont placés en préhybridation durant 4 h à 65 °C (sonde complexe) ou à 42 °C (sonde T7), dans une solution de préhybridation [tampon NaP 0,25 M, pH 7,2 (Na₂HPO₄, NaH₂PO₄) ; SDS 6,6 %, EDTA 1 mM, pH 8 et serum albumine bovine 1 % ].

La "sonde complexe" radioactive est produite par transcription inverse sur des ARN totaux représentant les gènes exprimés dans un tissu suite à un traitement donné, à partir d'oligo-dT20 ancrés. Ces sondes vont donc s'hybrider différentiellement sur les clones déposés sur les filtres. La différence d'intensité entre les signaux provenant des sondes complexes "stressée" et "témoin" permet d'identifier les clones les plus intéressants. Les valeurs relatives obtenues, sont réajustées entre-elles par calibrage avec une sonde T7 interne au plasmide déposé.

La première étape de la réaction de reverse transcription est une dénaturation à 70 °C pendant 10 min, des ARN et des oligonucléotides ancrés, (dT)20dA, (dT)20dG, (dT)20dC (2 µg de chaque), mélangés. Les formes dénaturées sont conservées par transfert direct dans la glace. Ces ARN sont ensuite dilués dans un mélange préchauffé à 42 °C pendant 5 min, composé de tampon de RT 1X (Tris-HCl 50 mM, pH 8,3 ; KCI 75 mM ; MgCl2 3 mM et DTT 10mM), de dNTP (0.8 mM de chaque, excepté pour dATP à 5 µM) et de dATP-33P à 2.775 MBq dans 7,5 µL (Amersham). Enfin, 800 U de "MMLV reverse transcriptase" (Promega) sont ajoutés à cette réaction de PCR et le mélange est incubé à 42 °C pendant 1 h et à 70 °C pendant 15 min, puis refroidi dans la glace (dénature l'enzyme et conserve la linéarisation des ADNc). Afin qu'il ne puisse pas y avoir d'interférences avec l'hybridation de la sonde, l'ARN matrice est détruit par ajout de 8 U de Ribonucléase H (Promega) à 37 °C pendant 30 min. La sonde est stockée sur de la glace en attendant la vérification de l'incorporation de dATP-33P. L'élution est réalisée avec de l'eau par fractions de 200 µL dont la radioactivité est estimée à l'aide d'un compteur à scintillation (Packard, TRI-CARB 1900 TR). Les fractions contenant la sonde sont rassemblées et sont utilisées comme "sonde complexe" afin d'hybrider les filtres à 65 °C pendant une nuit.

La quantité d'ADN plasmidique déposée au niveau de chaque spot est calibrée par hybridation avec une sonde T7. Cette sonde est produite à partir de 100 ng d'oligo T7 (20-mer) marqués à leur extrémité (5') par du γATP-³³P (1.85 MBq) fixés par réaction de la "T4 polynucleotide kinase" (10U, Promega) dans du tampon kinase 1X (Promega) à 37 °C pendant 30 min, puis bloquée sur glace. Les filtres sont hybridés avec le produit de la réaction, une nuit à 42 °C.

L'hybridation des filtres est réalisée pendant 16 h à 65 °C (sonde complexe) ou à 42 °C sonde T7), en présence de la sonde radiomarquée diluée dans le tampon d'hybridation (de même composition que le tampon de préhybridation).

Les filtres sont ensuite lavés 2 fois 10 min à 65 °C ou à 42 °C dans du SSC 2X ; SDS 0,1 %, 10 min à 65 °C ou à 42 °C dans du SSC 1X ; SDS 0,1 %, 5 min à 65 °C ou à température ambiante dans du SSC 0,5X ; SDS 0,1 %. (SSC1X: NaCl 0,15 M et 0,015 M de citrate de sodium).

Les filtres sont emballés dans des sacs plastiques et placés sur des écrans de type "low energy" (Kodak, Amersham), pendant six jours à l'intérieur de cassettes d'exposition. Les signaux présents sur les filtres sont ainsi quantifiés par le système Phospholmager (STORM 820, Molecular Dynamics, Amersham) et exprimés en unités relatives. Les valeurs obtenues avec la sonde T7 (sonde interne à chaque plasmide) sont utilisées pour calculer les intensités relatives du signal de chaque spot.

### III. G. Hybridation de type Northern

### III. G. 1. Electrophorèse et transfert des ARN

La technique utilisée pour l'électrophorèse sur gel dénaturant d'agarose et le transfert des ARN par capillarité sur une membrane de nylon est celle décrite dans Noiraux et al, 2000. Après le transfert, les ARN sont fixés sur la membrane par une incubation à 80°C pendant 2 h.

### III. G. 2. Préparation de la sonde

La sonde radioactive est préparée à partir d'un fragment ADN d'intérêt. Après digestion enzymatique, l'ADN a été séparé sur gel d'agarose 1 % (Sambrook *et al.,* 1989) du plasmide d'insertion et purifié sur colonne "DNA gel extraction kit" (Millipore). Le culot d'ADN est resuspendu dans de l'eau à une concentration finale de 125 ng/µL.

La sonde radioactive est réalisée avec le kit "Prime-a-gene Labeling System" (Promega), basé sur l'hybridation d'un mélange d'hexanucléotides avec le fragment d'ADN à marquer, dénaturé. Les hexanucléotides servent d'amorces au fragment de Klenow qui synthétise un brin complémentaire en incorporant du α32P-dCTP. La synthèse de la sonde s'effectue à 25 °C pendant 2 h en présence de 25 ng d'ADN matriciel dénaturé et de 1,51.106 Bq de α³²P-dCTP (activité spécifique 220 TBq.mmol-1). A la fin de la réaction, les nucléotides non incorporés sont séparés de la sonde par passage du mélange sur une colonne Sephadex G-50. Les fractions contenant la sonde sont rassemblées, dénaturées à 95 °C pendant 3 min, puis stockées dans la glace jusqu'à utilisation.

### III. G. 3. Hybridation du Northern

La membrane est préhybridée durant 4 h à 65 °C dans la solution de préhybridation. L'hybridation est réalisée pendant 16 h à 65 °C en présence de la sonde homologue radiomarquée (hybridation Céleri/Céleri). La membrane est ensuite lavée 2 fois 15 min à 65 °C dans du SSC 2X ; SDS 0,1 %, 30 min à 68°C dans du SSC 1X ; SDS 0,1 %, 15 min à 68°C dans du SSC 0,5X ; SDS 0,1 %.

La membrane est emballée dans un sac plastique et placée sur des écrans pendant 3 heures. Les dépôts d'ARN sont calibrés avec une sonde ribosomique 26S de Céleri, marquée au α³²P-dCTP.

### III. H. RACE-PCR

Les séquences codantes ainsi que les parties 5' et 3' non traduites sont clonées grâce au "Marathon cDNA amplification kit" (Clontech) à partir d'ARN totaux de phloème extraits soit de plantes "témoins T", soit de plantes "stressées S". Les amorces utilisées pour ces clonages sont choisies conformément aux prescriptions du kit. Les produits de PCR sont des ADN complémentaires et sont directement clonés dans le vecteur pGEM-T-Easy (Promega), puis dans la souche d'*E. coli* DH5α.

### III. 1. RT-PCR en temps réel

Les RT-PCR en temps réel ont été réalisées à partir de RT (reverse transcription) sur lesquelles une PCR est pratiquée à partir d'amorces (Invitrogen) et de sondes TaqMan (Applied Biosystems, U.K.). Celles-ci sont choisies dans les régions 3' non traduites des séquences codantes des transporteurs de mannitol testés. Ces régions présentant le moins de similitudes entre elles, le signal obtenu est spécifique de l'expression d'un de ces transporteurs.

La RT-PCR quantitative a été réalisée essentiellement comme décrit dans Wagner et al., 2001.

Les amorces spécifiques de AgMaT3 sont :
5'- ATA CAG CGG GGA TTA TAG CTT TG -3' et 5'- ATC CGC AGG TAC TCC AAA AAT TT -3', afin d'amplifier un fragment de 101 paires de bases spécifiques de la région 3' non codante. Le fragment amplifié est vérifié par séquençage. La sonde marquée au FAM est 6 FAM-TAC CCG GTA TAT TCA CTC-MGB (synthétisé par Applied Biosystems).

Pour l'amplification de Ag26S (gène contrôle), les amorces utilisées sont :
5'- AGC CGC TGG ACC CTA CCT-3' et 5'- AGT TAT CTT TTC TGT TTA ACA GCC T-3', tandis que la sonde fluorescente est 6 FAM-CTA AGC CGT TTC CAG G-MGB.

Les amorces et sondes sont déterminées à l'aide du logiciel Primer Express, v.1.0 (Applied Biosystems).

Les fragments sont obtenus par PCR en présence de "TaqMan Universal PCR Master Mix" 1X (Applied Biosystems, Roche), d'amorces 5' et 3' à 0,9 µM chacune, de sonde TaqMan 0,2 µM et du produit de RT diluée au 1/300^{e} (RT produite à partir de 10 µg d'ARN totaux). La fluorescence émise par les sondes TaqMan est détectée grâce au système de détection ABI PRISM 7700 (Applied Biosystems). Les signaux sont quantifiés grâce aux amorces et sondes ribosomiques 26S de Céleri. Ceci donne des valeurs Ct définies comme le numéro du cycle auquel la valeur ΔRn (intensité de fluorescence normalisée du colorant rapporteur) dépasse une valeur seuil générée par le logiciel. Ct est donc inversement proportionnel à la concentration de la séquence cible.

### III. J. Clonage des promoteurs et des séquences génomiques

Les séquences génomiques (introns+exons) et les promoteurs de séquences connues, sont obtenus par une technique basée sur la PCR, en s'affranchissant de toutes les étapes de criblage (banque phagique) traditionnellement utilisées. L'ADN génomique est divisé en quatre lots, chacun étant digéré par une enzyme différente. Les enzymes choisies donnent des coupures à bord franc. Selon l'emplacement des sites de restriction, les fragments obtenus seront de taille variable. Des adaptateurs de séquences connues sont ensuite ligaturés aux extrémités des fragments de digestion. Sur chacun des 4 lots, une PCR est réalisée avec une amorce spécifique du gène étudié (GSP1) et une amorce spécifique de l'adaptateur (AP1). La seconde PCR ou "nested" est pratiquée sur le produit de la première, avec des amorces (GSP2 et AP2) plus internes que les premières, afin d'optimiser la spécificité du résultat. Jusqu'à 4 fragments de PCR de taille variable sont ainsi obtenus. Le plus long, portant le plus d'informations, est purifié.

### III. J. 1. Extraction de l'ADN génomique

Les tissus (folioles jeunes) sont broyés en une fine poudre dans un mortier en présence d'azote liquide. Quinze millilitres de tampon CTAB 2X [CTAB 2 % (p/v) ; NaCl 1,4 M ; EDTA 20 mM ; Tris 100 mM (pH 8) ; β-mercaptoéthanol 0,2 % (v/v)] préchauffé à 60 °C sont ajoutés pour 3 à 5 g de poudre broyée et incubent 30 min à 60 °C. Les protéines et les débris cellulaires sont éliminés par une extraction au chloroforme/alcool isoamylique [24:1 (v/v)] suivie d'une précipitation à l'isopropanol froid (0,5 volume) et à -20 °C pendant 30 min. Si les filaments d'ADN sont visibles, il est alors possible de les attraper et de les laver à l'éthanol 76 % contenant de l'acétate-NH₄ 10 mM final, pendant 20 min. Sinon, centrifuger 5 min à 5000 *g* et laver le culot de la même façon. L'ADNg est à nouveau centrifugé et le culot est séché à l'air, puis repris dans 1 mL de tampon TE [Tris 50 mM ; EDTA 10 mM] additionné de RNase A à 10 µg.mL⁻¹ final et incube ainsi 30 min à 37 °C. L'ADNg est précipité par ajout successif de 1,5 mL de tampon TE, de 0,5 mL de NaCl (5 M) et 2 mL d'isopropanol froid. Après centrifugation à 5000 *g* pendant 10 min, le culot est lavé à l'éthanol 70 %, séché à l'air et repris dans du tampon TE (50 µL).

Les quantités d'ADN sont estimées par un dosage spectrophotométrique entre 210 et 310 nm. Une absorbance de 1 à 260 nm correspond à une concentration en ADN de 50 µg.mL-1.

### III. J. 2. Construction de la banque génomique

La banque génomique est construite selon le kit "Universal Genome Walker kit" (Clontech) à partir d'ADN génomique. Les amorces utilisées sont choisies conformément aux prescriptions du kit. Les produits de PCR obtenus sont directement clonés dans le vecteur pGEM-T-Easy, puis dans la souche d'E. coli DH5α en vue d'une minipréparation et du séquençage.

### III. K. Transformation d'Arabidopsis thaliana avec un promoteur de Céleri en amont d'un gène rapporteur

### III. K. 1. Technologie gateway

Le profil d'expression des promoteurs a été suivi par transfert de leur séquence en amont d'un gène rapporteur. L'utilisation de la technologie gateway permet le passage d'un fragment d'ADN (complémentaire ou génomique), à partir de son produit de PCR, dans un "vecteur donneur" (pDONR 207) puis dans un "vecteur destination" (pBI-GUS-R1R2 ou pBI-GFP-R1R2).

Les amorces produites (Invitrogen) pour cloner ces promoteurs contiennent les séquences attB, et sont choisies conformément aux données du kit "Gateway Technology" (Invitrogen). Les PCR ont été réalisées sur de l'ADN génomique obtenu selon le § III. J. 1. Après chaque ligature, les plasmides obtenus sont amplifiés dans la souche d'E. coli DH5α. Les plasmides utilisés contiennent le gène ccdB : chez la plupart des bactéries, il est létal. Ainsi, seuls les plasmides ayant effectué la réaction de recombinaison (insertion du fragment d'ADN et excision du gène ccdB) peuvent être amplifiés dans la bactérie.

### III. K. 2. Transformation d'Agrobactéries électrocompétentes

### III. K. 2. a. Préparation d'Agrobactéries compétentes

La préparation des agrobactéries *A. tumefaciens* LBA 4404 se fait selon Koncz et Schell (1986) afin de préparer des fractions aliquotes de 40 µL (concentrations de .3.10¹⁰ cellules.mL⁻¹) qui sont stockées à -80 °C jusqu'à utilisation.

### III. K. 2. b. Transformation d'Agrobactéries par électroporation

Les cuves à électroporation sont placées à 4°C dans de la glace. Une fraction aliquote d'agrobactéries (40 µL) est décongelée et 2 µL de solution d'ADN plasmidique sont ajoutés, le tout est déposé dans les cuves. Les agrobactéries subissent un choc électrique de 5 msec à 15 kV.cm⁻¹. Immédiatement, 960 µL de milieu YEB sont ajoutés, puis la suspension est transférée et incubée à 28°C sous agitation pendant 1 h pour permettre l'expression phénotypique du gène de résistance à l'antibiotique porté par le plasmide. Le milieu de transformation est ensuite étalé sur boîte YEB contenant les antibiotiques appropriés, placées à 28 °C pendant 16 h.

### III. K. 3. Transformation d'Arabidopsis thaliana

La transformation des Arabidopsis se fait selon la méthode décrite par Clough et Bent (1998), les agrobactéries transformées étant cultivées jusqu'à une absorbance de 0,8 à 600 nm puis ensuite utilisées pour transformer les hampes florales d*'Arabidopsis thaliana.*

### III. L. Etude de l'expression fonctionnelle hétérologue de transporteurs de plantes chez la levure Saccharomyces cerevisiae

### III. L. 1. Clonage d'ADNc dans le vecteur d'expression pDR

Le vecteur pDR possède le promoteur PMA1et le terminateur ADH qui permettent l'expression de transporteurs dans la levure. La séquence d'ADNc est extraite du vecteur d'amplification (pGEM-T-Easy), par digestion enzymatique, avec les mêmes enzymes de restriction (3U, 2h à 37°C) qui coupent le vecteur pDR, afin de réaliser un clonage orienté. Les produits des digestions sont contrôlés et purifiés sur gel d'agarose 1 %. Le plasmide pDR digéré est ensuite déphosphorylé par l'enzyme Shrimp Alkaline Phosphatase (4U, Promega) 1h à 37°C, empêchant sa recircularisation. Les différentes enzymes sont inhibées après chaque réaction, pendant 15 min à 65°C. La ligature de l'ADNc du transporteur dans pDR est alors possible grâce à la T4-DNA ligase (3U, Promega), une nuit à 16°C, selon un rapport insert/vecteur de 3. Le vecteur préparé est amplifié dans la souche d'E. coli DH5α avant transformation des levures.

La préparation des levures compétentes puis leur transformation se fait selon le protocole de Dohmen et al. (1991)

### III. L. 2. Absorption de polyols par les levures transformées

### III. L. 2. a. Préparation des levures pour l'absorption

Les levures sont préparées selon la méthode décrite par Noiraud et al. (2001),

### III. L. 2. b. Absorption de mannitol et de sorbitol radiomarqués par les levures

L'absorption des polyols, manitol et sorbitols radiomarqués, par les levures se mesure selon la méthode décrite par Noiraud et al. (2001), cent µL de suspension de levures sont incubés à 28°C en présence de 100µL d'une solution contenant 1,1 mM de mannitol (0,55mM final) avec 5,8 KBq de mannitol [2-3H] (activité spécifique 6,3.108 kBq.mmol-1). Pour le test d'absorption du sorbitol, les 100 µL de suspension de levure contienent 1,1 mM de sorbitol total (0,55 mM final) avec 16,6 KBq de sorbitol-¹⁴C (activité 1,02.10⁷ kBq.mmol⁻¹).

### III. L. 2. c. Dosage des protéines sur levures

500µL de cellules préparées pour l'absorption, lavées et resuspendues dans du SC MES sans uracile sont déposés dans un tube à essai et complétés à 2 mL avec de l'eau. 500µL de NaOH 15% sont ajoutés, et l'ensemble est porté à ébullition pendant 5 min. Après refroidissement, 175 µL de HCL 11,75 N sont ajoutés pour neutraliser la solution et l'ensemble est vortexé.

Les protéines sont dosées selon la technique de Bearden (1978), basée sur le changement de couleur du bleu de Coomassie lorsqu'il se lie à une protéine.

### Exemple 2

### I. Stress salin et Céleri

Afin de constituer une réserve de tissus pour la construction de la banque soustractive et la production des sondes complexes, du Céleri a été soumis à un stress salin, en modifiant certaines conditions par rapport aux traitements réalisés antérieurement (Noiraud, 1999). Afin de standardiser les conditions de récolte des organes, c'est l'âge réel des feuilles (à partir de la date d'émergence) et non leur aspect général (ex : la taille) qui est pris en compte. Le stress a un effet négatif sur la croissance et le développement des feuilles: une feuille soumise à un stress et une feuille non traitée peuvent paraître identiques, sans avoir le même âge.

Sur les neuf plantes de chaque lot ("témoins" et "stressées"), six sont exploitées pour la récolte des tissus et l'extraction d'ARN nécessaires à la confection des banques et des sondes. Seul le phloème des pétioles de feuilles ayant atteint un âge de 3 semaines a été employé pour la construction de la banque soustractive. Ces feuilles ont effectué tout leur développement en condition de stress.

### Aspect général du stress salin sur les plantes

Après un traitement de 3 semaines avec 300 mM NaCl, les plantes sont affectées dans leur croissance. L'observation de l'aspect général des Céleris (**fig.2**) montre une différence de taille et de volume entre les pieds "stressés" et "témoins", en corrélation avec la baisse de vitesse de croissance et l'absence d'émergence de feuilles chez les plantes traitées au NaCl :
Lors du stress salin, le Céleri présente un ralentissement de la croissance et de l'émergence de nouvelles feuilles. L'inhibition de la croissance est une stratégie de tolérance au stress. Cet arrêt est réversible car si les plantes, après 3 semaines de stress salin, sont arrosées avec une solution ne contenant pas de NaCl, très vite la croissance et la vitesse d'émergence des feuilles est rétablie à un niveau identique à celui noté avant le stress salin.

Les feuilles les plus externes (les plus âgées) entrent en sénescence plus rapidement, ce qui pourrait indiquer une stratégie pour éliminer les ions toxiques des organes les plus jeunes, en les accumulant et les séquestrant dans des organes sénescents. Cette séquestration dans les vieilles feuilles, représente une stratégie de tolérance au stress salin. Chez le Céleri, il est important de préserver intact le méristème végétatif pour qu'il devienne inflorescentiel.

Cette stratégie mise en oeuvre doit être coordonnée à l'échelle de la plante entière. Ceci est possible par la mise en route d'un nouveau programme d'expression de gènes. Ce sont ces gènes spécifiquement induits par ce type de stress, qui ont été caractérisés par les inventeurs. Cette étude s'est concentrée sur le phloème qui joue un rôle prépondérant dans les échanges inter-organes de molécules nutritives et de molécules informationnelles.

### II. Réalisation d'une banque soustractive

Pour identifier des gènes spécifiquement induits par le stress salin, une banque soustractive a été réalisée grâce au kit Clontech PCR-Select cDNA Subtraction qui permet d'identifier les gènes dont l'expression est stimulée dans une condition physiologique donnée (dans ce cas, le stress salin). En théorie, seuls ces gènes sont obtenus en fin d'analyse. Aux ADNc de plantes "stressées", sont soustraits les ADNc correspondant aux gènes exprimés constitutivement à l'état normal. Les cycles de PCR permettent d'amplifier les gènes spécifiques du stress salin.

La quantité de tissu de phloème extrait étant plus limitée qu'à partir de feuilles, une étape supplémentaire d'amplification initiale des messagers a été introduite (kit Smart PCR cDNA synthesis, Clontech). La banque soustractive a été réalisée conformément aux directives du fournisseur (kit Clontech PCR-Select cDNA Subtraction, Clontech). Si une première banque soustractive de phloème a été construite et présentait de bons résultats en relation avec une réponse au stress salin (dont protéines de choc thermique et une sous-unité de la caséine kinase 2), la quantité de clones obtenus était trop faible pour constituer une banque.

Une nouvelle banque soustractive de phloème a été réalisée dont les résultats sont détaillés ci-dessous : La taille moyenne des inserts est comprise entre 90 et 900 pb. Les clones obtenus après soustraction et amplification sont ligaturés dans le vecteur pGEM-T-Easy (Promega). Les colonies (les plus grosses, soit 736 parmi environ 750) sont cultivées individuellement sur des plaques de 96 puits. Une réplication a été réalisée en vue d'une conservation après congélation à -80 °C, une autre réplique a été cultivée afin d'extraire l'ADN plasmidique par minipréparation.

### II. A. Mise au point du système de criblage des banques soustractives

Le nombre de clones obtenus par la banque soustractive est beaucoup trop grand (736 pour la banque soustractive phloème) pour pouvoir les analyser indépendamment les uns des autres. Afin d'affiner la recherche, l'ADN plasmidique des clones obtenus a été déposé sur des filtres qui sont hybridé avec des sondes complexes produites à partir d'ARN extraits de tissus "stressés" ou "témoins". Les signaux sont ensuite quantifiés et analysés, et les clones dits positifs (selon le rapport NaCl/témoin) sont retenus. Le radioélément choisi pour le marquage des sondes est le Phosphore 33 avec une concentration de 50 ng.µL⁻¹ d'ADN plasmidique.

### II. B. Dépôt et hybridations des clones obtenus suite à la banque soustractive

Des filtres en nitrocellulose (96 dépôts) ont été réalisés pour identifier les clones: les minipréparations sont réalisées dans les plaques sur lesquelles ont poussé les bactéries hôtes après réplication de la banque congelée. Ainsi 736 clones indépendants ont pu être déposés sur membrane et analysés. Les membranes sont hybridées avec des sondes complexes réalisées par marquage au ³³P suite à une transcription reverse sur des ARN extraits soit de phloème témoin, soit de phloème de plantes stressées. Les marquages radioactifs obtenus sont comptés (Phospholmager,) et comparés. Trente clones ont présenté un signal fort en conditions de stress et leur ADN plasmidique a été séquencé **(tableau I).**

**Tableau I. Séquences d'ADNc obtenues à partir de la banque soustractive de phloème ayant montré un rapport élevé stressé/témoin et lors des hybridations différentielles. La taille approximative de ces fragments est indiquée en paires de bases. L'homologie et le numéro d'accession correspondant ont été obtenus par comparaison avec les bases de données BLAST.**

| Taille (pb) | Annotation fonctionnelle | Homologie | Numéro d'accession | BLAST E-value |
|---|---|---|---|---|
| 500 | oxidoreductase, Zn-binding dehydrogenase | *Arabidopsis* | NP_173786 | 5,1 |
| 300 | *aucun résultat* | - | - | - |
| 90 | metallothionein AgMT2 | *P. brachycarpa* | AAC62510 | 1e-04 |
| 440 | chlorophyll a/b binding protein of CP29 | Tomate | CAA43590 | 3e-43 |
| 350 | 14-3-3 family protein | Tomate | P93211 | 3e-58 |
| 310 | *aucun résultat* | - | - | - |
| 800 | NifU-like protein | *Arabidopsis* | NP_193953 | 1 e-05 |
| | ou dehydrin | Orge | AAD02257 | 0,001 |
| 800 | beta-galactosidase | *Arabidopsis* | T00787 | 2e-35 |
| 700 | hypersensitive-induced reaction protein | Orge | AAN17454 | 6e-44 |
| 320 | *inconnu* | *Arabidopsis* | AAK31144 | 1e-05 |
| 150 | *aucun résultat* | - | - | - |
| 600 | chlorophyll a/b-binding protein | Tomate | S14305 | 6e-23 |
| 170 | *aucun résultat* | - | - | - |
| 180 | mannitol transporter AgMaT3 | Céleri | AAG43998 | 2e-20 |
| 350 | metallothionein AgMT3 | Vigne | CAB85630 | 2e-09 |
| 470 | dnaK-type molecular chaperone hsc70 | Tomate | JC4786 | 2e-76 |
| 660 | high molecular weight heat shock protein | Pommier | AAF34134 | 5e-40 |
| 900 | lipid transfer protein | *Arabidopsis* | NP_177181 | 3e-13 |
| 400 | fiber annexin | Coton | T31428 | 1e-58 |
| 800 | RUB1 conjugating enzyme | Tomate | AAG23847 | 5e-54 |
| 900 | annamyl-alcohol dehydrogenase | *A. cordata* | P42495 | 2e-69 |
| 620 | 40S ribosomal protein S27 | *Arabidopsis* | NP_191670 | 2e-33 |
| 600 | 60S ribosomal protein L30 | Lupin | O49884 | 1e-55 |
| 650 | 60S ribosomal protein L28 | *Arabidopsis* | NP _194670 | 7e-41 |
| 450 | *aucun résultat* | - | - | - |
| 250 | *aucun résultat* | - | - | - |
| 250 | *aucun résultat* | - | - | - |
| 640 | *aucun résultat* | - | - | - |

### II. C. Résultats et interprétation bibliographique des clones positifs en relation avec le stress et le phloème

Parmi les 30 clones positifs, des séquences ont pu être identifiées qui présentent des similitudes avec des gènes dont le rôle dans les stress a été documenté (notamment protéines de choc thermique, HSP, déhydrines et métallothionéines). Trois séquences sont identiques et appartiennent au même gène et 8 autres ne correspondent à rien de connu. Parmi les 21 restantes, 3 séquences sont plus particulièrement étudiées.

### II. C. 1. AgMaT3, un troisième transporteur de mannitol chez le Céleri ?

La séquence partielle trouvée est très similaire aux 2 autres transporteurs de mannitol identifiés chez le Céleri: AgMaT1 et AgMaT2 (Noiraud *et al.,* 2001 a). La séquence partielle isolée dans la banque soustractive phloème est légèrement différente et a été nommée AgMaT3.

Des transporteurs comme AgMaT3 pourraient jouer un rôle important dans la tolérance au stress salin en permettant le transport dans la plante d'osmoprotectants comme le mannitol.

Ce transporteur représente un bon candidat en réponse au stress salin, dans le phloème.

### II. C. 2. Les métallothionéines

Quatre séquences correspondent à des métallothionéines (MT). Deux sont parfaitement identiques et appartenaient à la région 3' d'AgMT2 *(Apium graveolens* métallothionéine 2). Une troisième séquence est également homologue à AgMT2, mais située en amont des deux précédentes. Une quatrième séquence correspond également à une métallothionéine mais celle-ci est homologue à AgMT3. Ces dénominations sont attribuées selon Vilaine *et al.* (2003).

Les métallothionénines sont de petites protéines de faible masse moléculaire, riches en cystéines et capables de fixer des métaux (Zn, Cd), trouvées chez toutes les espèces tant animales que végétales et chez les Procaryotes. Elle sont également impliquées dans les réponses aux stress tels que les traitements avec des ions métalliques, le choc thermique (Hsieh *et al.,* 1995), la carence en glucose et en saccharose (Chevalier *et al.,* 1995) ou en cas de fortes concentrations de celui-ci (Chatthai *et al.,* 1997), les faibles températures (Reid et Ross, 1997), la blessure et l'infection virale (Choi *et al.,* 1996).

### II. D. Analyse complémentaire des clones

L'analyse de la banque soustractive a mis en évidence un certain nombre de séquences induites par le stress salin. Si ces banques ont été réalisées à partir d'ARN provenant de phloème, ceci n'exclut pas que les gènes identifiés soient également exprimés dans d'autres tissus. C'est pourquoi la localisation des séquences obtenues est précisée par northern reverse pour valider leur spécificité phloème. Les trente clones présentant les signaux les plus positifs suite à l'analyse de la banque sur filtre sont déposés sur des minifiltres hybridés par de nouvelles sondes complexes, produites à partir d'ARN extrait de phloème ou de xylème de pétioles de Céleris "témoins" ou "stressées" (PhT et PhN ou XT et XN, respectivement). Dix clones sont retenus à l'issue de cette analyse comme spécifiquement induits par le stress salin, avec pour certains d'entre eux un fort rapport phloème/xylème **(tableau II).**

**Tableau II. Séquences d'ADNc obtenues après les hybridations différentielles à partir de la banque soustractive de phloème avec les sondes complexes. La taille approximative de ces fragments est indiquée en paires de bases. Les clones sont classés selon la spécificité de leur expression durant un stress salin dans le phloème. Seuls les clones ayant un rapport supérieur à 2 sont insérés dans ce tableau. Le rapport entre phloème et xylème est calculé après hybridation avec des sondes complexes obtenues à partir d'ARN extraits du phloème et du xylème de plantes stressées par du sel.**

| **Taille (pb)** | **Annotation fonctionnelle** | **Homologie (n° d'accession)** | **BTAST E-value** | **Rapport stress/contrôle** | **Rapport phloème/xylème** |
|---|---|---|---|---|---|
| 500 | oxidoreductase, Zn-binding dehydrogenase | *Arabidopsis* NP_173786 | 51 | 308 | 56 |
| 350 | 14-3-3 family protein | Tomate P933211 | 3e-58 | 130 | 03 |
| 440 | chlorophyll a/b-binding protein | Tomate CAA43590 | 3e-43 | 101 | 04 |
| 350 | metallothionein AgMT3 | Vigne CAB85630 | 2e-09 | 10 | 68 |
| 700 | hypersensitive-induced reaction protein | Orge AAN17454 | 6e-44 | 42 | 38 |
| 650 | NifU-like protein ou dehydrin | *Arabidopsis* NP_193953 | 1e-05 | 33 | 07 |
| 310 | No score | | | 28 | 04 |
| 90 | metallothionein AgMT2 | *P. brachycarpa* AAC62510 | 1e-04 | 24 | 1 |
| 180 | mannitol transporter AgMaT3 | Céleri AAG43998 | 2e-20 | 27 | 1 |
| 800 | beta-galactosidase | *Arabidopsis* T00787 | 2e-35 | 23 | 1 |

Les criblages sont normalisés grâce à une sonde T7 (sonde interne du plasmide utilisé pour le clonage de la banque). Parmi les clones intéressants se trouvent le transporteur de mannitol AgMaT3 et la métallothionéine MT2. On trouve également la métallothionéine MT3, dont le signal dans le phloème témoin est nul mais dont le rapport Phloème/xylème est élevé.

Afin d'affiner ces résultats, des "Northern blot" ont été réalisés en déposant des ARN extraits de phloème, xylème et parenchyme de stockage de pétioles de Céleris "témoins" ou "stressés". Les membranes sont hybridées avec des sondes radiomarquées réalisées à partir des séquences sélectionnées pour leur spécificité tissulaire (les inserts des clones testés sont extraits par digestion et purifiés avant marquage). Les résultats de ces hybridations sont présentés sur la **fig. 3**. Ces hybridations sont quantifiés grâce à une sonde ribosomique 26S de Céleri et les intensités sont mesurées par le système de révélation (Phospholmager : STORM 820). Les valeurs obtenues sont ramenées en pourcentage; le 100 % pour l'expression de chaque sonde dans le phloème "témoin" (PhT)), les comparaisons sont réalisées à partir de ce tissu (**fig.4**).

La sonde *AgMaT3* s'est fixée plus spécifiquement sur le phloème "stressé" (PhN, 225 %), confirmant les précédents résultats. L'expression d'*AgMaT3* est également stimulée par le stress salin dans le parenchyme de stockage (PaN, 171 %), et dans le xylème. Les résultats du Northern confirment que *AgMaT3* est un gène dont l'expression est régulée par le stress salin.

Les 2 métallothionéines *AgMT2* et *AgMT3* sont été et réagissent assez différemment. *AgMT3* est principalement exprimé dans le phloème et cette expression est très fortement stimulée suite à un stress salin (224 %). La stimulation de l'expression *d'AgMT2* dans le phloème suite au stress salin est moins forte (133 %). L'expression de ces 2 métallothionéines est principalement observée dans le phloème chez les plantes témoins et stressées (même si elle est également stimulée dans le xylème suite au stress salin, l'expression reste largement inférieure à celle mesurée dans le phloème. Les métallothionéines sont donc spécifiques du phloème et leur expression, surtout pour *AgMT3,* est effectivement induite par le stress salin.

### II. E. Conclusion

Le but de cette première partie était d'identifier des gènes dont l'expression est induite (ou stimulée) lors d'un stress salin dans le phloème de Céleri. La réalisation de la banque soustractive a nécessité une mise au point pour obtenir un maximum de clones : l'étape finale d'amplification de la banque n'a pu être réalisée qu'en utilisant une souche de bactéries différente de celle préconisée par le fournisseur du kit. Les premières analyses sur un nombre réduit de clones choisis au hasard ont montré que la soustraction était efficace. Le grand nombre de clones obtenus a conduit les inventeurs à chercher un système permettant de contrôler l'efficacité de la soustraction. Les minifiltres réalisés ont permis de tester plus de 700 clones pour leur capacité à hybrider de façon préférentielle des ARN provenant de phloème de plantes stressées que de plantes témoins. La normalisation du signal était réalisée par la sonde T7 présente sur le plasmide de clonage.

Les clones donnant de façon reproductible les signaux les plus intenses ont été séquencés et soumis à une analyse informatique (**tableau I**). Pour affiner ces résultats et vérifier si les séquences identifiées étaient bien spécifiques du phloème et du stress salin, les 29 plasmides correspondant ont été redéposés sur une nouvelle membrane et soumis à des hybridations avec des sondes radiomarquées réalisées à partir de phloème de plantes stressées ou non mais aussi de xylème des mêmes plantes, ne gardant que les séquences présentant une spécificité et induits par le stress salin. Un nombre limité de séquences a ainsi été sélectionné avant une dernière vérification sur Northern, incluant le parenchyme de stockage comme tissu supplémentaire. De ces résultats, il apparaît clairement que les 2 métallothionéines ainsi que le transporteur de mannitol AgMaT3 présentent les profils les plus intéressants.

Les différents résultats obtenus ont amené les inventeurs à poursuivre les travaux par l'identification de promoteurs des gènes intéressants, à savoir les métallothionéines 2 et 3 et le nouveau transporteur de mannitol AgMaT3.

L'étape suivante est donc la recherche du promoteur de ces gènes, à l'aide d'une banque génomique et le suivi de leur activation dans les différents organes et tissus de la plante, lors d'un stress salin.

### III. Caractérisation de AgMaT3, un nouveau transporteur de mannitol du Céleri

L'analyse de la banque soustractive de phloème a permis de retenir trente clones présentant un signal fort en conditions de stress sur les 736 clones déposés sur les membranes. Une séquence partielle (181 bp) a été retenue car elle correspondrait à un transporteur de mannitol un composé qualifié de "soluté compatible" en réponse au stress salin. Aujourd'hui, 2 transporteurs de mannitol ont été identifiés chez le céleri: AgMaT1 et AgMaT2 (Noiraud *et al.,* 2001 a et b).

### III. 1. RACE-PCR sur la séquence partielle d'AaMaT3

Une RACE-PCR est réalisée sur des ADNc produits à partir d'ARN polyA extraits de phloème stressé, auxquels des adaptateurs ont été ligaturés, afin d'amplifier les parties 5' et 3'. Ces séquences d'ADNc 5' et 3' sont amplifiées par PCR entre des amorces choisies sur la séquence partielle de 181 pb (issue de la banque soustractive phloème), et des amorces spécifiques des adaptateurs selon les directives du kit "Marathon cDNA Amplification" (Clontech). Ces amorces sont choisies de telle sorte que les fragments 5' et 3' puissent se chevaucher et s'aligner afin de reconstituer la séquence d'ADNc complète. Une fois les séquences 5' et 3' obtenues, de nouvelles amorces sont dessinées afin d'amplifier l'intégralité de l'ADNc *AgMaT3.* (amorce 5' ; 5' GAC TAG TCC CAA GAA TCT GAG TTC ACC-3'
et amorce 3' ; 5'- CCG CTC GAG CAT CAC AAA GCT ATA ATC C-3'

L'ADNc d'AgMaT3 a une longueur totale de 1796 pb. Il renferme un cadre ouvert de lecture de 1443 pb. Cette séquence a globalement la même taille que les deux autres transporteurs de mannitol (1766 pb pour *AgMaT1* et 1781 pb pour *AgMaT2*)*.*

### III. 2. Analyse de la séquence peptidique déduite de l'ADNc AgMaT3

Les 1443 pb du cadre ouvert de lecture de l'ADNc AgMaT3 codent une protéine de 481 acides aminés, plus courte que celles codées par AgMaT1 (513 aa) et AgMaT2 (524 aa). AgMaT3 montre 82 % et 73 % de similitude avec AgMaT1 et AgMaT2, respectivement. Cette taille plus courte est en fait due à un artefact de clonage. En effet, en utilisant de nouvelles amorces (amorce 5' : 5'-AGC TTC GAC CAT TGT TTC TC-3' et amorce 3' 5'- CCG CTC GAG CAT CAC AAA GCT ATA ATC C-3'), les auteurs de la présente invention sont parvenu à cloner un nouvel ADNc, de longueur supérieure. La séquence correspondant à la protéine la plus longue a été nommée AgMaT3, la séquence correspondant à la protéine la plus courte étant rebaptisée AgMaT3'.

### III. 3. Analyse de la nouvelle séquence nucléotidique de l'ADNc AgMaT3

L'analyse informatique de la séquence génomique d'AgMaT3 a mis en évidence un nouvel ATG (donnant un cadre ouvert de lecture de 1572 bp, SEQ ID N°14). En prenant cet ATG comme point de départ de la traduction (**fig. 6** et **8**) la protéine codée est plus longue (524 aa, SEQ ID N°8) que celle obtenue par RACE-PCR (481 aa). L'alignement des séquences protéiques AgMaT1, AgMaT2 et AgMaT3 prouve que ce résultat paraît beaucoup plus vraisemblable, avec une meilleure homogénéité quant à la longueur et à l'alignement de ces trois séquences (**fig.6**).

### III. 4. Analyse de la nouvelle séquence peptidique déduite de l'ADNc AaMaT3

Les 1572 pb du cadre ouvert de lecture de l'ADNc AgMaT3 codent une protéine de 524 acides aminés, de même taille que celles codées par AgMaT2 et légèrement plus longue qu'AgMaT1 (518 aa) (**fig.7**). La similitude entre ces trois transporteurs est forte, mais nuancée par des portions protéiques différentes (**fig.7****,** espaces non grisés où les acides aminés ne trouvent d'homologue dans aucune des deux autres séquences étudiées), indiquant donc que ces transporteurs de mannitol sont bien distincts et notamment dans les parties N et C terminales. La protéine AgMaT3 de 524 acides aminés a une masse moléculaire théorique de 56 670 Da. Le point isoélectrique de cette protéine a été estimé à 8,3, valeur proche de celles déterminées pour AgMaT1 et AgMaT2 (8,3 et 8,4 respectivement).

### III. 5. Caractérisation fonctionnelle de la protéine AqMa T3

### Expression hétérologue dans la levure

L'analyse des séquences protéiques montre qu'AgMaT3 présente de fortes homologies avec les deux autres transporteurs de mannitol. Les ADNc d*'AgMaT3 et* d*'AgMaT3'* sont clonés séparément dans le vecteur navette pDR 196, puis servent à complémenter la souche RS453. Après transformation, les levures possédant le plasmide *AgMaT3*/pDR sont isolées sur milieu SC glucose pour leur prototrophie à l'uracile, le gène *URA3* étant porté par le plasmide.

Afin de vérifier que le transport de mannitol était réalisé par AgMaT3, la cinétique d'absorption de [³H]-mannitol de la souche complémentée par le plasmide contenant ou non l'ADNc *AgMaT3 ou AgMaT3'* a été suivie. Trois clones indépendants sont testés correspondant à 3 évènements de transformations avec AgMaT3/pDR chez la levure. L'absorption de mannitol marqué au tritium a été suivie en fonction du temps (**fig.5**). Les levures exprimant le plasmide *AgMaT3*/pDR absorbent beaucoup plus de mannitol que les levures transformées avec pDR. Les valeurs d'absorption à 3 min sont très proches de celles notées pour AgMaT1 dans les mêmes conditions de mesure. Ce résultat indique donc que AgMaT3 est un transporteur de mannitol. On notera que les résultats obtenus lors de l'absorption de mannitol par les levures *AgMaT3'*/pDR/RS453 indiquent que ce transporteur est fonctionnel alors que la protéine est délétée d'une quarantaine d'acides aminés.

### III. 6. Clonage de la séquence génomique AgMaT3

Le clonage des séquences génomiques est réalisé sur le même principe que celui des promoteurs, avec le kit "Universal Genome Walker" (Clontech) utilisant des amorces orientées en sens inverse de celles qui ont permis de séquencer le promoteur. La séquence codante d'AgMaT3 est entrecoupée par deux introns de 264 et 805 pb (**fig. 8**). La séquence génomique partielle représente 2772 pb, à partir du codon ATG. Les exons déduits de cette séquence génomique correspond parfaitement aux séquences d'ADNc sauf au niveau de l'initiation de la traduction. Présence d'un site de polyadénylation AATAAA en position 2822 (ou 179 pb après le codon stop) sur le brin sens (+) de la séquence génomique *d'AgMaT3* (**fig. 8**).

### III. 7. Analyse de l'expression d'AgMaT3 dans différents tissus de Céleri avant subi ou non un stress salin

Cette analyse est réalisée par RT-PCR en temps réel sur différents tissus (xylème, phloème, feuilles, racines et parenchyme) de plantes traitées ou non au NaCl 300 mM pendant trois semaines, afin d'étudier le niveau d'expression et leur stimulation en réponse au stress.

Les valeurs obtenues avec la sonde AgMaT3 sont normalisées par la sonde ribosomique 26S de Céleri. Une différence de 3,42 Ct (voir ΔΔ Ct dans le **tableau III)** entre phloème "témoin" et "stressé" indique qu'il faut 3,42 cycles supplémentaires pour obtenir le signal dans le phloème témoin de même intensité que dans le phloème "stressé" ; L'expression d'AgMaT3 est plus importante dans le phloème des plantes traitées au NaCl. De plus, les cycles de PCR permettant une amplification exponentielle, une différence de 3,42 Ct correspond à 2^{3,42}, soit 10,70 fois plus de transcrits AgMaT3 dans le phloème de plantes "stressées" que des "témoins". Ce résultat confirme ceux précédemment obtenus dans la banque soustractive et par Northern (**fig.3** et **tableau II**). De même, une différence de 7,52 Ct (voir ΔΔ Ct dans le **tableau III**) entre racines "témoin" et "stressées" indique qu'il faut 7,52 cycles supplémentaires pour obtenir le signal dans les racines témoin de même intensité que dans les racines "stressées".

**Tableau III. Résultats obtenus par RT-PCR en temps réel avec la sonde AgMaT3, dans différents tissus (phloème, xylème, parenchyme, feuilles, racines) de plantes témoins ou traitées au NaCl (300 mM pendant 3 semaines). Ces résultats ont été corrigés par les valeurs de la sonde ribosomique standard 26S de Céleri. Ct correspond au nombre de cycles nécessaires pour percevoir un signal d'intensité donné. Δ Ct est la différence entre les moyennes des Ct (moyennes des répétitions pour une sonde analysée dans un tissu donné) obtenus pour la sonde AgMaT3 après soustraction de la moyenne des Ct obtenus pour la sonde 26S. ΔΔ Ct correspond à la comparaison (différence entre deux Δ Ct ) entre les différents tissus. Plus la valeur de Ct est élevée, plus il a fallu de cycles pour obtenir un signal et donc moins il y avait de transcrits dans ce tissu.**

| **Tissu testé** | Δ **Ct AgMaT3 - 26S** | ΔΔ **Ct** |
|---|---|---|
| Phloème Témoin | 19,72 | 3,42 |
| Phloème NaCl | 16,31 | PhN>PhT |
| Feuilles Témoins | 19,05 | 0,58 |
| Feuilles NaCl | 19,63 | FN<FT |
| Xylème Témoin | 21,88 | 3,17 |
| Xylème NaCl | 18,72 | XN>XT |
| Racines Témoin | 21,13 | 7,52 |
| Racines NaCl | 13,61 | RN>RT |
| Parenchyme Témoin | 17,50 | 1,03 |
| Parenchyme NaCl | 16,47 | PaN>PaT |

### III. 8. Conclusion sur AgMaT3

Les informations obtenues lors de l'absorption de mannitol en fonction du temps ainsi que les alignements des séquences nucléotidiques et protéiques permettent de conclure qu'AgMaT3 code un troisième transporteur de mannitol chez le Céleri branche. Les études par RT-PCR en temps réel et par Northern confirment que l'expression d'*AgMaT3* est spécifique du stress salin. Ce transporteur de mannitol jouerait donc un rôle important dans la tolérance au stress salin.

### IV. Recherche de promoteurs et expression hétérologue chez A. thaliana

Pour rechercher des promoteurs spécifiquement induits par le stress salin dans le phloème, il était souhaitable de pouvoir disposer d'un système performant de clonage de promoteurs (les étapes de criblage d'une banque génomique sont particulièrement lourdes). De plus, une première tentative de recherche de promoteur du gène AgMaT1, à partir d'une banque génomique dans un phage λ, avait été sans succès (Cyril Maingourd, 2001). Dans l'optique de recherche de plusieurs promoteurs, il devenait nécessaire d'utiliser une technique plus rapide. C'est pourquoi la technique d'amplification par PCR de régions promotrices à partir des séquences ADN (Universal GenomeWalker kit, clontech) a été utilisée. La recherche de séquences promotrices chez le Céleri a été effectuée sur les gènes choisis : *AgMaT3* codant le nouveau transporteur de mannitol et les deux gènes de métallothionéines (*AgMT 2* et *AgMT* 3)*.*

### IV. A. Banque génomique et clonage de promoteurs

L'ADN génomique de Céleri est séparé en quatre lots sur lesquels on fait agir quatre enzymes de restriction différentes, EcoRV, Dral, Sspl et Hpal, laissant des extrémités à bord franc, des adaptateurs spécifiques y étant ligaturés. On obtient ainsi 4 fractions différentes sur lesquelles sont réalisées des PCR avec une amorce spécifique de la séquence à amplifier (choisie proche du codon ATG de début de traduction) et une amorce spécifique de l'adaptateur. Les fragments obtenus sont de taille variable avec un profil particulier à chaque digestion. Seuls les fragments amplifiés les plus longs sont retenus pour le clonage et le séquençage. Quelques mises au point sont nécessaires pour l'obtention de cette banque quant au choix des enzymes de restrictions (une des enzymes conseillées par le fournisseur ne coupant pas l'ADN de Céleri) et du matériel utilisé. Pour chaque gène étudié, une région promotrice en amont de l'ATG est clonée dans un vecteur intermédiaire (pDONR 207) puis transférée dans un vecteur d'expression binaire (pBi-GUS-R1R2 et pBi-GFP-R1 R2) par le système Gateway (Invitrogen). Ceci permet l'étude de l'expression de gènes rapporteurs (GUS ou GFP) sous contrôle des promoteurs décrits, après transformation de plants *d'Arabidopsis.*

Les constructions réalisées ont servi à transformer les agrobactéries. Après vérification de la présence des plasmides dans les agrobactéries, des plantes *d'Arabidopsis* sont transformées selon la technique décrite dans la partie Matériels et Méthodes.

### IV. B. Promoteur du gène codant le transporteur de mannitol AgMaT3

Le promoteur d'*AgMaT3* représente 589 pb en amont de l'ATG (**fig. 8****,** SEQ ID N°4). Les régions promotrices d*'AgMaT 1* et *AgMaT* 2 ont également été clonées et font 844 et 383 pb en amont de l'ATG. Ces trois séquences contiennent de nombreuses répétitions A/T caractéristiques des régions promotrices.

Les sites de liaison de facteurs de transcription en réponse à différents stimulus sont recherchés grâce au logiciel PLACE (http://www.dna.affrc.go.jp/sigscan/). Seuls les éléments cis régulés par le signal sucre et la lumière sont analysés. La position des cassettes est indiquée par rapport au site d'initiation de la traduction (codon ATG). Les protéines régulatrices peuvent se fixer sur le brin sens, noté (+) ou sur son complémentaire, le brin anti-sens, noté (-).

### IV. B. 1. Réponse à la lumière

La boîte GATA (GATABOX, S000039), requise pour une régulation par la lumière (Teakle *et al.,* 2002) a été identifiée 7 fois dans le promoteur d'*AgMaT3* (en -299 (-), -289 (-), -269 (+), -264 (+), -254 (-), - 154 (+) et -77 (+)). Le site de liaison GT-1 (GT1CONSENSUS, S000198) (Terzaghi et Cashmore, 1995) est présent 5 fois en amont *d'AgMaT3* (-392 (-), -291 (-), -264 (+), -201 (-) et -154 (+)). La séquence GATAA (I-BOX, S000199), très conservée en amont de gènes régulés par la lumière aussi bien chez les mono- et dicotylédones (Terzaghi et Cashmore, 1995), est retrouvé 3 fois en amont *d'AgMaT3* (en -290 (-), -264 (+) et -154 (+)).

La "Tbox" (TBOXATGAPB, S000383, ACTTTG), impliquée dans l'activation de gènes par la lumière, a été trouvée dans le promoteur d'*AgMaT3* en -455 (-). La transcription en réponse à la lumière du gène *psaDb* de Tabac dépend de l'élément Inr ("initiator" INRNTPSADB, S000395, C/TTCANT(C/T)₂, élément présent 2 fois en amont *d'AgMaT3* (-387 (+) et -189 (-)).

### IV. B. 2. Réponse aux sucres

Les sucres sont maintenant considérés comme des molécules capables de réguler l'expression de nombreux gènes.

La boîte PYRIMIDINEBOXOSRAMY1A (CCTTTT, S000259), de réponse à la gibbérelline et également impliquée dans la répression par les sucres (Morita *et al.,* 1998) est présente dans la séquence promotrice d'*AgMaT3*, deux fois (-398 (+) et -219 (-)). L'élément TATCCA (TATCCAOSAMY, S000403), site de fixation de protéines MYB (Lu *et al.,* 2002) est présent une seule fois dans le promoteur d'*AgMaT3* (-299 (+)). L'élément "W-box" (WBOXHVISO1, S000442, TGACT), site de liaison du facteur de transcription SUSIBA2 (sugar signaling in barley, inductible par le sucre), est identifié à trois reprises dans le promoteur d'*AgMaT3* (-419 (-), -367 (-) et -74 (-)). Ces éléments suggèrent une régulation par les sucres (glucose, saccharose).

### IV. C. Promoteurs d'AgMT2 et 3

Les métallothionéines ont été trouvées à la fois induites dans le phloème et en cas de stress salin, mais aussi en cas d'attaque par les pucerons et les virus (Fanchon Divol, 2003). Les résultats de la présente analyse du stress salin ont montré que les métallothionéines AgMT2 et AgMT3 sont spécifiquement exprimées en réponse à ce stress (**fig. 3**).

Les régions promotrices représentent 1319 et 648 pb et ont été clonées en amont des gènes rapporteurs (GUS et GFP) des vecteurs pBi, pour *AgMT2* et *AgMT3* (**fig. 9** et **10**).

### IV. D. Analyse des cassettes de réponse au stress

L'analyse informatique des promoteurs a révélé de nombreux éléments-cis potentiels liés à des stress abiotiques.

### IV. D. 1. Réponse à des stress abiotiques

L'acide abscissique (ABA) est présent chez toutes les plantes supérieures. Cette phytohormone intervient dans plusieurs événements du développement des graines et régule l'expression de nombreux gènes en réponse aux stress environnementaux comme la déshydratation, le sel et le froid (Busk et Pages, 1998). L'analyse des promoteurs d*'AgMaT3, AgMT2 et AgMT3* a mis en évidence des éléments cis-régulateurs ABRE (ABA-responsive element), sur lesquels de nombreux ABF (ABRE binding factor, membres d'une sous-famille de protéines bZIP) se lient en réponse à une voie de signalisation ABA dépendante (Kang *et al.,* 2002), afin d'induire l'expression d'un grand nombre de gènes (Choi *et al.,* 2000). Chez *Arabidopsis,* l'induction du gène *rd22* par l'ABA et la déshydratation fait intervenir des facteurs de transcription MYC et MYB (Abe *et al.,* 2003 et 1997) se liant sur sites spécifiques CANNTG (MYCCONSENSUAT, S000407) ou C/TAACG/TG (MYB2CONSENSUSAT, S000409), A/TAACCA (MYB1AT, S000408). La séquence CANNTG (S000407) est également le site de liaison d'ICE (inducer of CBF expression) qui régule la transcription des gènes codant un CBF, en réponse au froid (Chinnusamy *et al.,* 2003). Le site S000407 est présent en amont *d'AgMaT3* (-550 (+/-), **fig. 8** et **11**) et d*'AgMT2* (-369 (+/-) et -158 (+/-), **fig. 9** et **11**). Ce site n'a pas été identifié dans le promoteur *d'AgMT3* (**fig. 10** et **11**). La séquence S000409 a été analysée en amont du gène *AgMT2* en -820 (+), *d'AgMT3* en -575 (+). S000408 est présent dans le *promoteur AgMT2* en -642. Un facteur de transcription MYB d*'Arabidopsis,* induit par la déshydratation et le stress salin (Urao *et al.,* 1993) reconnaît et se lie à la séquence consensus TAACTG (MYB2AT, S000177). Celle-ci a été caractérisée en amont *d'AgMT3* en -575 (+).

La séquence CNGTTG/A (MYBCORE, S000176) représente le site de fixation de MYB qui est également induit par le stress hydrique chez *A. thaliana.* Cette séquence est identifiée dans *le promoteurs d'AgMT2* en -820 (-) et *AgMT3* (-575 (-) et -563 (-)). L'expression thermorégulée d'un gène hs (heat shock) de Soja (Rieping et Schoffl, 1992) est régulée via un élément HSE du promoteur (heat shock element), mais cette activité requiert des séquences additionnelles : des boîtes CCAAT (CCAATBOX1, S000030). Celles-ci sont présentes en amont du gène *AgMaT3* (-176 (+)). Cette boîte n'est pas présente en amont *d'AgMT3* alors qu'elle a été identifiée six fois dans le promoteur d'AgMT2 en -1280 (-), -834 (-), -1263 (-), -693 (-), -1057 (-) et -624 (+).

### IV. D. 2. Réponse à des stress biotiques

De nombreuses voies de signalisation en réponse aux stress biotiques et abiotiques sont entrecroisées. Les éléments de liaisons de facteurs de transcriptions en réponse à des attaques pathogènes et à la blessure ont également été retenus comme candidats potentiels lors de l'induction par le stress salin. Quelques-uns d'entre eux ont été énumérés lorsque leurs motifs ont été détectés sur les promoteurs d*'AgMaT3, AgMT2* et *AgMT* 3*.*

L'expression de nombreux gènes de défense sont régulés par l'intermédiaire d'éléments cis tels que la boîte GCC (GCCCORE, GCCGCC, S000430), identifiée en -284 (-) en amont *d'AgMaT3.*

Le motif AG (AGATCCAA, AGMOTIFNTMYB2, S000444) est un élément suffisant pour conférer une réponse à la blessure et suite à un traitement éliciteur (Sugimoto *et al.,* 2003). Le motif S000444 a été retrouvé dans *le promoteur d'AgMT3* en -452.

Les éléments les plus importants en réponse aux stress biotiques sont les boîtes W, sites de fixation des protéines WRKY (superfamille de facteurs de transcription, impliqués dans la régulation de programmes physiologiques variables comme la défense contre un pathogène, la sénescence et le développement des trichomes (Eulgem *et al.,* 2000)). Elles se lient aux boîtes W séquence palindromique TGAC comme la "WB box" (WBBOXPCWRKY1, TTTGACT, S000310), présente en amont *d'AgMaT3* en -74 (-) et d*'AgMT2* en -169 (+).

Le gène NPR1 *d'Arabidopsis,* régulateur positif de la résistance inductible contre les maladies, contient dans son promoteur la séquence "W-box" (WBOXATNPR1, TTGAC, S000390), site de fixation de WRKY induites par une infection pathogène ou un traitement à l'acide salicylique (Yu *et al.,* 2001). Celui-ci a été identifié trois fois dans *les promoteur d'AgMaT3* (-418 (-), -366 (-) et -73 (-)), d'*AgMT2* en -168 (+) et d'Ag*MT3* en -496. Ces éléments ont été placés en fonction de leur emplacement sur les régions promotrices d*'AgMaT3* d*'AgMT2* et *d'AgMT3* (**fig**. **11**)

La séquence promotrice *dAgMT2* comporte peu de sites de liaison MYB, MYC ou en réponse à l'ABA mais pas moins de cinq éléments HSE sur le brin (-) et un sur le brin (+), ainsi qu'un élément LTRE. L'expression de ces promoteurs en amont d'un gène rapporteur, dans les tissus et organes de la plante, est donc intéressante à suivre lors d'un stress salin.

### IV. E. Analyse fonctionnelle des régions promotrices par expression hétérologue chez A. thaliana

Chaque séquence identifiée et clonée chez le Céleri a été placée en amont de deux gènes rapporteurs GUS et GFP, afin de suivre leur expression chez *A. thaliana.* Le clonage a été réalisé en utilisant la technologie "gateway" (Invitrogen) entre les bordures droite et gauche d'un vecteur pBl. Les régions en amont de l'ATG qui ont été clonées pour chacun des gènes sont montrées dans la **fig. 12**. Les longueurs des régions clonées sont 548 (AgMaT3), 649 (AgMT3) et 1319 paires de bases (AgMT2). Toutes les séquences clonées incluent le site putatif d'initiation de la transcription (situé en position 541 pour AgMaT3, en position 1502 pour AgMT2 et en position 639 pour AgMT3).

La transformation effective des plantes est doublement vérifiée, par germination en présence de kanamycine et par contrôle PCR avec des amorces spécifiques NPT-II. Pour toutes les expériences, deux contrôles sont ajoutés : une lignée Col10 non transformée et des plantes transformées par le gène *uiad* sous le contrôle du promoteur AtPP2-1 (Dinant et al, 2003), qui donne un fort signal dans le phloème. Les expériences sont répétées sur 5 plantes pour chaque construit de promoteur. La survie des plantes est testée après un traitement au NaCl à des concentrations comprises entre 50 et 250 mM. La concentration idéale pour induire une réponse au stress salin, sans être létal pour la plante, ni empêcher le développement d'une hampe floral et la production de graines, a été déterminée à 100 mM.

La réponse est suivie par une inspection visuelle de la coloration des feuilles avant et après un stress salin et de la section transversale pour vérification de la coloration du phloème.

L'observation générale des feuilles des plantes contrôles (non soumises au stress) ne fait apparaître, après coloration de GUS, aucune coloration spéciale sauf dans le cas de AtPP2-1 et AgMT2. Dans tous les cas, la coloration est limitée aux nervures. Pour AtPP2-1 et AgMT2, toutes les nervures sont colorées positivement (type I à IV, f**ig. 13C**) alors que dans le cas de AgMaT3 et AgMT3, seules les nervures majeures (type I) sont colorées (**fig. 13A et E**). Dans tous les cas, les nervures dans les pétioles sont également colorées. De plus, les jeunes feuilles et celles matures sont colorées de façon identique.

Pour déterminer la localisation exacte de l'expression de GUS dans les faisceaux vasculaires, des sections transversales sont réalisées sur du matériel congelé. Pour les plantes montrant une forte coloration (AtPP2-1 et AgMT2 **fig. 13D**), la coloration bleue est évidente dans les cellules du phloème mais également parfois dans les cellules environnantes. Cela peut être dû à la diffusion de la coloration, du fait qu'il a déjà été montré que la coloration GUS est limitée aux cellules du phloème dans le cas de AtPP2-1 (Dinant et al 2003). Pour les construits AgMaT3 (**fig. 13B**) et AgMT3 (**fig. 13F**), la coloration est aussi évidente dans les cellules du phloème, confirmant ainsi l'identification initiale des ADNc correspondant comme spécifiques des cellules du phloème. Une coloration est également détectée des les cellules parenchymes du xylème. Parmi ces cellules se trouvent vraisemblablement des cellules associées aux vaisseaux (VAC « vessel-associated cells »), qui sont considérées comme très similaires du point de vue de la fonction aux cellules comparables du phloème (Fromard et al, 1995).

L'induction par un stress salin peut être soit spécifique du présent test, soit faire partie d'une réponse de la plante au stress d'ordre plus général. Afin de tester cette hypothèse, des groupes de 5 plantes provenant des mêmes transformants ont été soumis à un stress osmotique (absence d'arrosage pendant 4 jours), à un stress au froid (4 jours à 4°C), à un stress à la chaleur (4 jours à 30°C) et à un stress par blessure (feuilles pincées avec une pince 3 fois de suite, en prenant soin d'éviter les nervures majeures. Les plantes ont été récoltées le jour suivant le stress. Un groupe de 5 plantes indépendantes a été utilisés comme contrôle. Les plantes sont colorées comme pour le stress salin. Toutes les plantes (excepté pour les stress hydrique et salin) sont arrosées deux fois par jour avec 10mL d'eau du robinet ou une solution d'engrais (une fois pendant la période de stress). Les feuilles (totalement matures ou les jeunes pousses) sont échantillonnées à la fin de la période de stress et utilisées pour la coloration histochimiques GUS (Jefferson et al, 1987). D'autres groupes de feuilles sont congelées dans l'azote liquide et stockées à -80°C jusqu'aux tests GUS fluorimétriques (Jefferson et al, 1987).

Pour les plantes AgMT2, il a été difficile d'observer une différence entre les différents stress, dans la mesure où la coloration est déjà particulièrement intense dans les plantes contrôles. Toutefois, aucune différence n'a été observée dans le profil de coloration. Dans le cas de AgMaT3, toutes les formes de stress donnent des profils de coloration similaires, à l'exception du stress hydrique et du stress par blessure où aucun signal n'a été détecté, bien que les plantes soient toujours en bonne santé. Dans les plantes AgMT3, la coloration GUS est évidente dans toutes les formes de stress, à l'exception du stress par blessure.

Les réponses au stress par blessure utiliseraient une voie de signalisation différente et ne conduisent pas à l'activation des gènes étudiés.

Les résultats sont très semblables à ceux obtenus avec le céleri. Aussi bien la région 5' de AgMT3 que celle de AgMaT3 conduisent à l'expression du gène rapporteur dans le phloème (et dans une certaine mesure également dans le xylème), mais uniquement dans les plantes stressées et non dans les plantes contrôles. Dans le cas de AgMT2, une expression importante a été observée dans le phloème de plantes Arabidopsis transformées, et ce même dans des conditions de contrôle. Un point intéressant est le fort taux d'expression de la fusion entre le promoteur AgMT2 et GUS dans Arabidopsis dans toutes les nervures de la feuille (**fig. 13C**), ce qui indique que le profil d'expression est spécifique du phloème.

### Exemple 3 : transformation de Brassica

**Protocole de tranformation Brassica:** Dérivé de Brasileiro et al, 1992 et de Bethomieu (thèse de doctorat ; Obtention de Choux transgéniques tolérants aux noctuelles par transformation génétique avec un gène codant pour une endotoxine de *Bacillus thuringiensis*).

### 1. Semis et Germination

Les graines sont désinfectées 25 minutes sous agitation dans une solution de bayrochlore (3 comprimés/litre d'eau déminéralisée) avant d'être rincées à l'eau stérile puis séchées sur buvard. Stérilisées, elles sont semées dans des tubes sur un milieu de germination M1 (4.4 g/I de MS 5519 9 (Sigma), 20g/l de saccharose et 8g/l d'agar-agar (pH 5.8)). Les graines sont cultivées 15 jours à 20°C (photopériode de 16h) pour obtenir des plantules au stade 2-3 feuilles.

### 2. Culture des bactéries

On utilise 2 Agrobactéries: la C 58pMP90 contient le plasmide pSCV avec le T-DNA comportant la cassette d'expression du gène reporter (gène GUS), sous le contrôle des séquences promotrices de la présente invention (sélection Kanamycine :50 µg/mL; Rifampicine :20 µg/mL), la seconde contenant le t-DNA tumorigène (pTl82-139) (sélection Kanamycine :50 µg/mL; Rifampycine :50 µg/mL et Gentamycine :20 µg/mL)

Les bactéries sont pré-cultivées dans 20ml de milieu LB (Luria Bertani de Difco) contenant les antibiotiques de sélection plus 1 ml de glycérol-stock de la bactérie. Ces pré-cultures sont mises sous agitation (200 rpm), à 28°C pendant 24h. Les cultures sont réalisées en ajoutant 1 ml de la pré-culture à 20 ml de milieu LB contenant les antibiotiques de sélection. Les cultures sont mises sous agitation (200rpm), à 28°C, durant une nuit.

### 3. Préparation des bactéries

Si la densité optique à 600nm des 2 cultures d'agrobactéries après une nuit n'est pas de 1, elles sont centrifugées 10mn à 4000rpm. Le culot bactérien est repris avec un volume suffisant du milieu liquide M4 contenant 0.88 g/l de MS 5519 et 10g/l de saccharose (pH 5.8) afin d'arriver à la DO recherchée. Les 2 bactéries sont mélangées suivant le ratio Bact 2/Bact 1: 1/6

### 4. Inoculation des plantes et repiquage

Les plantules sont inoculées au moyen d'une pince à griffes DEMARTEL 20 cm, trempée dans le mélange bactérien et utilisée pour blesser la tige en trois points différents, au-dessus des cotylédons. Les plantules inoculées sont cultivées à une température de 20°C (photopériode 16h) jusqu'à apparition des tumeurs aux points de blessure. Les tumeurs sont repiquées sur un milieu M2 contenant 4.4 g/l de MS 5519, 30g/l de saccharose, 8g/l d'agar-agar, 400mg/l d'antibiotique (Augmentin) (pH 5.8)

### 5. Régénération des bourgeons

Environ 1 mois après le repiquage des tumeurs, les bourgeons apparaissent. Lorsqu'ils ont une taille de 3 à 4 cm, ils sont isolés et mis en tubes sur un milieu M3 contenant 4.4 g/l de MS 5519, 30g/l de saccharose, 8g/l d'agar-agar (pH 5.8) 200mg/l d'antibiotique (Augmentin) (pH 5.8).

Les tumeurs sont fragmentées et repiquées sur milieu M2 pour régénérer d'autres bourgeons. Des plantules sont enracinées 3 à 4 semaines après isolement des bourgeons sur milieu M3.

### 6. Acclimatation des plantes

Les plantules sont transférées en godets de terreau et maintenues à 22°C et 80% d'humidité relative pendant une semaine. Elles sont ensuite conduites comme des plantes issues de semis (température 20°C et hygrométrie ambiante), rempotées dans des pots de culture pour la production de graines. Les siliques arrivent à maturité en 5 mois.

### Exemple 4 : transformation de tomates.

Protocole de transformation Tomate (système de transformation binaire):
Dérivé de Filatti et al, 1987.

### 1/ Semis et germination

Les graines, placées dans un carré de moustiquaire fermé par des agrafes, sont désinfectées dans une solution de bayrochlore (2 à 3 pastilles par litre d'eau distillée et quelques gouttes de Tween) durant 20 minutes. Elles sont ensuite rincées trois fois pendant 15 minutes dans de l'eau distillée stérile, puis séchées rapidement dans du papier buvard. Les graines stérilisées sont semées dans des bocaux sur un milieu de germination T1 (MS 6899, Sigma) comprenant 2,2 g/l de MS 6899, 2ml/l de Vitamines de Nitsh & Nitsh, 1965, 30g/l de saccharose (pH 5.9, ajusté avec KOH), et 8g/l d'agar-agar. Les boites sont laissées 24 heures à l'obscurité, dans une étuve à 26°C. Placées à la lumière, les cotylédons se développent. Ils sont utilisables 7 à 8 jours après le semis.

### 2/ Préculture des explants sur TT2 solide

Un explant est découpé par cotylédon et placé ensuite sur le milieu T2 de préculture comprenant 4,4 g/l de MS 6899 2ml/l de Vitamines de Nitsh & Nitsh, 0,9 mg/l de Thiamine, 200mg de Potassium dihydrogenophosphate (KH2PO4), 30g/l de saccharose (pH 5.9, ajusté avec KOH), 8g/l d'agar-agar, 0,2mg/l de 2,4-D (Sigma), 0,1mg/l de Kinétine (Sigma), 200µmol/l d'Acétosyringone (Aldrich). La face inférieure des explants est posée contre le milieu. Les boites sont laissées à la lumière, à 26°C pendant 24h.

### 3/ Préparation des bactéries :

On utilise l'agrobactérie EHA 105 contenant le plasmide pBl101 avec le T-DNA comportant la cassette d'expression du gène de sélection (gène de résistance à la kanamycine) et le gène rapporteur (gène Gus de la béta-glucuronidase, sous le contrôle des séquences promotrices selon la présente invention). (antibiotique Kanamycine :50 µg/mL; Rifampicine :50 µg/mL)

Une pré-culture des bactéries est faite dans un tube Falcon de 50ml, par ensemencement de 1ml de glycérol-stock de la bactérie dans 10 ml de milieu de culture de milieu LB ou TP (Yeast-Tryptone de Difco) contenant les antibiotiques de sélection. Ces pré-cultures sont mises sous agitation (250 rpm), à l'obscurité, à 28°C pendant 24h. Les cultures sont réalisées en ajoutant 1 ml de la pré-culture à 20 ml de milieu LB ou YT contenant les antibiotiques de sélection. Les cultures sont mises sous agitation (250rpm), à l'obscurité, à 28°C pour la nuit. Le lendemain matin, la DO à 600nm d'un ml de culture bactérienne est mesurée. Si différente de 2, les cultures sont centrifugées pendant 10mn à 3000 RPM. Le surnageant est éliminé et le culot bactérien re-suspendu avec un volume adéquat de milieu T2 liquide.

### 4/ Coculture :

A 28,5ml de milieu de coculture (milieu T2 liquide avec seulement l'acétosyringone, sans Kinétine ni 2,4-D) par boîte de Pétri, sont ajoutés 2,5ml de solution bactérienne à la DO₆₀₀ₙₘ2.

Les explants sont prélevés sur le milieu de préculture et ajoutés dans les boites de Petri pour incuber avec les bactéries durant 30 minutes. Les explants sont ensuite séchés sur du papier buvard stérile puis replacés sur le même milieu T2 solide ayant servi pour la préculture. Les boites sont placées à l'obscurité, à 26°C pendant 48h.

### 5/ Lavage des explants :

Environ 150mL de milieu de lavage liquide MS 6899 est mis dans chaque pot stérile (usage unique) et on y fait tremper 50 explants au maximum (milieu T3 : 4,4g/L de MS 6899, 2mL/L de vitamines de Nitsh &Nitsh, 30g/L de Saccharose et 400mg/L d'Augmentin (pH 5.9 ajusté avec KOH)). Après une dizaine de minutes de trempage on récupère les explants à l'aide d'une pince et on les sèche rapidement sur du papier buvard stérile.

### 6/ Repiquage des explants en milieu de régénération :

Les explants sont repiqués sur milieu T3 solide coulé en boîtes de Pétri hautes. Milieu T3 solide : 4,4g/l de MS 6899, 2 ml/l de vitamines de Nitsh &Nitsh, 30g/l de Saccharose, 8g/l d'Agar-agar, 2mg/l de Zéatine *(Sigma),* 500mg/l d'Augmentin (600mg/l lorsqu'on utilise la bactérie LBA4404), 100mg/l de Kanamycine *(Sigma).* A l'issue de 2 semaines à la lumière à 26°C, les explants sont coupés en deux afin de différencier les événements de transformation (les 2 extrémités d'un même explant sont considérées comme distinctes) et les repiquer sur un milieu T3 neuf. Le nombre d'explants ayant régénéré 21 jours après la coculture est compté.

### 7/ Préparation du milieu de développement

Les bourgeons sont séparés de l'explant primaire 4 à 6 semaines après la coculture et placés sur milieu sélectif T4 (2,2g/l de MS 6899, 2 ml/l de vitamines de Nitsh &Nitsh, 20g/l de Saccharose, 8g/l d'Agar-agar, 0,5mg/l de Zéatine *(Sigma),* 300mg/l d'Augmentin (600mg/l lorsqu'on utilise la bactérie LBA4404), 50mg/l de Kanamycine *(Sigma).*

### 8/ Enracinement des bourgeons transformés :

Lorsque les bourgeons atteignent quelques centimètres de haut, ils sont repiqués en gros pots TPS sur le milieu d'enracinement T5 : 2,2g/l de MS 6899, 2 ml/l de vitamines de Nitsh &Nitsh, 20g/l de Saccharose, 2,5g/l de Phytagel, 0,5mg/l d'AIA *(Sigma),* 300mg/l d'Augmentin (600mg/l lorsqu'on utilise la bactérie LBA4404), 50mg/l de Kanamycine *(Sigma).* Les plantules synthétisant de la chlorophylle et développant des racines sont considérées comme transformées. Après détermination du taux de ploïdie de chaque plantule par cytométrie de flux ou comptage des amyloplastes, les plantules diploïdes sont acclimatées puis plantées en serre.

### 9/Acclimatation- Plantation en serre

Les plantules bien développées sont enracinées en godets de 10 cm, sur du *Steckmedium.* Elles sont recouvertes pendant deux semaines d'un film plastique afin de maintenir une forte hygrométrie. Les plantules sont alors plantées en pots de 7 litres remplis de *Pouzzolane.*

### Mise au point du protocole de stress salin

Un semis de graines de tomates (Kemer) non transgéniques a été effectué en conditions classiques de germination : 10 graines par boîte plastique avec papier buvard imbibé d'eau additionné de NaCl à différentes concentrations (H²O, 50, 100, 150, 200, 250 et 300 mM), à raison de deux boîtes par concentration.

Les graines ont été maintenues à l'obscurité durant 3 jours, puis ensuite exposées à la lumière, à une température de 25°C. Les observations ont été réalisées 5 à 6 jours après la germination.

Le Témoin H²O présente 90% de germination, les plantules (stade cotylédons) mesurent environ 2 cm. Les graines ayant reçu 50 mM de NaCl présentent 45% de germination et la taille des plantules est de 1 cm. Les graines ayant reçu 100 mM de NaCl et au delà ne présentent pas de germination au moment de l'observation, et n'ont pas germé 2 semaines plus tard.

La dose de 50 mM de NaCl a donc été retenue pour la réalisation du stress salin.

### Protocole de test GUS

### 1/Préparation du tampon de réaction.

Une solution A de Na2HPO4 à 0,2 M (Merck, N°238) 28,4 g/l est mélangée progressivement avec une solution B de NaH2PO4 à 0,2 M (Merck, N°6) 24 g/l jusqu'à obtention d'un pH 7, 5mg de X.glucuronide (Clontech) sont dissous dans 50µl de Diméthyl Formamide, et 5ml de NaPO4 0,2 M, pH7 (Solution A+B) sont ajoutés ensuite pour obtenir le tampon de réaction.

### 2/ Réalisation du test

200µl de tampon de réaction sont déposés dans les puits d'une plaque Elisa (96 puits), ou 700µl pour une plaque 24 puits. Les fragments de tissus (tiges, feuilles etc...) coupés très finement à l'aide d'un scalpel sont ensuite ajoutés. La plaque est incubée dans une étuve à 37°C pendant 18 heures environ. L'apparition d'une coloration bleue révèle l'activité de l'enzyme GUS. Plusieurs rinçages à l'alcool à 95° peuvent être effectués afin de décolorer les fragments de tissus, pour faciliter la lecture (facultatif).

L'intensité de coloration bleue est relevée selon le barème suivant :

| | |
|---|---|
| 0 | nulle |
| 1 | + |
| 2 | ++ |
| 3 | +++ |

### Protocole de préparation des coupes tissulaires pour les plantes transformées avec le construit pAgMT2-GUS-tNOS

Les tiges GUS positives sont incluses verticalement dans un plot d'agar à 6% ; le plot est ensuite collé sur la platine du vibratom (appareil électronique équipé d'une lame de rasoir permettant de réaliser des coupes fines de 1 µm), pour obtenir des coupes de 40 µm dans notre étude.

Ces coupes sont récupérées sur lame dans une goutte d'un mélange eau/Glycérol 50/50 ; elles sont ensuite observées entre lame et lamelle au microscope optique (Type LEICA).

### Résultats

Des plants de tomates (*Solanum lycopersicum*) du génotype KEMER ont été transformés selon le protocole décrit plus haut avec l'agrobactérie EHA 105 contenant le plasmide pBl101 avec le T-DNA comportant le construit pAgMat3-Gus-tnos ou pAgMT2-Gus-tNos. Dix à vingt évènements de transformation indépendants ont été obtenus pour chaque construit (plantes To).

Des tests PCR ont été réalisés sur les plantes T0 au moyen d'amorces amplifiant une séquence chevauchante entre le promoteur d'intérêt et le gène GUS, afin de contrôler si les plantes ont effectivement été transformées et comprennent le construit.

Les plantes To PCR positives ont été rempotées, et leurs graines ont été récoltées (graines T1) et semées sur un milieu de croissance additionné de kanamycine (100 mg/L) afin de produire les plantes T1. Une analyse PCR et des tests de ségrégation et KHl2 ont été réalisés sur les plants de tomates afin de ne conserver que les plantes T1 présentant une ségrégation 3 :1 (KHl2 inférieur à 5, indiquant une mono insertion). 6 plantes T1 par événement ont été rempotées pour observation de l'expression des promoteurs étudiés (tests GUS).

### I/ Stress salin et test GUS sur les plantes transformées avec le construit pAgMat3-GUS-tNOS

### 1) Transformation avec le Construit pAgMat3-GUS-tNOS

Les analyses PCR ont été réalisées au moyen des amorces suivantes :
Amorce promoteur :
   5'-GGGGACAAGTTTGTACAAAAAAGCAGGCTGAACAGAAACAATTGTGGATG-3' (SEQ ID 36)
Amorce GUS :
   5'-CGATCCAGACTGAATGCCC-3' (SEQ ID 37)

Les événements de transformation pour lesquels le test PCR est positif et les plantes T1 présentent une ségrégation 3 :1 (KHl2 inférieur à 5) sont présentés dans le tableau 4.

**Tableau IV: événements de transformation avec le construit pAgMat3-GUS-tNOS PCR positif et présentant un test khi2 < 5**

| **Construit** | **Evénement de transformation** | **Confirmation PCR Promoteur/GUS** | **Khi2** | **Nombre de plantes acclimatées** |
|---|---|---|---|---|
| pAgMat3-GUS-tNOS | 1T-LE-KE-010-5b | + | **0** | **10** |
| pAgMat3-GUS-tNOS | 1T-LE-KE-010-8a | + | **0** | **6** |
| pAgMat3-GUS-tNOS | 1T-LE-KE-010-10b | + | **0** | **10** |
| pAgMat3-GUS-tNOS | 1T-LE-KE-010-11a | + | **0** | **5** |
| pAgMat3-GUS-tNOS | 2T-LE-KE-010-4a | + | **1** | **0** |
| pAgMat3-GUS-tNOS | 2T-LE-KE-010-10a | + | **1** | **7** |
| pAgMat3-GUS-tNOS | 2T-LE-KE-010-11a | + | **1** | **2** |
| pAgMat3-GUS-tNOS | 2T-LE-KE-010-27a | + | **1** | **4** |
| pAgMat3-GUS-tNOS | 2T-LE-KE-010-28a | + | **0** | **8** |
| pAgMat3-GUS-tNOS | 2T-LE-KE-010-29b | + | **0** | **10** |
| pAgMat3-GUS-tNOS | 2T-LE-KE-010-51a | + | **0** | **10** |

### 2) Stress salin et test GUS

Les plantes T1 obtenues sont arrosées avec de l'eau à 50 mM de NaCl 2 fois par jour, 10 ml à chaque arrosage, pendant 4 jours. Le cinquième jour, les prélèvements de tissus sont effectués et les tests GUS sont réalisés. Deux contrôles sont également réalisés ; un contrôle positif GUS sous contrôle d'un promoteur constitutif, un contrôle de plantes KEMER non transformées.

Onze événements de transformation indépendants (test Khi2 inférieur à 5, indiquant une mono insertion) et 2 témoins (non transformé et Gus positif) ont été analysés. Lorsque suffisamment de plantes sont disponibles, 6 plantes T1 par événement de transformation ont été rempotées et utilisées pour les tests Gus : 4 ayant subi un stress salin (S) et 2 arrosées à l'eau claire (NS). S'il y a moins de 6 plantes, toutes les plantes disponibles ont été utilisées.

Le test Gus a été effectué séparément sur fragments racinaires et foliaires. La lecture a eu lieu 3 jours après le test (voir plaques à la figure 14).

Les plantes transformées ont montré une coloration bleue au niveau des racines. L'expression préférentielle de GUS dans le tissu racinaire indique une activité du promoteur AgMat3 dans ce tissu. Les résultats de ce test sont présentés dans le tableau 5 ci-dessous

**Tableau V : test GUS sur les plantes transformées avec le construit pAgMat3-GUS-tNOS et soumises ou non à un stress salin.**

| **Evènement de transformation** | **Numéro de Plante** | Stress / no stress **(S/NS)** | **4j 50 mM** | **4j 50 mM** |
|---|---|---|---|---|
| | | | **Test GUS Racines** | **Test GUS Feuilles** |
| | | | | |
| **1T-LE-KE-010-5b** | 37 | S | 1 | 1 |
| | 38 | S | 1 | 1 |
| | 39 | S | 3 | 0 |
| | 40 | S | 1 | 0 |
| | 41 | NS | 3 | 1 |
| | 42 | NS | 2 | 1 |
| | | | | |
| **1T-LE-KE-010-8a** | 44 | S | 1 | 1 |
| | 45 | S | 3 | 0 |
| | 46 | S | 1 | 0 |
| | 47 | S | 3 | 0 |
| | 48 | NS | 3 | 0 |
| | | | | |
| **1T-LE-KE-010-10b** | 49 | S | 1 | 0 |
| | 50 | S | 1 | 0 |
| | 51 | S | 1 | 0 |
| | 52 | S | 1 | 0 |
| | 53 | NS | 3 | 0 |
| | 54 | NS | 3 | 0 |
| | | | | |
| **1T-LE-KE-010-11a** | 55 | S | 3 | 0 |
| | 56 | S | 3 | 0 |
| | 57 | S | 0 | 0 |
| | 58 | S | 1 | 0 |
| | 59 | NS | 0 | 1 |
| | | | | |
| **2T-LE-KE-010-4a** | 60 | S | 2 | 0 |
| | 61 | S | 1 | 0 |
| | 62 | S | 3 | 0 |
| | 63 | S | 0 | 0 |
| | 64 | NS | 0 | 0 |
| | 65 | NS | 0 | 0 |
| | | | | |
| **2T-LE-KE-010-10a** | 66 | S | 2 | 0 |
| | 67 | | 1 | 0 |
| | 68 | S | 3 | 0 |
| | 69 | S | 1 | 0 |
| | 70 | NS | 1 | 0 |
| | 71 | NS | 1 | 1 |
| | | | | |
| **2T-LE-KE-010-11a** | 72 | S | 3 | 0 |
| | 73 | NS | 3 | 0 |
| | | | | |
| **2T-LE-KE-010-27a** | 75 | S | 1 | 0 |
| | 76 | S | 1 | 0 |
| | 77 | S | 3 | 0 |
| | 78 | NS | 3 | 0 |
| | | | | |
| **2T-LE-KE-010-28a** | 79 | S | 3 | 1 |
| | 80 | S | 3 | 0 |
| | 81 | S | 3 | 0 |
| | 82 | S | 3 | 0 |
| | 83 | NS | 3 | 0 |
| | 84 | NS | 3 | 0 |
| | | | | |
| **2T-LE-KE-010-29b** | 85 | S | 3 | 0 |
| | 86 | S | 3 | 0 |
| | 87 | S | 3 | 0 |
| | 88 | S | 0 | 0 |
| | 89 | NS | 2 | 0 |
| | 90 | NS | 2 | 0 |
| | | | | |
| **2T-LE-KE-010-51a** | 91 | S | 2 | 0 |
| | 92 | S | 2 | 0 |
| | 93 | S | 3 | 0 |
| | 94 | S | 2 | 0 |
| | 95 | NS | 1 | 0 |
| | 96 | NS | 1 | 0 |
| KEMER NT | 187 | S | 0 | 0 |
| | 188 | S | 0 | 0 |
| | 189 | NS | 0 | 0 |
| | 190 | NS | 0 | 0 |
| | | | | |
| Témoin Gus + | 193 | NS | 3 | 3 |
| | 194 | NS | 3 | 3 |

L'ensemble de ces données indique une expression racine spécifique du promoteur AgMat3.

### II/ Test GUS sur les plantes transformées avec le construit pAgMT2-GUS-tNOS

### 1) Transformation avec le Construit pAgMT2-GUS-tNOS

L'analyse PCR a été réalisée au moyen des amorces suivantes :
Amorce promoteur :
   5'-GGGGACAAGTTTGTACAAAAAAGCAGGCTGACCCACTATCAACAATGATC-3' (SEQ ID 38)
Amorce GUS :
   5'-CGATCCAGACTGAATGCCC-3' (SEQ ID 37)

Les événements de transformation pour lesquels le test PCR est positif et les plantes T1 présentent une ségrégation 3 :1 (KHl2 inférieur à 5) sont présentés dans le tableau 6.

**Tableau VI : événements de transformation avec le construit pAgMT2-GUS-tNOS PCR positif et présentant un test khi2 < 5**

| **Construit** | **Evénement de transformation** | **Confirmation PCR Promoteur/GUS** | **Khi2** | **Nombre de plantes acclimatées** |
|---|---|---|---|---|
| pAgMT2-GUS-tNOS | 1T-LE-KE-008-11a | + | **1** | **6** |
| pAgMT2-GUS-tNOS | 1T-LE-KE-008-16a | + | **2** | **10** |
| pAgMT2-GUS-tNOS | 1T-LE-KE-008-20a | + | **1** | **10** |
| pAgMT2-GUS-tNOS | 3T-LE-KE-008-23a | + | **0** | **9** |
| pAgMT2-GUS-tNOS | 3T-LE-KE-008-33b | + | **1** | **4** |
| pAgMT2-GUS-tNOS | 3T-LE-KE-008-40a | + | **0** | **6** |
| pAgMT2-GUS-tNOS | 3T-LE-KE-008-59a | + | **5** | **4** |
| pAgMT2-GUS-tNOS | 3T-LE-KE-008-64a | + | **0** | **6** |
| pAgMT2-GUS-tNOS | 3T-LE-KE-008-96a | + | **0** | **10** |
| pAgMT2-GUS-tNOS | 3T-LE-KE-008-103a | + | **4** | **10** |

### 2) Test GUS

Onze événements de transformation indépendants (test Khi2 inférieur à 5, indiquant une mono insertion) et 2 témoins (non transformé et Gus positif) ont été analysés. 6 plantes T1 par événement de transformation ont été rempotées et utilisées pour les tests Gus.

Le test Gus a été effectué sur des fragments de tiges et feuilles (exemple de plaque : figure 15).

Les résultats de ce test sont présentés dans le tableau 7 ci-dessous.

**Tableau VII : test GUS sur les plantes transformées avec le construit pAgMat3-GUS-tNOS et soumises ou non à un stress salin**

| **Evénement de transformation (T0)** | **N° plante (T1)** | **Tests Gus sur tiges** |
|---|---|---|
| | | |
| **1T-LE-KE-008-5a** | 1 | 0 |
| | 2 | 0 |
| | 3 | 0 |
| | 4 | 0 |
| | 5 | 0 |
| | 6 | 0 |
| | | |
| **1T-LE-KE-008-11a** | 7 | 1 |
| | 8 | 0 |
| | 9 | 0 |
| | 10 | 0 |
| | 11 | 0 |
| | 12 | 1 |
| | | |
| **1T-LE-KE-008-16a** | 13 | 1 |
| | 14 | 1 |
| | 15 | 0 |
| | 16 | 0 |
| | 17 | 1 |
| | 18 | 1 |
| | | |
| **1T-LE-KE-008-20a** | 19 | 0 |
| | 20 | 0 |
| | 21 | 0 |
| | 22 | 0 |
| | 23 | 0 |
| | 24 | 0 |
| | | |
| **3T-LE-KE-008-23a** | 97 | 2 |
| | 98 | 1 |
| | 99 | 1 |
| | 100 | 1 |
| | 101 | 2 |
| | 102 | 1 |
| | | |
| **3T-LE-KE-008-33b** | 103 | 2 |
| | 104 | 2 |
| | 105 | 2 |
| | 106 | 3 |
| | | |
| **3T-LE-KE-008-40a** | 107 | 1 |
| | 108 | 0 |
| | 109 | 0 |
| | 110 | 0 |
| | 111 | 0 |
| | 112 | 0 |
| | | |
| **3T-LE-KE-008-59a** | 113 | 1 |
| | 114 | 0 |
| | 115 | 0 |
| | 116 | 0 |
| | | |
| **3T-LE-KE-008-64a** | 117 | 2 |
| | 118 | 1 |
| | 119 | 0 |
| | 120 | 1 |
| | 121 | 1 |
| | 122 | 0 |
| | | |
| **3T-LE-KE-008-96a** | 123 | 1 |
| | 124 | 0 |
| | 125 | 0 |
| | 126 | 0 |
| | 127 | 0 |
| | 128 | 0 |
| | | |
| **3T-LE-KE-008-103a** | 129 | 0 |
| | 130 | 0 |
| | 131 | 0 |
| | 132 | 0 |
| | 133 | 0 |
| | 134 | 0 |
| **KEMER NT** | 187 | 0 |
| | | |
| **Témoin GUS+** | 193 | 3 |

Des fragments Gus positifs ont ensuite été utilisés pour une analyse histo-cytologique et une observation de la coloration au niveau des tissus vasculaires. La lecture a eu lieu 4 jours après le test. Les premières observations montrent une coloration bleue plutôt concentrée sur les tissus vasculaires de la tige, dans certains cas, uniquement sur les nervures des feuilles (figure 16).

### 3) Coupes tissulaires

La figure 17 présente les coupes tissulaires des plantes 13 (a) et 103, au niveau de la feuille (b) et de la tige (c). Ces données cytologiques indiquent une expression au niveau des tissus phloémiens. L'ensemble de ces données indique une expression phloème spécifique du promoteur AgMT2.

Les séquences SEQ ID identifiées dans la présente demande sont les suivantes :
SEQ ID N°1 : séquence promotrice issue du gène AgMaT3 *d'Apium graveolens ;*
SEQ ID N°2: séquence promotrice issue du gène AgMT2 *d'Apium graveolens ;*
SEQ ID N°3: séquence promotrice issue du gène AgMT3 *d'Apium graveolens ;*
SEQ ID N°4: séquence 5' complète issue du gène AgMaT3 *d'Apium graveolens ;*
SEQ ID N°5 : séquence 5' complète issue du gène AgMT2 *d'Apium graveolens ;*
SEQ ID N°6: séquence 5' complète issue du gène AgMT3 *d'Apium graveolens ;*
SEQ ID N°7: séquence complète du gène AgMaT3 ;
SEQ ID N°8 : protéine putative AgMaT3 déduite de la séquence d'ADN correspondante ;
SEQ ID N°9: séquence complète du gène AgMT2 ;
SEQ ID N°10 : protéine putative AgMT2 déduite de la séquence d'ADN correspondante ;
SEQ ID N°11: séquence complète du gène AgMT3 ;
SEQ ID N°12 : protéine putative AgMT3 déduite de la séquence d'ADN correspondante ;
SEQ ID N°13 : séquence du gène AgMaT3, sans la partie promotrice ;
SEQ ID N°14 : ADNc codant pour la protéine AgMaT3 (SEQ ID N°8).

### Bibliographie

Abe H., et al., 2003. Plant Cell 15 : 63-78.
Abe H., et al., 1997. Plant Cell 9 : 1859-1868.
Bearden J.C., 1978. Biochem. Biophys. Acta 533 : 525-529.
Bohnert H.J., et al., 2001. Plant Physiol. Biochem. 39 : 295-311.
Brasileiro A.C.M., et al., 1992. Transgenic Res 1:133-141
Busk P.K. et Pages M., 1998. Plant Mol. Biol. 37 : 425-435.
Chatthai M., et al., 1997. Plant Mol. Biol. 34 : 243-254.
Chevalier C., et al., 1995. Plant Mol. Biol. 28 : 473-485.
Chinnusamy V., et al., 2003. Genes Dev. 17 : 1043-1054.
Choi H., et al., 2000. Biol. Chem. 275 : 1723-1730.
Choi D., et al., 1996. Plant Physiol. 112: 353-359.
Clough SF and Bent AF, 1998, Plant J., 16 : 735-743
De Pascale S., et al., 2003. J. Amer. Soc. Hort. Sci. 128 : 136-143.
Dinant S., Clark A.M., Zhu Y., Vilaine F., Palauqui J.-C., Kusiak C. et Thompson G.A., 2003. Plant Physiol. 131 : 114-128.
Divol F., 2004. Thèse de Biologie, spécialité Sciences du Végétal, Université de Paris XI. Dohmen R.J., et al., 1991. Yeast 7 : 691-692.
Eulgem T., et al., 2000. Trends Plant Sci. 5 : 199-206.
Filatti J.J., et al., 1987. BiolTechnology 5: 726-730.
Fromard L., et al., 1995. Plant Physiol. 108 : 913-918
Hasegawa P.M., et al., 2000 a. Trends Plant Sci. 5 : 317-319.
Hasegawa P.M., et al., 2000 b. Annu. Rev. Plant Physiol. Plant Mol. Biol. 51 : 463-499.
Higo K., et al., 1999. Nucleic Acids Res. 27 : 297-300.
Hsieh H.-M., et al., 1995. Plant Mol. Biol. 28 : 381-389.
Jefferson R.A., et al., 1987. EMBO J. 6 : 3901-3907.
Kang J.Y., et al., 2002. Plant Cell 14 : 343-357.
Karakas B., et al., 1997. Plant Cell Environ. 20 : 609-616.
Kay R., et al., 1987. Science 236 : 1299-1302.
Koncz C and Schell J. 1986 Mol. Gen. Genet. 204
Koyama M.L., et al., 2001. Plant Physiol. 125 : 406-422.
Kreps J.A., et al., 2002. Plant Physiol. 130 : 2129-2141.
Lohaus G., et al., 2000. J. Exp. Bot. 51 : 1721-1732.
Lu C.A., et al., 2002. Plant Cell 14 : 1963-1980.
Maingourd C., 2001. DEA Interactions Cellulaires et Transports Membranaires, Université de Poitiers.
Masmoudi K., et al., 2001. Plant Physiol. Biochem. 39 : 971-979.
Morita A., et al., 1998. FEBS Lett. 423 : 81-85.
Nelson D.E., et al., 1999. Plant Physiol. 119 : 165-172.
Noiraud N., 1999. Thèse de Physiologie et Biochimie végétales, Université de Poitiers.
Noiraud N., Delrot S. et Lemoine R., 2000. Plant Physiol. 122 : 1447-1455.
Noiraud N., Maurousset L. et Lemoine R., 2001 a. Plant Cell 13 : 695-705.
Noiraud N., Maurousset L. et Lemoine R., 2001 b. Plant Physiol. Biochem. 39 : 717-728.
Popova O.V., et al., 2003. Plant Mol. Biol. 52 : 569-578.
Reid S.J. et Ross G.S., 1997. Physiol. Plant. 100 : 183-189.
Rieping M. et Schoffl F., 1992. Mol. Gen. Genet. 231 : 226-232.
Ruiz-Medrano R., Xoconostle-Càzares B. et Lucas W.J., 2001. Curr. Opin. Plant Biol. 4 : 202-209.
Salekdeh G.H., et al., 2002. Field Crops Res. 76 : 199-219.
Sambrook J., et al., 1989. Molecular cloning: A laboratory manual. Cold Spring Harbor laboratory (Eds), Cold Spring Harbor, New York.
Sauer N. et Stadler R., 1993. Plant J. 4 : 601-610.
Shen B., et al., 1997 a. Plant Physiol. 113 : 1177-1183.
Shen B., et al., 1997 b. Plant Physiol. 115 : 527-532.
Sugimoto K., et al., 2003. Plant J. 36 : 550-564.
Tarczynski M.C., et al., 1993. Science 259 : 508-510.
Teakle G.R., et al., 2002. Arabidopsis thaliana GATA factors: organisation, expression and DNA-binding characteristics. Plant Mol. Biol. 50 : 43-57.
Terzaghi W.B. et Cashmore A.R., 1995. Annu. Rev. Plant Physiol. Plant Mol. Biol. 46 : 445-474.
Urao T., et al.,1993. Plant Cell 5 : 1529-1539.
Vilaine F., Palauqui J.-C., Amselem J., Kusiak C., Lemoine R. et Dinant S., 2003. Plant J. 36 : 67-81.
Wagner H.J., et al.,2001. Transplantation 72 : 1012-1019.
Yu D., Chen C. et Chen Z., 2001. Plant Cell 13 : 1527-1540.

## Revendications

1. Séquence d'acides nucléiques comprenant tout ou partie de la séquence SEQ ID N° 2 ou de la séquence SEQ ID N°3, telle que :
- ladite séquence d'acides nucléiques présente une activité de promoteur transcriptionnel, et
- ladite partie comprend au moins 30 nucléotides consécutifs de SEQ ID N° 2 ou de SEQ ID N°3.

2. Séquence selon la revendication 1, présentant une activité de promoteur transcriptionnel dans les cellules végétales.

3. Séquence selon la revendication 2, où ladite séquence présente une activité de promoteur transcriptionnel préférentiellement dans les tissus vasculaires, notamment dans le phloème et/ou le xylème.

4. Séquence selon l'une des revendications 1 à 3 comprenant ou consistant en une séquence choisie parmi les séquences SEQ ID N°2, 3, 5 et 6.

5. Séquence d'acides nucléiques présentant par rapport à une séquence selon la revendication 1, moins de 5 mutations ponctuelles.

6. Séquence d'acides nucléiques présentant une activité de promoteur transcriptionnel et ayant au moins 70% d'identité avec la séquence SEQ ID N° 2 ou la séquence SEQ ID N°3, de préférence au moins 80% d'identité, de préférence 90% d'identité.

7. Séquence d'acides nucléiques complémentaire d'une séquence selon l'une des revendications 1 à 6.

8. Sonde d'acides nucléiques capable de s'hybrider avec SEQ ID N°2 ou SEQ ID N°3, dans des conditions de forte stringence, ladite sonde ayant au moins 25 nucléotides.

9. Construit d'ADN consistant en ou comprenant une séquence promotrice selon l'une quelconque des revendications 1 à 6, et une séquence d'intérêt à transcrire en aval, tel que la transcription de ladite séquence à transcrire soit sous le contrôle de la séquence promotrice.

10. Construit selon la revendication 9, où ladite séquence promotrice est hétérologue par rapport à la séquence d'intérêt à transcrire.

11. Cellule de plante transformée par un construit selon l'une des revendications 9 à 10.

12. Plante transgénique comprenant dans son génome une séquence selon la revendication 1 exogène par rapport à la plante.

13. Plante transgénique comprenant des cellules selon la revendication 11, ou partie d'une telle plante, notamment semences, matériel de reproduction, graines, racines, fleurs ou fruits, lesdites parties étant transgéniques.

14. Procédé de préparation d'une plante transgénique comprenant :
a. L'obtention d'un construit selon l'une des revendications 9 à 10 ;
b. L'introduction du construit dans une cellule provenant d'une plante d'intérêt,
c. La régénération d'une plante transgénique,
d. Optionnellement, la prolifération de la plante pour obtenir une descendance.

15. Utilisation d'une séquence selon l'une quelconque des revendications 1 à 6, ou bien d'un construit selon l'une des revendications 9 à 10, pour la réalisation de plantes transgéniques.

16. Plante transgénique comprenant dans son génome une séquence d'acides nucléiques comprenant tout ou partie de SEQ ID N° 2 ou SEQ ID N°3, telle que
- ladite séquence présente une activité de promoteur transcriptionnel,
- ladite partie comprend au moins 30 nucléotides consécutifs de SEQ ID N°2 ou SEQ ID N°3, et
- ladite séquence est en association fonctionnelle avec une séquence codante hétérologue et exprimant ladite séquence codante de façon spécifique dans le phloème.


SEQUENCE LISTING

<110> Vilmorin & Cie
      Centre National de la Recherche Scientifique
      Université de Poitiers

<120> Promoteurs de gènes pouvant être utilisés chez les plantes

<130> B6542ADA

<150> FR 06/00137
<151> 2006-01-06

<160> 38

<170> PatentIn version 3.1

<210> 1
<211> 548
<212> DNA
<213> Apium graveolens

<400> 1
aagaaatttt gaacagaaac aattgtggat gcacctctct atatatttgt acttttgcat 60

actgtcgtat atatagtaaa gaattactta ataggggtga atgtactcac cgggcaaagt 120

attagtaatt tcttcattaa aatatttaag agtcaattta atttgccact gccttttctt 180

tttcagttct tacttcaagg ttagtcaatc tattccggtg aataattacg aacgaatata 240

taatctctga gtagcaaaga ttcccaaaac tatccaaaat tatccggcgg ctgcggattt 300

gatacgataa aaaaatatcg ctataatcac aaatttattc aaatttacta aaaagggccc 360

acagcctaat taccaaaatc aaattgaact ttcccaattc cacgcaatcc tcaaagataa 420

acacagcaac caccaactaa tactctataa aaaatcatac aaaagtccaa ctaattacac 480

ccctcacgtt acgatagtca aattgtttag tcctttcccc caagcttcga ccattgtttc 540

tcaacatt 548

<210> 2
<211> 1319
<212> DNA
<213> Apium graveolens

<400> 2
gacccactat caacaatgat caagaatgaa caatacatat acatggtgta tagttaagat 60

ttcaggatat tttattggta tgattgttgc attggtatag atatagcatg ttgagaggaa 120

agtgtttgaa acagagattg caatataagt agggtaggct atagcagact actcttaaac 180

cgagcaccaa aagcttcttg attcatctct agataatatc agaatgatga ttgcagttca 240

aactatgtgc tgatactcgt aaaactcctc ttcagcagtg gctggttcga tttgttattg 300

gtaaatgaat ttctatactc gatcgcccgg cagaatcggc catctaacat tatatcaaaa 360

ttttatactt tttatcagca tcctaatgag ggtgttaata atctttaata ttaattgttt 420

gatagagatg atcgtctaat aaacgtaaat atcgtttaag atgatagttc accatcttct 480

ttagaaaggt aaactgattc agataagcat gcttcactta ttggagatgg tgacaacggc 540

attctgcttt tgattgatcg acgccctcgg ttccttatgc aaagcaactt tctcttttgt 600

tagtggacgt ctattagttt caatccattg tcattttctg ctgtaggaat cctttttatt 660

attggaccga aaaattgctc ttcaggctct acctgagaca tatcagatcg taaaccagat 720

caaacatatc caatgtaatc ggcttgtaga tcaggatttt atgtggggtg agatgtacgc 780

atattatata gccaggattt taggtggcat aagatgtacg catatcatat aacacttcta 840

cacttattga gtagaggcaa aacaattttc ttttagtgta tagaaaacta cagagtcgtg 900

cataatgttg aaatcgcatg tttgttggta cagattgaag gtccatgagc ttaagttaaa 960

taaaaaatct acgataaaaa tcaccatatg atattcaact ttttacacga gtcagaattc 1020

ataaaatgtt attacctgct caaaattgct aagttatgga atattattgc atggatatta 1080

tactactacc gcaaaaacta tctttaagat aacaatatga agtacaaata atggagttct 1140

tcaggccaca gacaaagctg taccagagga tatataactt ttcgtttgac tggtccaggt 1200

ggacggtcga tagttagcct cctttctcct ttttgcacta taaataaagc tcatgacttc 1260

acaaaacaaa gtcaccagat aagtgagagt gattaataca gagtccacaa cgatcttat 1319

<210> 3
<211> 649
<212> DNA
<213> Apium graveolens

<400> 3
ttctttattc tgcagctaga gcctacaaga aagtgttcca aaatatcaaa agtgactaat 60

cgattagtta taacttaaca tgttttaact ggtgttttaa ccgatgaatg catatattaa 120

tgaatgcaag atcatctatt tacgaataaa aaatcaataa ttaattgaca tttatcgttt 180

tagaaagaaa gtgttcgaaa atatcaaaag atccaaatcg acgaattgta acttaatatg 240

ttttaactag cgaatacata ttaatgaatg caagatcatc tatttacgaa taaaaaatca 300

ataattaatt aacacatttt tatttgaaaa ccgttttaga aacaaatttg ggagtttccg 360

attctctttt aagattaata tatttgaaaa gttaaaaaca caattaaatt cagaaaatgg 420

gaaagtatca agttgatgaa tatgagatac ttaaaaagga tggacgagag aaggatagca 480

taggccccaa gctccattat caagattcct caagtaacct ttattcattg aagcgtgtgc 540

tcttctcgtg ccactccatc tataaatacc agcccaaatc acacttctgg aaaatatagc 600

aaactacaaa gctctacaat acactcttgc ataccacctt acttcaagc 649

<210> 4
<211> 589
<212> DNA
<213> Apium graveolens

<400> 4
aaaatgtttc atattcacaa aagaaatttt gaacagaaac aattgtggat gcacctctct 60

atatatttgt acttttgcat actgtcgtat atatagtaaa gaattactta ataggggtga 120

atgtactcac cgggcaaagt attagtaatt tcttcattaa aatatttaag agtcaattta 180

atttgccact gccttttctt tttcagttct tacttcaagg ttagtcaatc tattccggtg 240

aataattacg aacgaatata taatctctga gtagcaaaga ttcccaaaac tatccaaaat 300

tatccggcgg ctgcggattt gatacgataa aaaaatatcg ctataatcac aaatttattc 360

aaatttacta aaaagggccc acagcctaat taccaaaatc aaattgaact ttcccaattc 420

cacgcaatcc tcaaagataa acacagcaac caccaactaa tactctataa aaaatcatac 480

aaaagtccaa ctaattacac ccctcacgtt acgatagtca aattgtttag tcctttcccc 540

caagcttcga ccattgtttc tcaacatttt gtaatctctc tctctctcg 589

<210> 5
<211> 1573
<212> DNA
<213> Apium graveolens

<400> 5
aacgttgcca aataacttta tgagattgtt tgatgggtag acattgaaga taaaataaag 60

ttgtttcttc ttttgccggg agatattctc aaatctcaaa tattctggac cgaagaagtt 120

tatttgcaat tgcctagttc acaaaataaa ataaacagag ttcacttttg tagagcaatt 180

attcgaaatc caccaccatt gacggatgca gctcatcttg gacccactat caacaatgat 240

caagaatgaa caatacatat acatggtgta tagttaagat ttcaggatat tttattggta 300

tgattgttgc attggtatag atatagcatg ttgagaggaa agtgtttgaa acagagattg 360

caatataagt agggtaggct atagcagact actcttaaac cgagcaccaa aagcttcttg 420

attcatctct agataatatc agaatgatga ttgcagttca aactatgtgc tgatactcgt 480

aaaactcctc ttcagcagtg gctggttcga tttgttattg gtaaatgaat ttctatactc 540

gatcgcccgg cagaatcggc catctaacat tatatcaaaa ttttatactt tttatcagca 600

tcctaatgag ggtgttaata atctttaata ttaattgttt gatagagatg atcgtctaat 660

aaacgtaaat atcgtttaag atgatagttc accatcttct ttagaaaggt aaactgattc 720

agataagcat gcttcactta ttggagatgg tgacaacggc attctgcttt tgattgatcg 780

acgccctcgg ttccttatgc aaagcaactt tctcttttgt tagtggacgt ctattagttt 840

caatccattg tcattttctg ctgtaggaat cctttttatt attggaccga aaaattgctc 900

ttcaggctct acctgagaca tatcagatcg taaaccagat caaacatatc caatgtaatc 960

ggcttgtaga tcaggatttt atgtggggtg agatgtacgc atattatata gccaggattt 1020

taggtggcat aagatgtacg catatcatat aacacttcta cacttattga gtagaggcaa 1080

aacaattttc ttttagtgta tagaaaacta cagagtcgtg cataatgttg aaatcgcatg 1140

tttgttggta cagattgaag gtccatgagc ttaagttaaa taaaaaatct acgataaaaa 1200

tcaccatatg atattcaact ttttacacga gtcagaattc ataaaatgtt attacctgct 1260

caaaattgct aagttatgga atattattgc atggatatta tactactacc gcaaaaacta 1320

tctttaagat aacaatatga agtacaaata atggagttct tcaggccaca gacaaagctg 1380

taccagagga tatataactt ttcgtttgac tggtccaggt ggacggtcga tagttagcct 1440

cctttctcct ttttgcacta taaataaagc tcatgacttc acaaaacaaa gtcaccagat 1500

aagtgagagt gattaataca gagtccacaa cgatcttata atcttaatca ttctctttac 1560

tatccattcg aaa 1573

<210> 6
<211> 690
<212> DNA
<213> Apium graveolens

<400> 6
aaagcaacga tttcttttta ccaaattttg ttctttattc tgcagctaga gcctacaaga 60

aagtgttcca aaatatcaaa agtgactaat cgattagtta taacttaaca tgttttaact 120

ggtgttttaa ccgatgaatg catatattaa tgaatgcaag atcatctatt tacgaataaa 180

aaatcaataa ttaattgaca tttatcgttt tagaaagaaa gtgttcgaaa atatcaaaag 240

atccaaatcg acgaattgta acttaatatg ttttaactag cgaatacata ttaatgaatg 300

caagatcatc tatttacgaa taaaaaatca ataattaatt aacacatttt tatttgaaaa 360

ccgttttaga aacaaatttg ggagtttccg attctctttt aagattaata tatttgaaaa 420

gttaaaaaca caattaaatt cagaaaatgg gaaagtatca agttgatgaa tatgagatac 480

ttaaaaagga tggacgagag aaggatagca taggccccaa gctccattat caagattcct 540

caagtaacct ttattcattg aagcgtgtgc tcttctcgtg ccactccatc tataaatacc 600

agcccaaatc acacttctgg aaaatatagc aaactacaaa gctctacaat acactcttgc 660

ataccacctt acttcaagct cttaacaacc 690

<210> 7
<211> 3697
<212> DNA
<213> Apium graveolens

<220>
<221> CDS
<222> (590)..(742)
<223>

<220>
<221> CDS
<222> (2175)..(3230)
<223>

<220>
<221> CDS
<222> (1007)..(1369)
<223>

<400> 7
   aaaatgtttc atattcacaa aagaaatttt gaacagaaac aattgtggat gcacctctct 60
   atatatttgt acttttgcat actgtcgtat atatagtaaa gaattactta ataggggtga 120
   atgtactcac cgggcaaagt attagtaatt tcttcattaa aatatttaag agtcaattta 180
   atttgccact gccttttctt tttcagttct tacttcaagg ttagtcaatc tattccggtg 240
   aataattacg aacgaatata taatctctga gtagcaaaga ttcccaaaac tatccaaaat 300
   tatccggcgg ctgcggattt gatacgataa aaaaatatcg ctataatcac aaatttattc 360
   aaatttacta aaaagggccc acagcctaat taccaaaatc aaattgaact ttcccaattc 420
   cacgcaatcc tcaaagataa acacagcaac caccaactaa tactctataa aaaatcatac 480
   aaaagtccaa ctaattacac ccctcacgtt acgatagtca aattgtttag tcctttcccc 540
   caagcttcga ccattgtttc tcaacatttt gtaatctctc tctctctcg atg gcc gga 598
   Met Ala Gly
   1
   act tct ggc cct tcc ggc ggt gtc gtc gcc gat ccc aag aat ctg agt 646
   Thr Ser Gly Pro Ser Gly Gly Val Val Ala Asp Pro Lys Asn Leu Ser
   5 10 15
   tca cct tta ttt gac gcg gag aaa aaa ccc aaa aat aac aag tat gct 694
   Ser Pro Leu Phe Asp Ala Glu Lys Lys Pro Lys Asn Asn Lys Tyr Ala
   20 25 30 35
   ttt gct tgt tcc att tta gct tcc atg act tca att cta ctt ggt tat 742
   Phe Ala Cys Ser Ile Leu Ala Ser Met Thr Ser Ile Leu Leu Gly Tyr
   40 45 50
   ggtacgatgt ctttcaaacc tacacttatt ttcagtacac tcacaatgtc acaaattctt 802
   cgtaatcgat ctctcttcca cgatcagtat ttttatttta attcaattta ttaaagcttt 862
   cggacagatc tctttagttt taccgacaca tattacatta gaataatctg aacaacactc 922
   tgcatgtctt tttgtcttaa ctatcttatg atctaaacac tctacttgtg ttgtgttata 982
   accttttcaa tttttatttt ttta gat acg ggg gtt atg agt gga gca gca 1033
   Asp Thr Gly Val Met Ser Gly Ala Ala
   55 60
   atc tac ata aaa aaa gat ctc cgt ttc acc gat gta caa atc gaa atc 1081
   Ile Tyr Ile Lys Lys Asp Leu Arg Phe Thr Asp Val Gln Ile Glu Ile
   65 70 75
   atc gtc gga atc atc aac atc ttc tct ctt ctc ggc tct ttt ctc gcc 1129
   Ile Val Gly Ile Ile Asn Ile Phe Ser Leu Leu Gly Ser Phe Leu Ala
   80 85 90
   gga aga acc tcc gat tgg att ggc cgg aga tac aca atg gtt cta gcc 1177
   Gly Arg Thr Ser Asp Trp Ile Gly Arg Arg Tyr Thr Met Val Leu Ala
   95 100 105
   ggt ggc ata ttt ttt gcc gga gct ttt tta atg gga tgt gct aca aac 1225
   Gly Gly Ile Phe Phe Ala Gly Ala Phe Leu Met Gly Cys Ala Thr Asn
   110 115 120
   ttt gag ttt tta atg gtg ggt cgg ttt gtc gcc ggg atc gga gta ggg 1273
   Phe Glu Phe Leu Met Val Gly Arg Phe Val Ala Gly Ile Gly Val Gly
   125 130 135 140
   tat gct atg atg atc gct ccg gtt tat aca act gag gtt gct ccg gcg 1321
   Tyr Ala Met Met Ile Ala Pro Val Tyr Thr Thr Glu Val Ala Pro Ala
   145 150 155
   tct tct cgg ggt ttt ctc act tct ttc ccg gag gtc ttt att aat gct 1369
   Ser Ser Arg Gly Phe Leu Thr Ser Phe Pro Glu Val Phe Ile Asn Ala
   160 165 170
   ggtgcgtttt tattcgctaa ttaatttata tttatttatt tatttgtata aattagatat 1429
   aatttttaat ttaaattgaa taaaacttta ctgtactaaa gatcagataa cgtatatctc 1489
   gtgcaaatgt tgtggaacac atcatgaaga taataatatt aagcatatat taaaataatg 1549
   ttttattaaa aatatgtgaa ctgttgtttg gtgttaactg ctttttttta tttggtgttc 1609
   gtgatgtttt aacaacactg accaatatgt aagtgtgtac aactttacca acaaaagata 1669
   ctgttattaa agtacagatt atgtgaatat tatttatata aaataaaaaa atatgtaggt 1729
   ccagtgaagc atttttcgtc gtttagatgt gtggtcctta ttgataggta gagttgtgta 1789
   tcttttgctt ttgtacacgt ttacaataag atatttggtt gtcaatttaa cagctgtata 1849
   gctttgatga cctgtgttat atattatgtg gtgtacatag gttgtgattg tgatgtttat 1909
   ctgataatta gtctgttttt atttgttata tttttgggtt tgaattggtt aacatgtagt 1969
   gatgagttgt tgaactcgat ttaattgtat atagttggac agttgtgatt actcgatttg 2029
   atcgaatata attggtattg gacagttgtg attggttttt tttggataat gttggcggct 2089
   ttttcttgat tatcgtttgg atgataatga aatgttaatt gttattgaca ttacatggta 2149
   gatgtaacta tgttgtttgt taaca gga gtt atg ctg ggg tat gta tcc aac 2201
   Gly Val Met Leu Gly Tyr Val Ser Asn
   175 180
   ttt gca ttt gca aag ctt ccg ctt tgg tta ggc tgg agg ttt atg ctt 2249
   Phe Ala Phe Ala Lys Leu Pro Leu Trp Leu Gly Trp Arg Phe Met Leu
   185 190 195
   gga att gga gca gtt cct tcg gtt ggc tta gcc att ggt gta ttg tat 2297
   Gly Ile Gly Ala Val Pro Ser Val Gly Leu Ala Ile Gly Val Leu Tyr
   200 205 210
   atg cct gag tct ccg cgt tgg ctt gtc atg agg ggt caa ctt ggc gaa 2345
   Met Pro Glu Ser Pro Arg Trp Leu Val Met Arg Gly Gln Leu Gly Glu
   215 220 225
   gca agg cgt gta ctg gaa aag act tcg gag agc aaa gaa gaa gct cga 2393
   Ala Arg Arg Val Leu Glu Lys Thr Ser Glu Ser Lys Glu Glu Ala Arg
   230 235 240 245
   caa aga cta gaa gat atc aag gag gct gct gga att cca gaa gaa tgt 2441
   Gln Arg Leu Glu Asp Ile Lys Glu Ala Ala Gly Ile Pro Glu Glu Cys
   250 255 260
   aat gat gac gtt gtt gaa gtt cct aaa cgt agc aaa gac gat gct gtg 2489
   Asn Asp Asp Val Val Glu Val Pro Lys Arg Ser Lys Asp Asp Ala Val
   265 270 275
   tgg aaa gaa ttg ttc ctt cat cct aca cca gct gtt cgc cat gct gct 2537
   Trp Lys Glu Leu Phe Leu His Pro Thr Pro Ala Val Arg His Ala Ala
   280 285 290
   atc act ggc att ggt att cat ttc ttc caa atg gct agt ggt gtt gat 2585
   Ile Thr Gly Ile Gly Ile His Phe Phe Gln Met Ala Ser Gly Val Asp
   295 300 305
   gct gtt gtt ttg tac agt cct cga att ttt gag aag gct ggg tta aag 2633
   Ala Val Val Leu Tyr Ser Pro Arg Ile Phe Glu Lys Ala Gly Leu Lys
   310 315 320 325
   agt gat aac cac aag cta ctc gcc acc att ggt gtt gga gtc tgc aaa 2681
   Ser Asp Asn His Lys Leu Leu Ala Thr Ile Gly Val Gly Val Cys Lys
   330 335 340
   act att ttt gtt ttg ata tca aca ttt ttg cta gac aaa gtc gga cgg 2729
   Thr Ile Phe Val Leu Ile Ser Thr Phe Leu Leu Asp Lys Val Gly Arg
   345 350 355
   cgc cca ctg atg ctt tcg agt atg ggg ggc atg gta att gct cta ctc 2777
   Arg Pro Leu Met Leu Ser Ser Met Gly Gly Met Val Ile Ala Leu Leu
   360                 365                 370
   gta ctc tca ggc tca ttg tct gta att aat cac tcg cat caa acc gtt      2825
   Val Leu Ser Gly Ser Leu Ser Val Ile Asn His Ser His Gln Thr Val
       375                 380                 385
   ccc tgg gct gtt gct ttg gca ata att tcg gtg tat ggc ttt gtg tcg      2873
   Pro Trp Ala Val Ala Leu Ala Ile Ile Ser Val Tyr Gly Phe Val Ser
   390                 395                 400                 405
   gtg ttt tca agt ggg atg ggg cca att gct tgg gtg tat agt tcg gag      2921
   Val Phe Ser Ser Gly Met Gly Pro Ile Ala Trp Val Tyr Ser Ser Glu
                   410                 415                 420
   gtg ttt cct ttg agg ctt aga gcc caa ggt tgc agt atc gga gtg gca      2969
   Val Phe Pro Leu Arg Leu Arg Ala Gln Gly Cys Ser Ile Gly Val Ala
               425                 430                 435
   gtc aat cgt ggt gtt agt ggc att atc gga atg aca ttt ata tca atg      3017
   Val Asn Arg Gly Val Ser Gly Ile Ile Gly Met Thr Phe Ile Ser Met
           440                 445                 450
   tac aag gcc ttg act att ggt ggt gca ttc ttt gta ttc gct gtg gtt      3065
   Tyr Lys Ala Leu Thr Ile Gly Gly Ala Phe Phe Val Phe Ala Val Val
       455                 460                 465
   gca gca att gga tgg gta ttc atg ttc aca atg ttt cct gaa act caa      3113
   Ala Ala Ile Gly Trp Val Phe Met Phe Thr Met Phe Pro Glu Thr Gln
   470                 475                 480                 485
   gga aga aat ctt gaa gaa att gag gta ttg ttt ggc agt tac ttt ggc      3161
   Gly Arg Asn Leu Glu Glu Ile Glu Val Leu Phe Gly Ser Tyr Phe Gly
                   490                 495                 500
   tgg agg aaa aca ttg aag gat ttg aag aag aaa gaa gcg gca gaa gca      3209
   Trp Arg Lys Thr Leu Lys Asp Leu Lys Lys Lys Glu Ala Ala Glu Ala
               505                 510                 515
   aag aat gtc tgc att gtt gct taaaattcaa atacagcggg gattatagct      3260
   Lys Asn Val Cys Ile Val Ala
           520
   ttgtgatgtt aaatgtgttt gagcgagggt gcaaaaccaa acatacccgg tatattcact      3320
   cctaagtaga atttctggag tacctgcgga tttgtttgtg ttaactaagg gcgattttat      3380
   caaaatcctt ggtacccttg gaactcctct aataaattta aaacagtatt gtggttttta      3440
   cttgattcgt gacattccta catttctgct tctcatctct agttttatgt acgcatataa      3500
   ttgtgcttag tactcctacg ttattgctca acctctgttt gtgaatcgaa tatggtttgc      3560
   tgacatcttc cgagaccaga aacggaaaga gtaaatgttt tttcgcatgt gcaattataa      3620
   catcaatgtc ttgcgtttaa ttggtatgat atatgttctc ttgtttgcag cttctttgct      3680
   cagttcatat gcacaat                                                     3697


<210>  8
<211>  524
<212>  PRT
<213>  Apium graveolens

<400>  8

Met Ala Gly Thr Ser Gly Pro Ser Gly Gly Val Val Ala Asp Pro Lys
1               5                   10                  15

Asn Leu Ser Ser Pro Leu Phe Asp Ala Glu Lys Lys Pro Lys Asn Asn
            20                  25                  30

Lys Tyr Ala Phe Ala Cys Ser Ile Leu Ala Ser Met Thr Ser Ile Leu
        35                  40                  45

Leu Gly Tyr Asp Thr Gly Val Met Ser Gly Ala Ala Ile Tyr Ile Lys
    50                  55                  60

Lys Asp Leu Arg Phe Thr Asp Val Gln Ile Glu Ile Ile Val Gly Ile
65                  70                  75                  80

Ile Asn Ile Phe Ser Leu Leu Gly Ser Phe Leu Ala Gly Arg Thr Ser
                85                  90                  95

Asp Trp Ile Gly Arg Arg Tyr Thr Met Val Leu Ala Gly Gly Ile Phe
            100                 105                 110

Phe Ala Gly Ala Phe Leu Met Gly Cys Ala Thr Asn Phe Glu Phe Leu
        115                 120                 125

Met Val Gly Arg Phe Val Ala Gly Ile Gly Val Gly Tyr Ala Met Met
    130                 135                 140

Ile Ala Pro Val Tyr Thr Thr Glu Val Ala Pro Ala Ser Ser Arg Gly
145                 150                 155                 160

Phe Leu Thr Ser Phe Pro Glu Val Phe Ile Asn Ala Gly Val Met Leu
                165                 170                 175

Gly Tyr Val Ser Asn Phe Ala Phe Ala Lys Leu Pro Leu Trp Leu Gly
            180                 185                 190

Trp Arg Phe Met Leu Gly Ile Gly Ala Val Pro Ser Val Gly Leu Ala
        195                 200                 205

Ile Gly Val Leu Tyr Met Pro Glu Ser Pro Arg Trp Leu Val Met Arg
    210                 215                 220

Gly Gln Leu Gly Glu Ala Arg Arg Val Leu Glu Lys Thr Ser Glu Ser
225                 230                 235                 240

Lys Glu Glu Ala Arg Gln Arg Leu Glu Asp Ile Lys Glu Ala Ala Gly
                245                 250                 255

Ile Pro Glu Glu Cys Asn Asp Asp Val Val Glu Val Pro Lys Arg Ser
            260                 265                 270

Lys Asp Asp Ala Val Trp Lys Glu Leu Phe Leu His Pro Thr Pro Ala
        275                 280                 285

Val Arg His Ala Ala Ile Thr Gly Ile Gly Ile His Phe Phe Gln Met
    290                 295                 300

Ala Ser Gly Val Asp Ala Val Val Leu Tyr Ser Pro Arg Ile Phe Glu
305                 310                 315                 320

Lys Ala Gly Leu Lys Ser Asp Asn His Lys Leu Leu Ala Thr Ile Gly
                325                 330                 335

Val Gly Val Cys Lys Thr Ile Phe Val Leu Ile Ser Thr Phe Leu Leu
            340                 345                 350

Asp Lys Val Gly Arg Arg Pro Leu Met Leu Ser Ser Met Gly Gly Met
        355                 360                 365

Val Ile Ala Leu Leu Val Leu Ser Gly Ser Leu Ser Val Ile Asn His
    370                 375                 380

Ser His Gln Thr Val Pro Trp Ala Val Ala Leu Ala Ile Ile Ser Val
385                 390                 395                 400

Tyr Gly Phe Val Ser Val Phe Ser Ser Gly Met Gly Pro Ile Ala Trp
                405                 410                 415

Val Tyr Ser Ser Glu Val Phe Pro Leu Arg Leu Arg Ala Gln Gly Cys
            420                 425                 430

Ser Ile Gly Val Ala Val Asn Arg Gly Val Ser Gly Ile Ile Gly Met
        435                 440                 445

Thr Phe Ile Ser Met Tyr Lys Ala Leu Thr Ile Gly Gly Ala Phe Phe
    450                 455                 460

Val Phe Ala Val Val Ala Ala Ile Gly Trp Val Phe Met Phe Thr Met
465                 470                 475                 480

Phe Pro Glu Thr Gln Gly Arg Asn Leu Glu Glu Ile Glu Val Leu Phe
                485                 490                 495

Gly Ser Tyr Phe Gly Trp Arg Lys Thr Leu Lys Asp Leu Lys Lys Lys
            500                 505                 510

Glu Ala Ala Glu Ala Lys Asn Val Cys Ile Val Ala
        515                 520

<210>  9
<211>  2042
<212>  DNA
<213>  Apium graveolens

<220>
<221>  CDS
<222>  (1574)..(1801)
<223>

<220>
<221>  misc_feature
<222>  (1920)..(1920)
<223>  undefined


<220>
<221>  misc_feature
<222>  (1979)..(1979)
<223>  undefined


<220>
<221>  misc_feature
<222>  (1994)..(1994)
<223>  undefined


<220>
<221>  misc_feature
<222>  (2017)..(2017)
<223>  undefined


<400>  9
aacgttgcca aataacttta tgagattgtt tgatgggtag acattgaaga taaaataaag      60

ttgtttcttc ttttgccggg agatattctc aaatctcaaa tattctggac cgaagaagtt     120

tatttgcaat tgcctagttc acaaaataaa ataaacagag ttcacttttg tagagcaatt     180

attcgaaatc caccaccatt gacggatgca gctcatcttg gacccactat caacaatgat     240

caagaatgaa caatacatat acatggtgta tagttaagat ttcaggatat tttattggta     300

tgattgttgc attggtatag atatagcatg ttgagaggaa agtgtttgaa acagagattg     360

caatataagt agggtaggct atagcagact actcttaaac cgagcaccaa aagcttcttg     420

attcatctct agataatatc agaatgatga ttgcagttca aactatgtgc tgatactcgt     480

aaaactcctc ttcagcagtg gctggttcga tttgttattg gtaaatgaat ttctatactc     540

gatcgcccgg cagaatcggc catctaacat tatatcaaaa ttttatactt tttatcagca     600

tcctaatgag ggtgttaata atctttaata ttaattgttt gatagagatg atcgtctaat     660

aaacgtaaat atcgtttaag atgatagttc accatcttct ttagaaaggt aaactgattc     720

agataagcat gcttcactta ttggagatgg tgacaacggc attctgcttt tgattgatcg     780

acgccctcgg ttccttatgc aaagcaactt tctcttttgt tagtggacgt ctattagttt     840

caatccattg tcattttctg ctgtaggaat cctttttatt attggaccga aaaattgctc     900

ttcaggctct acctgagaca tatcagatcg taaaccagat caaacatatc caatgtaatc     960

ggcttgtaga tcaggatttt atgtggggtg agatgtacgc atattatata gccaggattt    1020

taggtggcat aagatgtacg catatcatat aacacttcta cacttattga gtagaggcaa    1080

aacaattttc ttttagtgta tagaaaacta cagagtcgtg cataatgttg aaatcgcatg    1140

tttgttggta cagattgaag gtccatgagc ttaagttaaa taaaaaatct acgataaaaa    1200

tcaccatatg atattcaact ttttacacga gtcagaattc ataaaatgtt attacctgct    1260

caaaattgct aagttatgga atattattgc atggatatta tactactacc gcaaaaacta    1320

tctttaagat aacaatatga agtacaaata atggagttct tcaggccaca gacaaagctg 1380

taccagagga tatataactt ttcgtttgac tggtccaggt ggacggtcga tagttagcct 1440

cctttctcct ttttgcacta taaataaagc tcatgacttc acaaaacaaa gtcaccagat 1500

aagtgagagt gattaataca gagtccacaa cgatcttata atcttaatca ttctctttac 1560

tatccattcg aaa atg tct tgc tgt gga gga aac tgt ggt tgt ggt gct 1609
Met Ser Cys Cys Gly Gly Asn Cys Gly Cys Gly Ala
1               5                   10

ggc tgc aag tgc ggc aat ggc tgt gga gga tgc gga atg tac cct gat 1657
Gly Cys Lys Cys Gly Asn Gly Cys Gly Gly Cys Gly Met Tyr Pro Asp
        15                  20                  25

gtg gag aag aac aca act gca acc atc att gat gga gtt gca cca aca 1705
Val Glu Lys Asn Thr Thr Ala Thr Ile Ile Asp Gly Val Ala Pro Thr
    30                  35                  40

aag acg ttt tct cag ggt tca gag atg agc ttt aca act gaa gga ggg 1753
Lys Thr Phe Ser Gln Gly Ser Glu Met Ser Phe Thr Thr Glu Gly Gly
45                  50                  55                  60

cat gcc tgc aag tgc gga tca aac tgc aca tgt aat ccg tgc aaa tgt 1801
His Ala Cys Lys Cys Gly Ser Asn Cys Thr Cys Asn Pro Cys Lys Cys
                65                  70                  75

taacgatgaa atggaactga gtacctaaca aagcagctag cgtttctcca atattgtact 1861

ataataatgc cgcagcctgt gttttatgct attccagact tggatgtgtt taatgttgnt 1921

aggctgctta atcttttttt tttatgtttt tcgtaactac cgttgctctc tgctttgngg 1981

gtactggtat tgncttaagg gttaattcat ctgcgngatg aaactaatgg catgaaattc 2041

t 2042

<210> 10
<211> 76
<212> PRT
<213> Apium graveolens

<400> 10

Met Ser Cys Cys Gly Gly Asn Cys Gly Cys Gly Ala Gly Cys Lys Cys
1               5                   10                  15

Gly Asn Gly Cys Gly Gly Cys Gly Met Tyr Pro Asp Val Glu Lys Asn
            20                  25                  30

Thr Thr Ala Thr Ile Ile Asp Gly Val Ala Pro Thr Lys Thr Phe Ser
        35                  40                  45

Gln Gly Ser Glu Met Ser Phe Thr Thr Glu Gly Gly His Ala Cys Lys
    50                  55                  60

Cys Gly Ser Asn Cys Thr Cys Asn Pro Cys Lys Cys
65                  70                  75

<210> 11
<211> 1102

<212>  DNA
<213>  Apium graveolens

<220>
<221>  CDS
<222>  (691)..(891)
<223>

<220>
<221>  misc_feature
<222>  (802)..(802)
<223>  undefined


<400>  11
aaagcaacga tttcttttta ccaaattttg ttctttattc tgcagctaga gcctacaaga      60

aagtgttcca aaatatcaaa agtgactaat cgattagtta taacttaaca tgttttaact     120

ggtgttttaa ccgatgaatg catatattaa tgaatgcaag atcatctatt tacgaataaa     180

aaatcaataa ttaattgaca tttatcgttt tagaaagaaa gtgttcgaaa atatcaaaag     240

atccaaatcg acgaattgta acttaatatg ttttaactag cgaatacata ttaatgaatg     300

caagatcatc tatttacgaa taaaaaatca ataattaatt aacacatttt tatttgaaaa     360

ccgttttaga aacaaatttg ggagtttccg attctctttt aagattaata tatttgaaaa     420

gttaaaaaca caattaaatt cagaaaatgg gaaagtatca agttgatgaa tatgagatac     480

ttaaaaagga tggacgagag aaggatagca taggccccaa gctccattat caagattcct     540

caagtaacct ttattcattg aagcgtgtgc tcttctcgtg ccactccatc tataaatacc     600

agcccaaatc acacttctgg aaaatatagc aaactacaaa gctctacaat acactcttgc     660

ataccacctt acttcaagct cttaacaacc atg tcg aac acc tgt ggc aac tgt      714
                                 Met Ser Asn Thr Cys Gly Asn Cys
                                 1               5

gac tgc tcc gac aag agc cag tgc gtg aag aag gga gcc agc tat ggc      762
Asp Cys Ser Asp Lys Ser Gln Cys Val Lys Lys Gly Ala Ser Tyr Gly
    10                  15                  20

ctt gac att gtt gaa act gga aag agc tat gtc cag acc nct gtg atg      810
Leu Asp Ile Val Glu Thr Gly Lys Ser Tyr Val Gln Thr Xaa Val Met
25                  30                  35                  40

gaa gtc tcg gca act gag aac gac ggc aag tgc aaa tgc gga aca agc      858
Glu Val Ser Ala Thr Glu Asn Asp Gly Lys Cys Lys Cys Gly Thr Ser
                45                  50                  55

tgc act tgt gtg aac tgc agt tgc ggt ggt cac taagcagccc ttctccatcc    911
Cys Thr Cys Val Asn Cys Ser Cys Gly Gly His
            60                  65

tccagacaac tataatatgt tacaaataga acttgtgcat gcatgagctc tgtacaataa     971

aactgtgact atagtgtcaa gtgtctgagt gtcaattagt ccccttgtat ttcagtttcc    1031

ttgttacctg tgtaatgtgt tcacagctgc tacattagta cagttgtgtt aaatgaatca    1091

cttcctagtt t                                                         1102


<210>  12
<211>  67
<212>  PRT

<213>  Apium graveolens

<220>
<221>  misc_feature
<222>  (38)..(38)
<223>  The 'Xaa' at location 38 stands for Thr, Ala, Pro, or Ser.

<400>  12

Met Ser Asn Thr Cys Gly Asn Cys Asp Cys Ser Asp Lys Ser Gln Cys
1               5                   10                  15

Val Lys Lys Gly Ala Ser Tyr Gly Leu Asp Ile Val Glu Thr Gly Lys
            20                  25                  30

Ser Tyr Val Gln Thr Xaa Val Met Glu Val Ser Ala Thr Glu Asn Asp
        35                  40                  45

Gly Lys Cys Lys Cys Gly Thr Ser Cys Thr Cys Val Asn Cys Ser Cys
    50                  55                  60

Gly Gly His
65

<210>  13
<211>  3108
<212>  DNA
<213>  Apium graveolens

<400>  13
atggccggaa cttctggccc ttccggcggt gtcgtcgccg atcccaagaa tctgagttca      60

cctttatttg acgcggagaa aaaacccaaa aataacaagt atgcttttgc ttgttccatt     120

ttagcttcca tgacttcaat tctacttggt tatggtacga tgtctttcaa acctacactt     180

attttcagta cactcacaat gtcacaaatt cttcgtaatc gatctctctt ccacgatcag     240

tatttttatt ttaattcaat ttattaaagc tttcggacag atctctttag ttttaccgac     300

acatattaca ttagaataat ctgaacaaca ctctgcatgt ctttttgtct taactatctt     360

atgatctaaa cactctactt gtgttgtgtt ataacctttt caatttttta ttttttagat      420

acgggggtta tgagtggagc agcaatctac ataaaaaaag atctccgttt caccgatgta     480

caaatcgaaa tcatcgtcgg aatcatcaac atcttctctc ttctcggctc ttttctcgcc     540

ggaagaacct ccgattggat tggccggaga tacacaatgg ttctagccgg tggcatattt     600

tttgccggag cttttttaat gggatgtgct acaaactttg agtttttaat ggtgggtcgg     660

tttgtcgccg ggatcggagt agggtatgct atgatgatcg ctccggttta tacaactgag     720

gttgctccgg cgtcttctcg gggttttctc acttctttcc cggaggtctt tattaatgct     780

ggtgcgtttt tattcgctaa ttaatttata tttatttatt tatttgtata aattagatat     840

aatttttaat ttaaattgaa taaaacttta ctgtactaaa gatcagataa cgtatatctc     900

gtgcaaatgt tgtggaacac atcatgaaga taataatatt aagcatatat taaaataatg     960

ttttattaaa aatatgtgaa ctgttgtttg gtgttaactg ctttttttta tttggtgttc    1020

gtgatgtttt aacaacactg accaatatgt aagtgtgtac aactttacca acaaaagata 1080
ctgttattaa agtacagatt atgtgaatat tatttatata aaataaaaaa atatgtaggt 1140
ccagtgaagc atttttcgtc gtttagatgt gtggtcctta ttgataggta gagttgtgta 1200
tcttttgctt ttgtacacgt ttacaataag atatttggtt gtcaatttaa cagctgtata 1260
gctttgatga cctgtgttat atattatgtg gtgtacatag gttgtgattg tgatgtttat 1320
ctgataatta gtctgttttt atttgttata tttttgggtt tgaattggtt aacatgtagt 1380
gatgagttgt tgaactcgat ttaattgtat atagttggac agttgtgatt actcgatttg 1440
atcgaatata attggtattg gacagttgtg attggttttt tttggataat gttggcggct 1500
ttttcttgat tatcgtttgg atgataatga aatgttaatt gttattgaca ttacatggta 1560
gatgtaacta tgttgtttgt taacaggagt tatgctgggg tatgtatcca actttgcatt 1620
tgcaaagctt ccgctttggt taggctggag gtttatgctt ggaattggag cagttccttc 1680
ggttggctta gccattggtg tattgtatat gcctgagtct ccgcgttggc ttgtcatgag 1740
gggtcaactt ggcgaagcaa ggcgtgtact ggaaaagact tcggagagca aagaagaagc 1800
tcgacaaaga ctagaagata tcaaggaggc tgctggaatt ccagaagaat gtaatgatga 1860
cgttgttgaa gttcctaaac gtagcaaaga cgatgctgtg tggaaagaat tgttccttca 1920
tcctacacca gctgttcgcc atgctgctat cactggcatt ggtattcatt tcttccaaat 1980
ggctagtggt gttgatgctg ttgttttgta cagtcctcga atttttgaga aggctgggtt 2040
aaagagtgat aaccacaagc tactcgccac cattggtgtt ggagtctgca aaactatttt 2100
tgttttgata tcaacatttt tgctagacaa agtcggacgg cgcccactga tgctttcgag 2160
tatggggggc atggtaattg ctctactcgt actctcaggc tcattgtctg taattaatca 2220
ctcgcatcaa accgttccct gggctgttgc tttggcaata atttcggtgt atggctttgt 2280
gtcggtgttt tcaagtggga tggggccaat tgcttgggtg tatagttcgg aggtgtttcc 2340
tttgaggctt agagcccaag gttgcagtat cggagtggca gtcaatcgtg gtgttagtgg 2400
cattatcgga atgacattta tatcaatgta caaggccttg actattggtg gtgcattctt 2460
tgtattcgct gtggttgcag caattggatg ggtattcatg ttcacaatgt ttcctgaaac 2520
tcaaggaaga aatcttgaag aaattgaggt attgtttggc agttactttg gctggaggaa 2580
aacattgaag gatttgaaga agaaagaagc ggcagaagca aagaatgtct gcattgttgc 2640
ttaaaattca aatacagcgg ggattatagc tttgtgatgt taaatgtgtt tgagcgaggg 2700
tgcaaaacca aacatacccg gtatattcac tcctaagtag aatttctgga gtacctgcgg 2760
atttgtttgt gttaactaag ggcgatttta tcaaaatcct tggtaccctt ggaactcctc 2820
taataaattt aaaacagtat tgtggttttt acttgattcg tgacattcct acatttctgc 2880
ttctcatctc tagttttatg tacgcatata attgtgctta gtactcctac gttattgctc 2940
aacctctgtt tgtgaatcga atatggtttg ctgacatctt ccgagaccag aaacggaaag 3000
agtaaatgtt ttttcgcatg tgcaattata acatcaatgt cttgcgttta attggtatga 3060
tatatgttct cttgtttgca gcttctttgc tcagttcata tgcacaat 3108

<210>  14
<211>  1575
<212>  DNA
<213>  Apium graveolens

<400>  14
atggccggaa cttctggccc ttccggcggt gtcgtcgccg atcccaagaa tctgagttca      60

cctttatttg acgcggagaa aaaacccaaa aataacaagt atgcttttgc ttgttccatt     120

ttagcttcca tgacttcaat tctacttggt tatgatacgg gggttatgag tggagcagca     180

atctacataa aaaaagatct ccgtttcacc gatgtacaaa tcgaaatcat cgtcggaatc     240

atcaacatct tctctcttct cggctctttt ctcgccggaa gaacctccga ttggattggc     300

cggagataca caatggttct agccggtggc atattttttg ccggagcttt tttaatggga     360

tgtgctacaa actttgagtt tttaatggtg ggtcggtttg tcgccgggat cggagtaggg     420

tatgctatga tgatcgctcc ggtttataca actgaggttg ctccggcgtc ttctcggggt     480

tttctcactt ctttcccgga ggtctttatt aatgctggag ttatgctggg gtatgtatcc     540

aactttgcat ttgcaaagct tccgctttgg ttaggctgga ggtttatgct tggaattgga     600

gcagttcctt cggttggctt agccattggt gtattgtata tgcctgagtc tccgcgttgg     660

cttgtcatga ggggtcaact tggcgaagca aggcgtgtac tggaaaagac ttcggagagc     720

aaagaagaag ctcgacaaag actagaagat atcaaggagg ctgctggaat tccagaagaa     780

tgtaatgatg acgttgttga agttcctaaa cgtagcaaag acgatgctgt gtggaaagaa     840

ttgttccttc atcctacacc agctgttcgc catgctgcta tcactggcat tggtattcat     900

ttcttccaaa tggctagtgg tgttgatgct gttgttttgt acagtcctcg aatttttgag     960

aaggctgggt taaagagtga taaccacaag ctactcgcca ccattggtgt tggagtctgc    1020

aaaactattt ttgttttgat atcaacattt ttgctagaca aagtcggacg gcgcccactg    1080

atgctttcga gtatgggggg catggtaatt gctctactcg tactctcagg ctcattgtct    1140

gtaattaatc actcgcatca aaccgttccc tgggctgttg ctttggcaat aatttcggtg    1200

tatggctttg tgtcggtgtt ttcaagtggg atggggccaa ttgcttgggt gtatagttcg    1260

gaggtgtttc ctttgaggct tagagcccaa ggttgcagta tcggagtggc agtcaatcgt    1320

ggtgttagtg gcattatcgg aatgacattt atatcaatgt acaaggcctt gactattggt    1380

ggtgcattct ttgtattcgc tgtggttgca gcaattggat gggtattcat gttcacaatg    1440

tttcctgaaa ctcaaggaag aaatcttgaa gaaattgagg tattgtttgg cagttacttt    1500

ggctggagga aaacattgaa ggatttgaag aagaaagaag cggcagaagc aaagaatgtc    1560

tgcattgttg cttaa                                                      1575

<210>  15
<211>  50
<212>  DNA
<213>  Artificial

<220>

<223>  primer

<400>  15
ggggacaagt ttgtacaaaa aagcaggctg aacagaaaca attgtggatg                50


<210>  16
<211>  50
<212>  DNA
<213>  artificial

<220>
<223>  primer

<400>  16
ggggaccact ttgtacaaga aagctgggta atgttgagaa acaatggtcg                50


<210>  17
<211>  50
<212>  DNA
<213>  Artificial

<220>
<223>  primer

<400>  17
ggggacaagt ttgtacaaaa aagcaggctg acccactatc aacaatgatc                50


<210>  18
<211>  50
<212>  DNA
<213>  Artificial

<220>
<223>  primer

<400>  18
ggggaccact ttgtacaaga aagctgggta taagatcgtt gtggactctg                50


<210>  19
<211>  50
<212>  DNA
<213>  Artificial

<220>
<223>  primer

<400>  19
ggggacaagt ttgtacaaaa aagcaggctt ctttattctg cagctagagc                50


<210>  20
<211>  50
<212>  DNA
<213>  Artificial

<220>
<223>  primer

<400>  20
ggggaccact ttgtacaaga aagctgggtg cttgaagtaa ggtggtatgc                50


<210>  21
<211>  23
<212>  DNA

<213>  Artificial

<220>
<223>  primer

<400>  21
atacagcggg gattatagct ttg                                              23


<210>  22
<211>  23
<212>  DNA
<213>  Artificial

<220>
<223>  primer

<400>  22
atccgcaggt actccaaaaa ttt                                              23


<210>  23
<211>  18
<212>  DNA
<213>  Artificial

<220>
<223>  probe

<400>  23
tacccggtat attcactc                                                    18


<210>  24
<211>  18
<212>  DNA
<213>  Artificial

<220>
<223>  primer

<400>  24
agccgctgga ccctacct                                                    18


<210>  25
<211>  25
<212>  DNA
<213>  Artificial

<220>
<223>  primer

<400>  25
agttatcttt tctgtttaac agcct                                            25


<210>  26
<211>  16
<212>  DNA
<213>  Artificial

<220>
<223>  probe

<400>  26
ctaagccgtt tccagg                                                      16

<210>  27
<211>  27
<212>  DNA
<213>  Artificial

<220>
<223>  primer

<400>  27
gactagtccc aagaatctga gttcacc                                          27


<210>  28
<211>  28
<212>  DNA
<213>  Artificial

<220>
<223>  primer

<400>  28
ccgctcgagc atcacaaagc tataatcc                                         28


<210>  29
<211>  20
<212>  DNA
<213>  Artificial

<220>
<223>  primer

<400>  29
agcttcgacc attgtttctc                                                  20


<210>  30
<211>  28
<212>  DNA
<213>  Artificial

<220>
<223>  primer

<400>  30
ccgctcgagc atcacaaagc tataatcc                                         28


<210>  31
<211>  518
<212>  PRT
<213>  Apium graveolens

<400>  31

Met Thr Gly Ala Ser Met Ile Thr Gly Glu Val Ser Val Asp Ser Tyr
1               5                   10                  15


Asp Thr Asn Lys Pro Lys Pro Lys Arg Asn Lys Tyr Ala Phe Ala Cys
            20                  25                  30


Ala Leu Leu Ala Ser Met Asn Ser Ile Leu Leu Gly Tyr Asp Thr Gly
        35                  40                  45


Val Leu Ser Gly Ala Ser Ile Tyr Ile Lys Glu Asp Leu His Phe Ser
    50                  55                  60

Asp Val Gln Ile Glu Ile Ile Ile Gly Ile Ile Asn Ile Tyr Ser Leu
65                  70                  75                  80

Leu Gly Ser Ala Ile Ala Gly Arg Thr Ser Asp Trp Ile Gly Arg Arg
                85                  90                  95

Tyr Thr Met Val Leu Ala Gly Ile Ile Phe Phe Leu Gly Ala Ile Phe
            100                 105                 110

Met Gly Leu Ala Thr Asn Phe Ala Phe Leu Met Phe Gly Arg Phe Val
        115                 120                 125

Ala Gly Ile Gly Val Gly Tyr Ala Met Met Ile Ala Pro Val Tyr Thr
    130                 135                 140

Ala Glu Val Ala Pro Ser Ser Ser Arg Gly Phe Leu Thr Ser Phe Pro
145                 150                 155                 160

Glu Val Phe Ile Asn Ser Gly Val Leu Leu Gly Tyr Val Ser Asn Phe
                165                 170                 175

Ala Phe Ala Lys Cys Pro Leu Trp Leu Gly Trp Arg Ile Met Leu Gly
            180                 185                 190

Ile Gly Ala Phe Pro Ser Val Ala Leu Ala Ile Ile Val Leu Tyr Met
        195                 200                 205

Pro Glu Ser Pro Arg Trp Leu Val Met Gln Gly Arg Leu Gly Glu Ala
    210                 215                 220

Arg Thr Val Leu Glu Lys Thr Ser Thr Ser Lys Glu Glu Ala His Gln
225                 230                 235                 240

Arg Leu Ser Asp Ile Lys Glu Ala Ala Gly Ile Asp Lys Asp Cys Asn
                245                 250                 255

Asp Asp Val Val Gln Val Pro Lys Arg Thr Lys Asp Glu Ala Val Trp
            260                 265                 270

Lys Glu Leu Ile Leu His Pro Thr Lys Pro Val Arg His Ala Ala Ile
        275                 280                 285

Thr Gly Ile Gly Ile His Phe Phe Gln Gln Ala Cys Gly Ile Asp Ala
    290                 295                 300

Val Val Leu Tyr Ser Pro Arg Ile Phe Glu Lys Ala Gly Ile Lys Ser
305                 310                 315                 320

Asn Ser Lys Lys Leu Leu Ala Thr Ile Ala Val Gly Val Cys Lys Thr
                325                 330                 335

Val Phe Ile Leu Ile Ser Thr Phe Gln Leu Asp Lys Ile Gly Arg Arg
            340                 345                 350

Pro Leu Met Leu Thr Ser Met Gly Gly Met Val Ile Ala Leu Phe Val
        355                 360                 365

Leu Ala Gly Ser Leu Thr Val Ile Asn Lys Ser His His Thr Gly His
    370                 375                 380

Trp Ala Gly Gly Leu Ala Ile Phe Thr Val Tyr Ala Phe Val Ser Ile
385                 390                 395                 400

Phe Ser Ser Gly Met Gly Pro Ile Ala Trp Val Tyr Ser Ser Glu Val
                405                 410                 415

Phe Pro Leu Arg Leu Arg Ala Gln Gly Cys Ser Ile Gly Val Ala Val
            420                 425                 430

Asn Arg Gly Met Ser Gly Ile Ile Gly Met Thr Phe Ile Ser Met Tyr
        435                 440                 445

Lys Ala Met Thr Ile Gly Gly Ala Phe Leu Leu Phe Ala Val Val Ala
    450                 455                 460

Ser Ile Gly Trp Val Phe Met Tyr Thr Met Phe Pro Glu Thr Gln Gly
465                 470                 475                 480

Arg Asn Leu Glu Glu Ile Glu Leu Leu Phe Gly Ser Tyr Phe Gly Trp
                485                 490                 495

Arg Lys Thr Leu Lys Asp Leu Lys Ala Lys Glu Ala Ala Glu Ala Lys
            500                 505                 510

Ser Arg Glu Ser Glu Val
        515

<210>  32
<211>  524
<212>  PRT
<213>  Apium graveolens

<400>  32

Met Ala Gly Ala Phe Asn Val Ser Gly Asp Asp Ser Gly Gly Ser Lys
1               5                   10                  15

Asp Val Gly Ser Pro Val Ile Phe Asp Ala Val Lys Lys Pro Lys Arg
            20                  25                  30

Asn Lys Tyr Ala Phe Gly Cys Ala Ile Leu Ala Ser Met Thr Ser Val
        35                  40                  45

Leu Leu Gly Tyr Asp Ile Gly Val Met Ser Gly Ala Ala Ile Tyr Ile

50                  55                  60

Lys Asp Gln Leu His Val Ser Asp Val Lys Leu Glu Ile Val Val Gly
65                  70                  75                  80

Ile Ile Asn Phe Phe Ser Leu Val Gly Ser Ala Leu Ala Gly Arg Thr
                85                  90                  95

Ser Asp Trp Ile Gly Arg Arg Tyr Thr Met Val Leu Ala Gly Ala Ile
            100                 105                 110

Phe Phe Val Gly Ala Ile Leu Met Gly Phe Ala Thr Asn Tyr Ser Phe
        115                 120                 125

Leu Met Phe Gly Arg Phe Val Ala Gly Ile Gly Val Gly Tyr Ala Leu
    130                 135                 140

Met Ile Ala Pro Val Tyr Thr Ala Glu Val Ser Ser Ala Ser Ser Arg
145                 150                 155                 160

Gly Phe Leu Thr Ser Phe Pro Glu Val Phe Ile Asn Ile Gly Val Leu
                165                 170                 175

Leu Gly Tyr Val Ser Asn Tyr Ala Phe Ser Lys Leu Pro Ala Asn Leu
            180                 185                 190

Gly Trp Arg Phe Met Leu Gly Ile Gly Ala Ile Pro Ser Ile Gly Leu
        195                 200                 205

Ala Ile Gly Val Leu Gly Met Pro Glu Ser Pro Arg Trp Leu Val Met
    210                 215                 220

Lys Gly Arg Leu Gly Glu Ala Arg Gln Val Leu Asp Lys Thr Ser Asp
225                 230                 235                 240

Ser Lys Glu Glu Ser Arg Leu Arg Leu Ser Asp Ile Lys Gln Ala Ala
                245                 250                 255

Gly Ile Pro Glu Glu Cys Asn Asp Asp Ile Val Val Met Pro Lys Arg
            260                 265                 270

Arg Asn Asp Glu Ala Val Trp Lys Glu Leu Leu Leu His Pro Thr Pro
        275                 280                 285

Ser Val Arg His Ala Phe Ile Ala Gly Val Gly Leu His Phe Phe Gln
    290                 295                 300

Gln Ser Ser Gly Ile Asp Ala Gly Gly Leu Tyr Ser Pro Arg Ile Phe
305                 310                 315                 320

Glu Lys Ala Gly Ile Thr Ser Thr Asp Leu Lys Leu Leu Ala Thr Ile
                325                 330                 335

Ala Val Gly Ile Ser Lys Thr Leu Phe Ile Leu Val Ala Thr Phe Leu
            340                 345                 350

Leu Asp Arg Ile Gly Arg Arg Pro Leu Leu Leu Thr Ser Met Gly Gly
        355                 360                 365

Met Ile Ile Ser Leu Thr Leu Leu Gly Thr Ser Leu Ala Val Ile Asp
    370                 375                 380

His Ser Asp His Thr Val His Trp Ala Val Ala Leu Ala Ile Phe Gly
385                 390                 395                 400

Val Leu Ala Tyr Val Gly Thr Phe Ser Ile Gly Leu Gly Pro Ile Ala
                405                 410                 415

Trp Gly Tyr Ser Ser Glu Val Phe Pro Leu Arg Leu Arg Ala Gln Gly
            420                 425                 430

Cys Ser Ile Gly Val Ala Val Asn Arg Gly Thr Ser Gly Ile Ile Ser
        435                 440                 445

Met Thr Phe Leu Ser Leu Tyr Lys Ala Ile Ser Ile Ala Gly Ala Phe
    450                 455                 460

Tyr Leu Phe Ala Ala Ile Ala Gly Val Ala Trp Ile Phe Ile Phe Thr
465                 470                 475                 480

Leu Leu Pro Glu Thr Gln Gly Arg Ser Leu Glu Glu Met Gly Leu Leu
                485                 490                 495

Phe Gly Thr Tyr Phe Gly Trp Arg Lys Thr Leu Lys Gly Leu Lys Asn
            500                 505                 510

Arg Glu Ala Glu Glu Ala Lys Asn Ala Asn Val Ile
        515                 520

<210>  33
<211>  1849
<212>  DNA
<213>  Apium graveolens

<400>  33
caatgtagga tattattagc ctcatctgca acaaactctt tactagtatt gctctcactc      60

tcctttattt tctctcttct aaatatgacc ggagcttcta tgattaccgg cgaagtttcc     120

gtcgattcat atgatactaa caagcctaaa cctaaaagga ataagtatgc ttttgcttgt     180

gctcttttag cttccatgaa ttccatctta ctcggctatg acaccggagt gttgagtgga     240

gcatcaatat acataaagga agatctccat ttctccgacg ttcaaatcga aataatcatc     300

ggaatcatca acatctactc tcttcttggt tcggccatag ccggaaggac ctcggactgg     360

ataggcagac gttacaccat ggtactagct ggtatcatat tttttctagg agccattttc     420

atggggcttg ctacaaactt tgcctttctc atgtttggtc gctttgttgc tggaattggt 480

gtcggttatg ccatgatgat cgctcccgtc tacactgccg aggttgctcc gtcgtcttcc 540

cgtggtttcc tcacttcttt tcctgaggtt ttcattaatt ctggtgtgtt gctcgggtat 600

gtatccaact ttgcatttgc caagtgccca ctttggttag gctggagaat tatgctggga 660

attggagcat ttccttcagt tgccttggcc ataattgtgt tatatatgcc agagtcccca 720

cgttggctcg ttatgcaggg tcgacttggt gaagcgagga ctgtacttga gaaaacttct 780

acttccaaag aagaagctca ccaaagactg tctgatatta aggaagctgc tgggattgat 840

aaagattgta atgacgatgt tgttcaagtt ccaaaacgta ccaaagacga agcagtgtgg 900

aaagaattga ttcttcaccc tacaaaacct gttcgccacg ctgcaattac gggtattggt 960

attcatttct tccaacaggc ttgtggtatt gatgctgttg ttttatacag ccctcgaatt 1020

tttgaaaaag ctggtatcaa aagtaatagt aaaaagctcc ttgcgacaat tgctgttgga 1080

gtctgcaaaa cagtctttat tctgatatca acgtttcagc tggacaaaat tggacgacgc 1140

cccctgatgc taacaagtat ggggggtatg gttattgctc tatttgtact ggcaggctca 1200

ttgacggtta ttaacaaatc acatcatact ggtcattggg ctggtggttt ggcaatattt 1260

acagtgtatg cttttgtgtc gatattttca agtggcatgg gtccaattgc ttgggtctat 1320

agctccgagg tgttcccttt gaggctaaga gctcaaggtt gtagtatcgg agtggcagtt 1380

aaccgtggca tgagtggcat aattggaatg acatttatat cgatgtacaa agccatgact 1440

attggtggtg cattcctttt atttgctgtg gttgcatcta tcggatgggt ctttatgtac 1500

acaatgttcc ccgagacaca aggtagaaat ctcgaagaaa ttgagttatt gtttggcagc 1560

tactttggct ggaggaagac attgaaggat ttgaaggcaa aagaagctgc tgaagcaaag 1620

agtcgcgaga gtgaagttta gcagtcagat gaatttaggg ttcaaagatg ttatattagc 1680

tctgtgtaga gggtagtttt agagaagccc ttagtatgtg ttggagtatg tgtgattatt 1740

aaccatcacc cgataattta gaataagggt gtcaaagaac aattacccat ttcttatgtg 1800

gtaatctatt gaaaagaatt tgcccaatgg taaaaaaaaa aaaaaaaaa 1849

<210> 34
<211> 1781
<212> DNA
<213> Apium graveolens

<400> 34
tcggcaccag atcacactcc atgttcatgt gccacattgt accctaaaac agtacttagc 60

tcttcatctc tcttaaagat ggctggagct ttcaatgttt ccggtgacga ttccggcggc 120

tctaaagatg taggatcacc tgtaatattt gatgctgtca agaaacctaa acgaaataaa 180

tatgcttttg gttgcgctat tttagcttcc atgacttccg ttttgcttgg ttatgatatt 240

ggagtgatga gtggagcagc aatttacata aaagatcagc tccatgtgtc ggacgtaaaa 300

ctggagattg ttgtcggaat aattaacttc ttttcacttg ttgggtctgc tcttgccgga 360

agaacctccg actggattgg caggcgttac acaatggtgc tagccggtgc tatatttttt 420

gttggagcaa tacttatggg atttgcaacc aactattcat ttctgatgtt tggtcgattc 480

gtcgccggaa tcggagtagg ttatgccctc atgattgctc cggtgtacac ggccgaggtt 540

tcttcagcat catctcgtgg atttcttact tcattcccgg aagttttcat taatatcggt 600

gtactgctcg gatatgtctc gaattatgca ttttccaaac tccctgccaa cttgggctgg 660

cgattcatgc ttggaatcgg agcaatacct tcaatcggtt tagcgattgg tgtcctaggc 720

atgcccgaat cacctcgttg gctcgtcatg aaaggccgtc tcggcgaagc tagacaagtc 780

ctagacaaaa cctcagattc caaagaagaa tctcgcctta gattatccga catcaaacag 840

gctgctggca tacccgaaga atgcaacgac gatatcgttg taatgcctaa acggaggaat 900

gacgaggcag tgtggaaaga gttgcttctc catcctacac catcagtccg tcacgcgttc 960

attgctggcg ttggtctaca ttttttccaa caatcaagtg gcatagacgc tggtggtttg 1020

tacagtcctc gaattttcga aaaggctgga atcacgagta ccgacttaaa actgctagca 1080

acaatagctg ttggaatctc gaagacactc ttcatcctag tagctacatt tttactcgat 1140

cgaatcggac gacgtccatt gcttctcaca agcatgggag gaatgatcat atccttaact 1200

ctcctgggaa catccttggc tgttattgac cactcagatc acacagttca ttgggccgtg 1260

gcattggcaa tcttcggagt tttagcatac gtgggcacgt tttctattgg gctagggcca 1320

attgcatggg gttatagttc agaggtgttc ccattacgac taagggccca aggatgtagc 1380

attggagtag ctgttaacag gggtacaagt ggaattatct cgatgacatt tttgtcgcta 1440

tacaaagcca taagtatagc aggggcattc tatttatttg cagctattgc aggagtggca 1500

tggatattta tattcacatt acttcctgaa acacaaggga ggagccttga agaaatgggg 1560

ttactgtttg gaacctattt tggttggaga aaaactttga agggtttgaa gaacagagaa 1620

gctgaggaag ctaaaaatgc taatgtcata tagaaagttt atatttgtca agtatgggta 1680

gtctacttta tatgactctg ttaccggtac ataattttgt aaaataatgt gaattcggga 1740

acaaatccat gtgtgatgag accaaaaaaa aaaaaaaaaa a 1781

<210> 35
<211> 1908
<212> DNA
<213> Apium graveolens

<400> 35
ccctcacgtt acgatagtca aattgtttag tcctttcccc caagcttcga ccattgtttc 60

tcaacatttt gtaatctctc tctctctcga tggccggaac ttctggccct tccggcggtg 120

tcgtcgccga tcccaagaat ctgagttcac ctttatttga cgcggagaaa aaacccaaaa 180

ataacaagta tgcttttgct tgttccattt tagcttccat gacttcaatt ctacttggtt 240

atgatacggg ggttatgagt ggagcagcaa tctacataaa aaaagatctc cgtttcaccg 300

atgtacaaat cgaaatcatc gtcggaatca tcaacatctt ctctcttctc ggctcttttc 360

tcgccggaag aacctccgat tggattggcc ggagatacac aatggttcta gccggtggca 420

tattttttgc cggagctttt ttaatgggat gtgctacaaa ctttgagttt ttaatggtgg 480

gtcggtttgt cgccgggatc ggagtagggt atgctatgat gatcgctccg gtttatacaa 540

ctgaggttgc tccggcgtct tctcggggtt ttctcacttc tttcccggag gtctttatta 600

atgctggagt tatgctgggg tatgtatcca actttgcatt tgcaaagctt ccgctttggt 660

taggctggag gtttatgctt ggaattggag cagttccttc ggttggctta gccattggtg 720

tattgtatat gcctgagtct ccgcgttggc ttgtcatgag gggtcaactt ggcgaagcaa 780

ggcgtgtact ggaaaagact tcggagagca aagaagaagc tcgacaaaga ctagaagata 840

tcaaggaggc tgctggaatt ccagaagaat gtaatgatga cgttgttgaa gttcctaaac 900

gtagcaaaga cgatgctgtg tggaaagaat tgttccttca tcctacacca gctgttcgcc 960

atgctgctat cactggcatt ggtattcatt tcttccaaat ggctagtggt gttgatgctg 1020

ttgttttgta cagtcctcga atttttgaga aggctgggtt aaagagtgat aaccacaagc 1080

tactcgccac cattggtgtt ggagtctgca aaactatttt tgttttgata tcaacatttt 1140

tgctagacaa agtcggacgg cgcccactga tgctttcgag tatggggggc atggtaattg 1200

ctctactcgt actctcaggc tcattgtctg taattaatca ctcgcatcaa accgttccct 1260

gggctgttgc tttggcaata atttcggtgt atggctttgt gtcggtgttt tcaagtggga 1320

tggggccaat tgcttgggtg tatagttcgg aggtgtttcc tttgaggctt agagcccaag 1380

gttgcagtat cggagtggca gtcaatcgtg gtgttagtgg cattatcgga atgacattta 1440

tatcaatgta caaggccttg actattggtg gtgcattctt tgtattcgct gtggttgcag 1500

caattggatg ggtattcatg ttcacaatgt ttcctgaaac tcaaggaaga aatcttgaag 1560

aaattgaggt attgtttggc agttactttg gctggaggaa aacattgaag gatttgaaga 1620

agaaagaagc ggcagaagca aagaatgtct gcattgttgc ttaaaattca aatacagcgg 1680

ggattatagc tttgtgatgt taaatgtgtt tgagcgaggg tgcaaaacca aacatacccg 1740

gtatattcac tcctaagtag aatttctgga gtacctgcgg atttgtttgt gttaactaag 1800

ggcgatttta tcaaaatcct tggtaccctt ggaactcctc taataaattt aaaacagtat 1860

tgtggttttt acttgaaaaa aaaaaaaaaa aaaaaaaaaa aaaaaaaa 1908

<210> 36
<211> 50
<212> DNA
<213> Artificial

<220>
<223> Primer

<400> 36
ggggacaagt ttgtacaaaa aagcaggctg aacagaaaca attgtggatg 50

<210> 37
<211> 19
<212> DNA
<213> artificial

<220>
<223> Primer

<400>  37
cgatccagac tgaatgccc                                                    19


<210>  38
<211>  50
<212>  DNA
<213>  artificial

<220>
<223>  Primer

<400>  38
ggggacaagt ttgtacaaaa aagcaggctg acccactatc aacaatgatc                  50